(19) Europäisches Patentamt
European Patent Office
Office européen des brevets

(11) Numéro de publication: 0 143 675
B1

(12) # FASCICULE DE BREVET EUROPÉEN

(45) Date de publication du fascicule du brevet: 07.02.90

(51) Int. Cl.[5]: C 07 H 13/12, C 07 H 15/00, A 61 K 31/70

(21) Numéro de dépôt: 84401743.4

(22) Date de dépôt: 30.08.84

(54) **Nouveaux dérivés de nitrosourée, leur procédé de préparation et compositions pharmaceutiques les contenant.**

(30) Priorité: 30.08.83 FR 8313878

(43) Date de publication de la demande: 05.06.85 Bulletin 85/23

(45) Mention de la délivrance du brevet: 07.02.90 Bulletin 90/06

(84) Etats contractants désignés: AT BE CH DE FR GB IT LI LU NL SE

(56) Documents cité:
EP-A-0 067 019
US-A-3 940 383

**Le dossier contient des informations techniques présentées postérieurement au dépôt de la demande et ne figurant pas dans le présent fascicule.**

(73) Titulaire: DROPIC Société Civile de Gestion de Droit de Propriété Industrielle CHOAY
10 Avenue Matignon
F-75008 Paris (FR)

(72) Inventeur: Roger, Pierre
6, rue Paul Valéry
F-78423 Montigny le Bretonneux (FR)
Inventeur: Choay, Patrick
7, Cour Jasmin
F-75016 Paris (FR)
Inventeur: Monneret, Claude
9, avenue Lamoricière
F-75012 Paris (FR)
Inventeur: Fournier, Jean-Paul
10, rue Fontenay
F-78000 Versailles (FR)

(74) Mandataire: Gutmann, Ernest
S.C. Ernest Gutmann - Yves Plasseraud 67, boulevard Haussmann
F-75008 Paris (FR)

EP 0 143 675 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe dipposition (Art. 99(1) Convention sur le brevet européen).

LIBERGRAF, STOCKHOLM 1990

## Description

La présente invention est relative à de nouveaux dérivés de nitrosourées et, de façon plus spécifique, à de nouvelles désoxy–2–sucres–nitrosourées et désoxy–4–sucres nitrosourées, à leurs procédés de préparation et à leurs applications thérapeutiques.

On sait que diverses nitrosourées présentent une activité cytostatique et oncostatique puissante décelée dans le cadre d'expérimentations pharmacologiques et de traitements cliniques: c'est le cas, en particulier, de la (1,3–bis–2–chloroéthyl)–1–nitrosourée [BCNU] commercialisée sous la marque "BICNU" (cf. Dictionnaire VIDAL 1984), de la (chloro–2–éthyl)–1–cyclohexyl–3–nitrosourée [CCNU] commercialisée sous la marque "BELUSTINE" (cf. Dictionnaire VIDAL 1984) et de la (chloro–2–éthyl)–1–(méthyl–4–cyclohexyl)–3–nitrosourée [Me CCNU]: cf. G. MATHE et Y. KENIS: Expansion Scientifique, 1975, 3ème Ed. "La Chimiothérapie des cancers (leucémies, hématosarcomes et tumeurs solides)" et T. H. WASSERMAN, M. SLAVIK & S. K. CARTER, Cancer Treat. Rev., 1974, 1, p. 131, "Review of CCNU in clinical cancer therapy". G. P. WHEELER et Alia (Cancer Res., 1974, 34 194) attribuent leur action oncostatique à une alkylation et à une carbamoylation de protéines. Il a en outre été suggéré que leur caractère lipophile est essentiel dans la mesure où il conditionne le franchissement des membranes cellulaires en particulier la barrière hemato-meningée. Cependant, ces composés ont l'inconvénient de présenter une certaine toxicité, notamment hématologique, aux doses auxquelles ils se révèlent actifs. Par conséquent, cette toxicité limite leur utilisation à des doses inférieures à celles qui semblent nécessaires pour l'éradication de cellules cancéreuses et a incité une équipe de chercheurs à viser à l'obtention de médicaments plus actifs et moins toxiques que les précédents, en synthétisant des dérivés de nitrosourées dont le caractère hydrophile est augmenté par rapport aux précédents, tels que des sucresnitrosourées, dans lesquelles la molécule de sucre est du ribose, du xylose ou du glucose: cf. J. L. IMBACH et Alia, Biomedicine, 1975, 23, p. 410 - 413, "The oncostatic and immunosuppressive action of new nitrosourea derivatives containing sugar radicals". C'est ainsi que ces auteurs ont mis en évidence l'activité oncostatique des quatre composés suivants sur la leucémie L 1210 et leur faible toxicité: la (chloro–2–éthyl)–1–(ribofuranosylisopropylidène–2',3'–paranitro benzoate–5')–3–nitrosourée [RFCNU], la (chloro–2–éthyl–1–désoxy–2'–glucopyranosyl-tétracétate 14,3', 4", 6')–3–nitrosourée [GCNU], la (chloro–2–éthyl)–1–(ribopyranosyl-triacétate 2', 3', 4')–3–nitrosourée [RPCNU] et la (chloro–2-3-éthyl)–1–(xylopyranosyl triacétate–2', 3', 4')–3–nitrosourée [XPCNU]. Ces composés sont préparés en faisant réagir l'amino-sucre approprié sur de l'isocyanate de chloro–2–éthyle, puis en procédant à la nitrosation de l'urée obtenue.

Ces composés se présentent sous forme huileuse, état physique difficile à manier en thérapeutique. C'est pourquoi, dans le cadre des expérimentations effectuées sur ces produits, on a eu de préférence recours à leur solidification par blocage des groupes hydroxy.

Dans un travail ultérieur, J. L. MONTERO et Alia (Eur. J. Med. Chem. Chimica Therapeutica, mars/avril 1976, 11, n° 2, p. 183 - 187: "Synthèse de nouvelles glycosylnitrosourées à visées oncostatiques - les nitroso-uréido–1–désoxy–1–glucopyranoses"), ont décrit des glycosyl-nitrosourées dans lesquelles la liaison sucreazote se situe en position anomère, qui présentent une activité oncostatique, à savoir le [(chloro–2–éthyl)-3–nitroso–3–ureido]–1–désoxy–1–bêta–D–glucopyranose et le tétra–O–acétyl–2, 3, 4, 6 [(chloro–2–éthyl)–3–nitroso-3–ureido]–1–désoxy–1–bêta–D–glucopyranose, et qui présentent en outre l'avantage d'une toxicité réduite sur la moëlle osseuse et de ne pas être diabétogène alors que la streptozotocine ou 2–désoxy–2–(3–méthyl-2–nitrosouréido)–D–glucopyranose, qui est un composé d'origine naturelle, présente des propriétés antibiotiques, antinéoplasiques, et présente également des propriétés diabétogènes indésirables ainsi qu'une toxicité rénale et hématologique élevées (cf. Drugs of the future, vol. IV, n° 2, 1979, p. 137 - 139).

Le brevet US n° 3 940 383 décrit de nouveaux analogues de streptozotocine, c'est-à-dire des composés dans lesquels la position en 3 est substituée par un groupe $CH_3N-CNH$.

$$\begin{array}{cc} | & \| \\ NO & O \end{array}$$

Mais, ces composés ne sont pas actifs sur les tumeurs solides.

Le brevet européen n° 67019 décrit des composés d'aldohexopyranosyle-, d'aldopentohexopyranosyle- et d'aldopentofuranosyle- nitrosourées présentant des propriétés antitumorales, dans lesquels les positions 2 et 4 du cycle sont toujours substituées par un groupe hydroxyle.

Par ailleurs, ces composés ne sont pas démontrés comme étant actifs sur les tumeurs solides.

L'un des aspects de l'invention est de proposer de nouveaux dérivés nitrosourées, dont la courbe d'activité présente un plateau très large bien en deçà du seuil de toxicité.

Un autre aspect de l'invention est de proposer de nouveaux dérivés de nitrosourée présentant un bon indice thérapeutique.

Un autre aspect de l'invention est également de proposer de nouveaux dérivés de nitrosourées, présentant des propriétés physiques facilitant leur utilisation en thérapeutique.

Un autre aspect de l'invention est de proposer de nouveaux dérivés de nitrosourées se présentant sous une forme solide et stable.

Ces différents aspects ont été obtenus grâce à une nouvelle série de dérivés de désoxy–2–sucres-nitrosourées et de désoxy–4–sucres-nitrosourées, qui se distinguent des dérivés de nitrosourées connus, notamment par la nature du synthon osidique, qui est un désoxy–2–sucre ou un désoxy–4–sucre.

On a constaté qu'en ayant recours à un désoxy-2–sucre ou à un désoxy–4–sucre, substitué sur le carbone

en 3 et/ou en 6 du susdit sucre, par un groupe nitrosourée, et susceptible de porter différents substituants sur le carbone en 4 du susdit sucre, lorsqu'il s'agit d'un désoxy-2-sucre ou sur le carbone en 2 du susdit sucre, lorsqu'il s'agit d'un désoxy-4-sucre, on obtenait de nouveaux dérivés de nitrosourées dont l'activité est considérablement augmentée et dont la toxicité est réduite par rapport aux composés déjà connus. L'un des intérêts de ces dérivés peut être rattaché à l'hypothèse selon laquelle les désoxy-2-sucre et les désoxy-4-sucre présentent une certaine labilité au niveau de la liaison osidique, laquelle est susceptible d'entraîner la formation d'un sucre libre dans certains milieux biologiques et de permettre de disposer de composés d'abord lipophiles, qui deviennent ensuite hydrophiles, ce qui faciliterait le passage des barrières cellulaires et entraînerait un accroissement de l'activité antitumorale.

L'invention a pour objet de nouveaux dérivés de nitrosourées, caractérisés en ce qu'ils répondent à la formule générale (I) suivante:

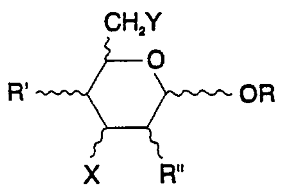

(I)

dans laquelle:

- R représente un atome d'hydrogène, un groupe alcoyle de 1 à 30, de préférence de 1 à 12 atomes de carbone ou un groupe aralcoyle de 7 à 12, de préférence de 7 à 9 atomes de carbone, éventuellement substitué par un ou plusieurs, notamment jusqu'à 3, atomes d'halogène groupes $NO_2$, $NH_2$, $CF_3$ ou groupes alcoxy de 1 à 4 atomes de carbone,
- X représente un groupe hydroxy ou un groupe $NR_1R_2$
- Y représente un atome d'hydrogène

```
                                        R'1
                                        /
  un groupe hydroxy ou un groupe N
                                        \
                                         R'2
```

où $R_1$ et/ou $R'_1$ représentent chacun un atome d'hydrogène ou ou un groupe $-\underset{\underset{O}{\|}}{C}-\underset{\underset{NO}{|}}{N}-CH_2CH_2Hal$,

Hal étant un halogène, de préférence Cl,

et $R_2$ et/ou $R'_2$ représentent chacun un atome d'hydrogène, un groupe alcoyle comprenant de 1 à 6 atomes de carbone, un groupe aralcoyle comprenant 7 à 12, de préférence de 7 à 9 atomes de carbone, un groupe cycloalcoyle comprenant de 3 à 6 atomes de carbone, un groupe aryle de 4 à 10 atomes de carbone, les groupes aryle et aralcoyle étant éventuellement substitués par un ou plusieurs, notamment jusqu'à 3. atomes d'halogène, groupes $NO_2$, $NH_2$, $CF_3$ ou groupes alcoxy de 1 à 4 atomes de carbone.

- R' ou R" représentent l'hydrogène, OH, OM, M représentant un groupe alcoyle comprenant de 1 à 30, de préférence de 1 à 12 atomes de carbone, un groupe aryle de 4 à 10 atomes de carbone, un groupe aralcoyle comprenant de 7 à 12, de préférence de 7 à 9 atomes de carbone, les groupes aryle et aralcoyle étant éventuellement substitués par un ou plusieurs, notamment jusqu'à 3, atomes d'halogène, groupes $NO_2$, $NH_2$, $CF_3$ ou groupes alcoxy de 1 à 4 atomes de carbone, ou M pouvant représenter également un groupe acyle de 2 à 8 atomes de carbone, de préférence 2 ou 3, ou un groupe aroyle de 5 à 12, de préférence de 5 à 9 atomes de carbone, non substitué ou substitué par un ou plusieurs, notamment jusqu'à 3, groupes $NO_2$, $NH_2$, $CF_3$, halogène, alcoxy de 1 à 4 atomes de carbone sous reserve qu'au moins X représente

```
   R1
  /
-N,
  \
   R2
```

avec $R_1$ représentant $-\underset{\underset{O}{\|}}{C}-\underset{\underset{NO}{|}}{N}-CH_2CH_2Hal$, ou Y représente

```
   R'1
  /
-N
  \
   R'2
```

avec $R'_1$ représentant

```
-C-N-CH2CH2Hal
 || |
 O NO
```

et sous réserve que soit R' soit R" représente l'hydrogène, R' et R" ne pouvant pas représenter simultanément l'hydrogène.

Une classe préférée de composés selon l'invention est constituée par ceux de formule 1 dans laquelle R' représente l'hydrogène et R" représente OH. Une autre classe préférée de composés selon l'invention est constituée par ceux de formule 1 dans laquelle R" représente l'hydrogène et R' représente OH.

Dans la figure 1, et certaines des figures ci-après, on a représenté les liaisons entre les groupes R, R', R", $CH_2Y$ et X d'une part et la structure cyclique d'autre part, par le symbole ~~ . Cette représentation signifie que chacun des groupes R, R', R", $CH_2Y$ et X peut être soit en position α, soit en position β, selon la représentation de HAYWORTH, et selon un arrangement compatible avec les exigences de stéréochimie.

Dans la suite de la description, le terme alcoyle inclue les alcoyle linéaires, ramifiés ou cycliques (cycloalcoyle).

Une classe préférée de composés selon l'invention est constituée par les composés répondant à la formule II ci-après:

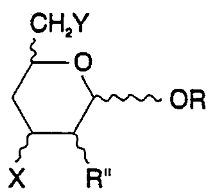

(II)

dans laquelle

- R représente un atome d'hydrogène, un groupe alcoyle de 1 à 30, de préférence de 1 à 12 atomes de carbone ou un groupe aralcoyle de 7 à 12, de préférence de 7 à 9 atomes de carbone, éventuellement substitué par un ou plusieurs, notamment jusqu'à 3, atomes d'halogène, groupes $NO_2$, $NH_2$, $CF_3$ ou groupes alcoxy de 1 à 4 atomes de carbone,
- X représente un groupe hydroxy ou un groupe $NR_1$, $R_2$
- Y représente un atome d'hydrogène,

```
          R'1
         /
un groupe -N
         \
          R'2
```

où $R_1$ et/ou $R'_1$ représentent chacun un atome d'hydrogène ou un groupe

```
-C-N-CH2CH2Hal,
 || |
 O NO
```

Hal étant un halogène, de préférence Cl,
et $R_2$ et/ou $R'_2$ représentent chacun un atome d'hydrogène, un groupe alcoyle comprenant de 1 à 6 atomes de carbone, un groupe aralcoyle comprenant de 7 à 12, de préférence de 7 à 9 atomes de carbone, un groupe cycloalcoyle comprenant de 3 à 6 atomes de carbone, un groupe aryle de 4 à 10 atomes de carbone, les groupes aryle et aralcoyle étant éventuellement substitués par un ou plusieurs, notamment jusqu'à 3 atomes d'halogène, groupe $NO_2$, $NH_2$, $CF_3$ ou groupes alcoxy de 1 à 4 atomes de carbone.

R" représente de préférence OH, mais peut être remplacé par OM, M représentant un groupe alcoyle comprenant de 1 à 30, de préférence de 1 à 12 atomes de carbone, un groupe aryle de 4 à 10 atomes de carbone, un groupe aralcoyle comprenant de 7 à 12, de préférence de 7 à 9 atomes de carbone, les groupes aryle et aralcoyle étant éventuellement substitués par un ou plusieurs, notamment jusqu'à, 3 atomes d'halogène, groupes $NO_2$, $NH_2$, $CF_3$, ou groupes alcoxy de 1 à 4 atomes de carbone, où M représentant un groupe acyle de 2 à 8 atomes de carbone, de préférence 2 ou 3, un groupe aroyle de 5 à 12, de préférence de 5 à 9 atomes de carbone, non substitué ou substitué par un ou plusieurs, notamment jusqu'à 3, groupes $NO_2$, $NH_2$, $CF_3$, halogène, alcoxy de 1 à 4 atomes de carbone.

sous reserve qu'au moins X représente

$R_1$
/
–N,
\
$R_2$

avec $R_1$ représentant –C–N–$CH_2CH_2$Hal,
|| |
O NO

ou Y représente

R'$_1$
/
–N
\
R'$_2$

avec R'$_1$ représentant –C–N–$CH_2CH_2$Hal
|| |
O NO

Ces composés de formule II représentent le cas particulier dans lequel R' représente l'hydrogène.

Parmi les composés de formule II, une classe préférée de composés selon l'invention est constituée par ceux de formule III ci-après:

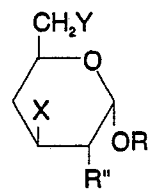

(III)

dans laquelle R, R", X et Y ont les significations indiquées ci-dessus.

Les composés selon l'invention de formule (III) appartiennent à la classe des composés désoxy–4, alpha–D–xylohexopyranosides.

Parmi les composés de formule (II), une classe préférée de composés selon l'invention est constituée par ceux de formule IV ci-après:

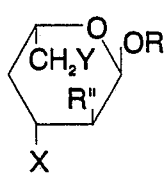

(IV)

dans laquelle R, R", X et Y ont les significations indiquées précédemment.

Les composés de formule (IV) appartiennent à la classe des composés désoxy–4, alpha-L-xylohexopyranosides.

Parmi les composés de formule I, une classe préférée de composé est constituée par ceux de formule IV ci-après:

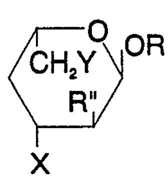

(IV)

dans laquelle R, R", X ont les significations indiquées précédemment et Y représente un groupe hydroxy.

Une classe particulièrement préférée de composés selon l'invention est constituée par les composés répondant à la formule V ci-après:

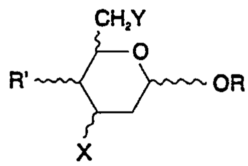

(V)

dans laquelle

- R représente un atome d'hydrogène, un groupe alcoyle de 1 à 30, de préférence de 1 à 12 atomes de carbone ou un groupe aralcoyle de 7 à 12, de préférence de 7 à 9 atomes de carbone, éventuellement substitué par un ou plusieurs, notamment jusqu'à 3, atomes d'halogène, groupes $NO_2$, $NH_2$, $CF_3$ ou groupes alcoxy de 1 à 4 atomes de carbone,
- X représente un groupe hydroxy ou un groupe $NR_1$, $R_2$
- Y représente un atome d'hydrogène

R'₁ → $R'_1$

un groupe hydroxy ou un groupe N (/ $R'_1$, \ $R'_2$)

où $R_1$ et/ou $R'_1$ représentent chacun un atome d'hydrogène ou ou un groupe $-\underset{\Vert}{C}-\underset{|}{N}-CH_2CH_2Hal$, (C=O, N–NO)

Hal étant un halogène, de préférence Cl,
et $R_2$ et/ou $R'_2$ représentent chacun un atome d' hydrogène, un groupe alcoyle comprenant de 1 à 6 atomes de carbone, un groupe aralcoyle comprenant de 7 à 12, de préférence de 7 à 9 atomes de carbone, un groupe cycloalcoyle comprenant de 3 à 6 atomes de carbone, un groupe aryle de 4 à 10 atomes de carbone, les groupes aryle et aralcoyle étant éventuellement substitués par un ou plusieurs, notamment jusqu'à 3, atomes d'halogène, groupes $NO_2$, $NH_2$, $CF_3$ ou groupes alcoxy de 1 à 4 atomes de carbone. R' représente de préférence OH, OH pouvant être remplacé par OM, M représentant un groupe alcoyle comprenant de 1 à 30, de préférence de 1 à 12 atomes de carbone, un groupe aryle de 4 à 10 atomes de carbone, un groupe aralcoyle comprenant de 7 à 12 de préférence de 7 à 9 atomes de carbone, les groupes aryle et aralcoyle étant éventuellement substitués par un ou plusieurs, notamment jusqu'à 3, atomes d'halogène, groupes $NO_2$, $NH_2$, $CF_3$ ou groupes alcoxy de 1 à 4 atomes de carbone, ou M pouvant représenter un groupe acyle de 2 à 8 atomes de carbone, de préférence 2 ou 3, un groupe aroyle de 5 à 12, de préférence de 5 à 9 atomes de carbone, non substitué ou substitué par un ou plusieurs, notamment jusqu'à 3, groupes $NO_2$, $NH_2$, $CF_3$, halogène, alcoxy de 1 à 4 atomes de carbone. sous reserve qu'au moins X représente

$R_1$
/
–N,
\
$R_2$

avec $R_1$ représentant $-\underset{\Vert}{C}-\underset{|}{N}-CH_2CH_2Hal$,
O NO

ou Y représente

$R'_1$
/
–N
\
$R'_2$

avec $R'_1$ représentant $-\underset{\Vert}{C}-\underset{|}{N}-CH_2CH_2Hal$
O NO

Ces composés de formule V représentent le cas particulier de la formule (I) dans lequel R" représente un atome d'hydrogène.

Parmi les composés de formule (V), une classe préférée de composés de l'invention est constituée par ceux

de formule (VI) ci–après:

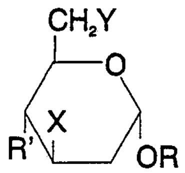

(VI)

dans laquelle R, R', X et Y ont les significations précédemment indiquées.

Les composés de formule (VI) appartiennent à la classe des composés désoxy-2, alpha–D–arabinohexopyranosides.

Parmi les composés de formule (V), une autre classe préférée de composés par l'invention est constituée par ceux de formule (VII) ci-après:

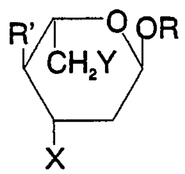

(VII)

dans laquelle R, R', X et Y ont les significations précédemment indiquées.

Ces composés appartiennent à la classe des composés désoxy–2, alpha–L–arabinohexopyranosides.

Une classe préférée de composés selon l'invention est constituée par ceux de formules (I), (V), (VI) et (VII) dans lesquelles:

- R représente un groupe alcoyle de 1 à 12 atomes de carbone, aralcoyle de 7 à 12 atomes de carbone;
- R' ou R" représente un groupe OM, M étant un groupe alcoyle comprenant de 1 à 12 atomes de carbone, un groupe aryle comprenant de 4 à 10 atomes de carbone;
- X représente un groupe $NR_1R_2$, $R_1$ représentant $–C–N–CH_2Ch_2$ Hal,
  
  || |
  
  O NO
  
  Hal étant un halogène, notamment Cl
- Y représente un atome d'hydrogène ou un groupe hydroxy

  Une autre classe préférée de composés selon l'invention est constituée par ceux de formules (I), (V), (VI) et (VII) dans lesquelles:

- R représente un groupe alcoyle de 1 à 12 atomes de carbone, halogénoaralcoyle de 4 à 10 atomes de carbone;
- R' ou R" représente un groupe OM. M étant un groupe acyle de 2 à 8 atomes de carbone, un groupe aroyle de 5 à 12 atomes de carbone;
- X représente un groupe $NR_1R_2$,$R_1$ représentant $–C–N–CH_2CH_2$ Hal,
  
  || |
  
  O NO
  
  Hal étant un halogène, notamment Cl,
- Y représente un atome d'hydrogène ou un groupe hydroxy.

Une autre classe préférée de composée selon l'invention est constituée par ceux de formules (I), (II), (III). (IV), (V), (VI) et (VII) et dans lesquelles:

- R représente un groupe alcoyle de 1 à 12 atomes de carbone, un groupe aralcoyle de 7 à 12 atomes de carbone;
- R' ou R" représente OH;
- X représente un groupe $NR_1R_2$, $R_1$ représentant $–C–N–CH_2CH_2$ Hal,
  
  || |
  
  O NO
  
  Hal représentant un halogène, notamment Cl,
- Y représente un atome d'hydrogène.

Une autre classe de composés préférés selon l'invention est constituée par ceux de formule (I), (II), (III). (IV), (V), (VI) et (VII) dans lesquelles:

- R représente un groupe alcoyle de 7 à 12 atomes de carbone, un groupe aralcoyle de 1 à 12 atomes de carbone;

- R' ou R" représente OH;
- X représente un groupe alcoylamino, dans lequel le groupe alcoyle a de 1 à 6 atomes de carbone, ou arylamino dans lequel le groupe aryle a de 4 à 10 atomes de carbone, et
- Y représente $NR'_1NR'_2$, $R'_1$ représentant $-C-N-CH_2CH_2$ Hal,
  || |
  O NO
  Hal représentant un halogène, notamment Cl,

Une autre classe de composés préférés selon l'invention est constituée par ceux de formule (I), (II), (III), (IV), (V), (VI) et (VII) dans lesquelles:

- R représente un groupe alcoyle de 1 à 12 atomes de carbone. un groupe aralcoyle de 7 à 12 atomes de carbone;
- R' ou R" représente OH;
- X représente un groupe hydroxy.
- Y représente $NR'_1$, $R'_2$, $R'_1$ représentant $-C-N-CH_2CH_2$ Hal,
  || |
  O NO
  Hal représentant un halogène, notamment Cl,

Une autre classe de composés préférés selon l'invention est constituée par ceux de formule (I), (II), (III), (IV), (V), (VI) et (VII) dans lesquelles:

- R représente un groupe alcoyle de 1 à 12 atomes de carbone, un groupe aralcoyle de 7 à 12 atomes de carbone,
- R' ou R" représente OH,
- X représente un groupe $NR_1R_2$, $R_1$ représentant $-C-N-CH_2CH_2$ Hal,
  || |
  O NO
  Hal étant un halogène, notamment Cl,

- Y représente un groupe alcoylamino, dans lequel le groupe alcoyle a de 1 à 6 atomes de carbone ou arylamino dans lequel le groupe aryle a de 4 à 10 atomes de carbone.

Une autre classe préférée de composés selon l'invention est constituée par ceux de formules (V), (VI) et (VII) dans lesquelles:

- R représente un groupe alcoyle de 1 à 12 atomes de carbone, aralcoyle de 7 à 12 atomes de carbone;
- R' représente un groupe OM, M étant un groupe alcoyle comprenant de 1 à 12 atomes de carbone, un groupe aryle comprenant de 4 à 10 atomes de carbone;
- X représente un groupe $NR_1R_2$, $R_1$ représentant $-C-N-CH_2CH_2$ Hal,
  || |
  O NO
  Hal étant un halogène, notamment Cl,

- Y représente un atome d'hydrogène ou un groupe hydroxy.

Une autre classe préférée de composés selon l'invention est constituée par ceux des formules (V), (VI) et (VII) dans lesquelles:

- R représente un groupe alcoyle de 1 à 12 atomes de carbone, halogénoaralcoyle de 4 à 10 atomes de carbone;
- R' représente un groupe OM. M étant un groupe acyle de 2 à 8 atomes de carbone, un groupe aroyle de 5 à 12 atomes de carbone;
- X représente un groupe $NR_1R_2$, $R_1$ représentant $-C-N-CH_2CH_2$ Hal,
  || |
  O NO
  Hal étant un halogène, notamment Cl,

- Y représente un atome d'hydrogène ou un groupe hydroxy.

Une autre classe préférée de composés selon l'invention est constituée par ceux de formules (V), (VI) et (VII) et dans lesquelles:

- R représente un groupe alcoyle de 1 à 12 atomes de carbone. un groupe aralcoyle de 7 à 12 atomes de carbone;
- R' représente OH;

- X représente un groupe:$-NR_1R_2$, $R_1$ représentant $-C-N-CH_2CH_2$ Hal,
  || |
  O NO

  Hal étant un halogène, notamment Cl,

- Y représente un atome d'hydrogène.

Une autre classe de composés préférés selon l'invention est constituée par ceux de formule (V), (VI) et (VII) dans lesquelles:

- R représente un groupe alcoyle de 1 à 12 atomes de carbone, un groupe aralcoyle de 7 à 12 atomes de carbone;
- R' représente OH;
- X représente un groupe alcoylamino, dans lequel le groupe alcoyle a de 1 à 6 atomes de carbone, ou arylamino dans lequel le groupe aryle a de 4 à 10 atomes de carbone, et
- Y représente $-NR'_1R'_2$, $R'_1$ représentant $-C-N-CH_2CH_2$ Hal,
  || |
  O NO

  Hal étant un halogène, notamment Cl.

Une autre classe de composés préférés selon l'invention est constituée par ceux de formule (V), (VI) et (VII) dans lesquelles:

- R représente un groupe alcoyle de 1 à 12 atomes de carbone, un groupe aralcoyle de 7 à 12 atomes de carbone;
- R' représente OH;
- X représente un groupe hydroxy
- Y représente $-NR'_1$, $R'_2$, $R'_1$ représentant $-C-N-CH_2CH_2$ Hal,
  || |
  O NO

  Hal étant un halogène, notamment Cl.

Une autre classe de composés préférés selon l'invention est constituée par ceux de formule (V). (VI) et (VII) dans lesquelles:

- R représente un groupe alcoyle de 1 à 12 atomes de carbone, un groupe aralcoyle de 7 à 12 atomes de carbone;
- R' représente OH;
- X représente un groupe $NR_1R_2$, $R_1$ représentant $-C-N-CH_2CH_2$ Hal,
  || |
  O NO

  Hal étant un halogène, notamment Cl.

- Y représente un groupe alcoylamino, dans lequel le groupe alcoyle a de 1 à 6 atomes de carbone ou arylamino dans lequel le groupe aryle a de 4 à 10 atomes de carbone.

Conformément à l'invention, les nouveaux dérivés de nitrosourées de formule générale I sont des tridésoxy-2, 3, 6, α-D-arabinohexopyranosyl-nitrosourées, des didésoxy-2, 3-α -D-arabinohexopyranosyl-nitrosourées, des didésoxy-2, 6-α -D-arabinohexopyranosyl-nitrosourées, des tridésoxy-2, 3, 6, α-L-arabinohexopyranosyl-nitrosourées, des didésoxy-2, 3-α -L-arabinohexopyranosyl-nitrosourées, des didésoxy-2, 6-α -L-arabinohexopyranosyl-nitrosourées, des tridésoxy-3, 4, 6, α-D-xylohexopyranosyl-nitrosourées, des didésoxy-3, 4, α -D-xylohexopyranosyl-nitrosourées, des didésoxy-4, 6, α -D-xylohexopyranosyl-nitrosourées, des tridesoxy-3, 4, 6, α-L-xylohexopyranosyl-nitrosourées, des didésoxy-3, 4, α-L-xylohexopyranosyl-nitrosourées, des didésoxy-4, 6, α-L-xylohexopyranosyl-nitrosourées.

Une classe préférée de composés selon l'invention est constituée par ceux de formule suivante:

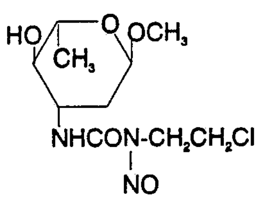

IC 84 1530

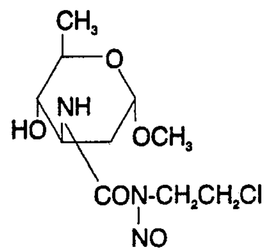

IC 81 1183

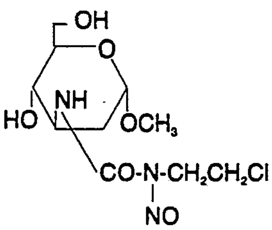

IC 81 1184

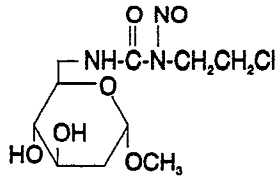

IC 83 1350

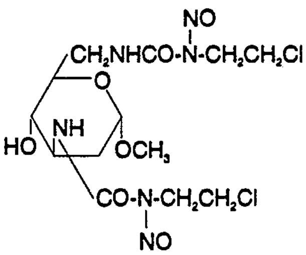

IC 83 1374

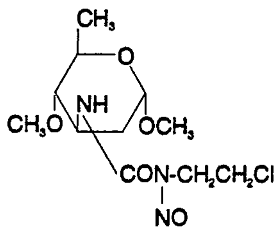

IC 83 1373

La présente invention a également pour objet un procédé de préparation des nouveaux dérivés de formule générale I conformes à l'invention, qui consiste à faire réagir, dans une première étape, un groupe oside de formule générale (I bis)

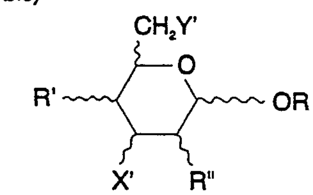

(I bis)

- dans laquelle R, R' et R" ont les significations indiquées ci-dessus.
- x' représente un groupe hydroxy ou $-NHR_2$ ;
- Y' représente un atome d'hydrogène. un groupe hydroxy ou $-NHR'_2$
- $R_2$ et $R'_2$ identiques ou différents, représentent, indépendamment l'un de l'autre, un atome d'hydrogène, un groupe alcoyle de 1 à 6 atomes de carbone, un groupe aralcoyle de 7 à 12 atomes de carbone, aryle de 4 à 10 atomes de carbone, cycloalcoyle de 3 à 6 atomes de carbone, les groupes aryle et aralcoyle étant éventuellement substitués par un ou plusieurs notamment jusqu'à 3, atomes d'halogène, groupes $NO_2$, $NH_2$, $CF_3$ ou groupes alcoxy de 1 à 4 atomes de carbone, et dans laquelle l'un au moins des groupes X' ou Y' représente $-NHR_2$ ou $-NHR'_2$ sur un isocyanate d'halogéno-2-éthyle pour transformer le groupe $-NHR_2$, ou $-NHR'_2$ du composé de formule I bis respectivement en

$NR_2C\ NHCH_2\ CH_2Hal$ ou $NR'_2\ C\ NHCH_2CH_2Hal$,
||                                ||
O                                O

Hal étant un atome d'halogène, notamment le chlore, et dans une deuxième étape, à soumettre le composé obtenu à l'issue de la première étape à une nitrosation, à l'aide d'un nitrite d'un métal alcalin, de préférence le nitrite de sodium, pour transformer les groupes

$NR_2CNHCH_2CH_2Hal$ ou
||
O

$-NR'_2C\ NHCH_2CH_2Hal$ respectivement en
|
O

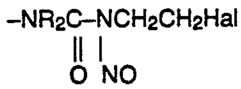
ou

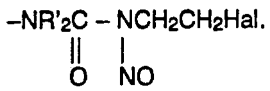

Le procédé décrit ci-dessus peut être illustré par le schéma suivant dans le cas ou X' représente $NHR_2$.

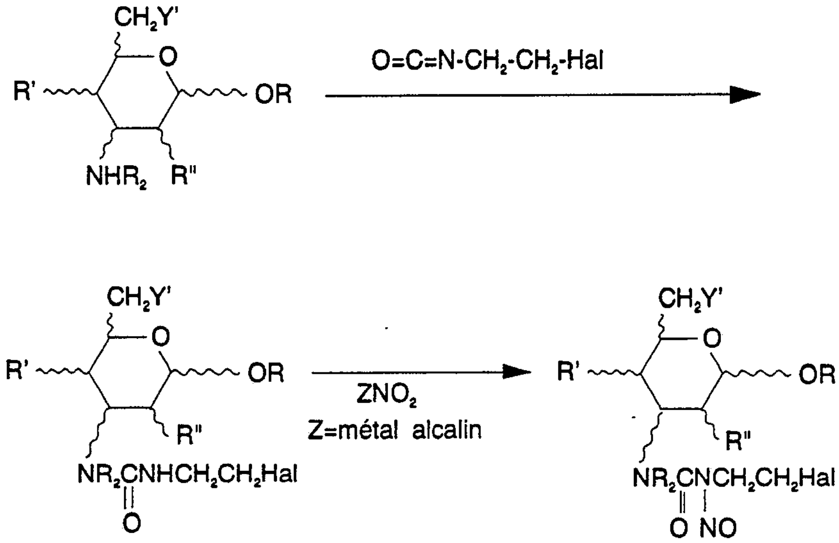


Dans le cas ou Y' représente $-NHR'_2$, le procédé décrit ci-dessus peut être illustré par le schéma suivant:

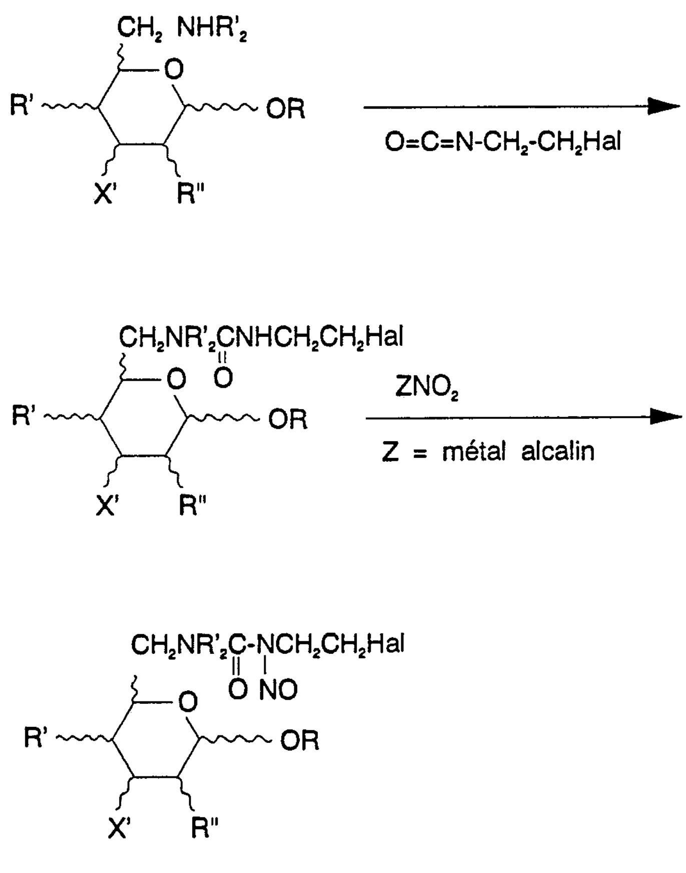

La présente invention a également pour objet un procédé de préparation de nouveaux composés de formule générale VI conformes à l'invention, qui consiste à faire réagir, dans une première étape, un groupe oside de formule générale VI bis:

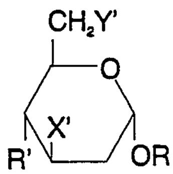

(VI bis)

dans laquelle:

- R et R' sont tels que définis ci-dessus;
- X' représente un groupe $-NHR_2$ ou hydroxy:
- Y' représente un atome d'hydrogène, un groupe hydroxy ou $-NHR'_2$
- $R_2$ et $R'_2$, identiques ou différents, représentent indépendamment l'un de l'autre un atome d'hydrogène, un groupe alcoyle de 1 à 6 atomes de carbone, un groupe aralcoyle de 7 à 12 atomes de carbone, aryle de 4 à 10 atomes de carbone, cycloalcoyle de 3 à 6 atomes de carbone, les groupes aryle et aralcoyle étant éventuellement substitués par un ou plusieurs notamment jusqu'à 3, atomes d'halogène, groupes $NO_2$, $NH_2$, $CF_3$ ou groupes alcoxy de 1 à 4 atomes de carbone,
  et dans laquelle l'un au moins des groupes X' et Y' représente $-NHR_2$ ou $NHR'_2$
  Sur un isocyanate d'halogéno–2–éthyle pour transformer le groupe $-NHR_2$ ou $-NHR'_2$ du composé de formule VI bis respectivement en $NR_2-C-NH-CH_2-CH_2$ Hal ou
  ||
  O

  $NR'_2-C-NH-CH_2-CH_2$ Hal,
  ||
  O
  Hal étant un atome d'halogène, notamment le chlore,
  et dans une deuxième étape à soumettre le composé obtenu à l'issue de l'étape précédente à une nitrosation, à l'aide d'un nitrite d'un métal alcalin, de préférence le nitrite de sodium pour transformer les groupes
  $-NR_2-CNHCH_2-CH_2-$Hal ou $-NR'_2-CNHCH_2-CH_2$ Hal respectivement en
  || ||
  O O

  $-NR_2-C-N-CH_2-CH_2-$Hal ou $-NR'_2-C-N-CH_2-CH_2$ Hal.
  || | || |
  O NO O NO

  La présente invention a également pour objet un procédé de préparation de nouveaux composés de formule générale VII conformes à l'invention, qui consiste à faire réagir, dans une première étape, un groupe oside de formule générale VII bis:

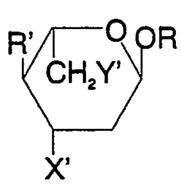

(VII bis)

dans laquelle:

- R et R' sont tels que définis ci-dessus;
- R' représente un groupe $-NHR_2$ ou hydroxy;
- Y' représente un atome d'hydrogène, un groupe hydroxy ou $-NHR'_2$
- $R_2$ et $R'_2$,identiques ou différents, représentent indépendamment l'un de l'autre un atome d'hydrogène, un groupe alcoyle de 1 à 6 atomes de carbone, un groupe aralcoyle de 7 à 12 atomes de carbone, aryle de 4 à 10 atomes de carbone, cycloalcoyle de 3 à 6 atomes de carbone, les groupes aryle et aralcoyle étant éventuellement substitués par un ou plusieurs notamment jusqu'à 3, atomes d'halogène, groupes $NO_2$, $NH_2$, $CF_3$ ou groupes alcoxy de 1 à 4 atomes de carbone,
  et dans laquelle l'un au moins des groupes X' et Y' représente $-NHR_2$ ou $NHR'_2$
  Sur un isocyanate d'halogéno–2–éthyle pour transformer le groupe $-NHR_2$ ou $-NHR'_2$ du composé de formule VII

bis respectivement en

NR2–C–NH–CH2–CH2Hal ou NR'2–C–NH–CH2–CH2 Hal. Hal étant un
        ||                                  ||
        O                                   O
atome d'halogène, notamment le chlore,
et dans une deuxième étape à soumettre le composé obtenu à l'issue de l'étape précédente à une nitrosation, à l'aide d'un nitrite d'un métal alcalin, de préférence le nitrite de sodium pour transformer les groupes
–NR2CNCH2CH2Hal ou –NR'2CNHCH2CH2 Hal respectivement en
        ||                          ||
        O                           O

–NR2–C–N–CH2–CH2Hal ou –NR'2–C–N–CH2–CH2Hal
        || |                         || |
        O NO                         O NO

Pour préparer les composés de formule (I bis), qui entrent dans la préparation des composés selon l'invention, on peut avoir recours à l'un des procédés décrits ci-dessous.

**I Préparation des composés de formule**

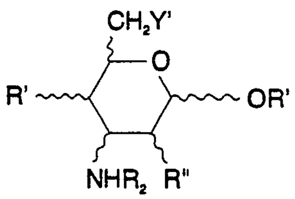

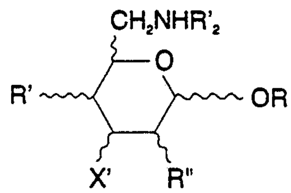

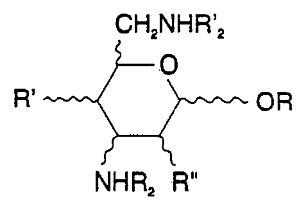

**IA Première variante**

Les composés de formule

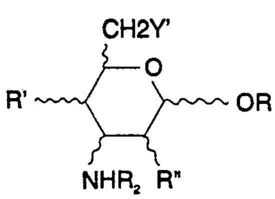

R, R', R", R2 et Y' ayant les significations précédemment indiquées, qui entrent dans la préparation des composés de formule

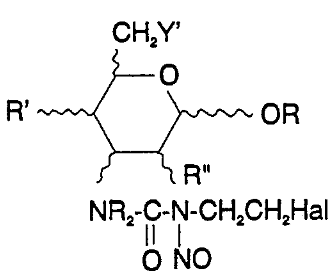

Hal = halogène

de l'invention, peuvent être obtenus à partir des composés de formule

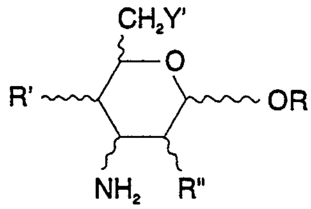

lesquels sont soumis

- dans une première étape, à l'action d'un aldéhyde $R_2C$ H,
  ||
  O

  pour transformer l'amine en imine
- dans une seconde étape, à l'action d'un agent réducteur, tel qu'un hydrure, par exemple le borohydrure ou le cyanoborohydrure de sodium, pour transformer l'imine en amine secondaire.

Le procédé qui vient d'être décrit peut également s'appliquer à l'obtention des composés de formule

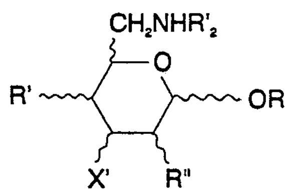

dans lesquels R, R', R", $R'_2$, X' ont les significations indiquées ci-dessus,
ainsi qu'à l'obtention des composés de formule

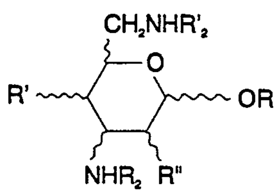

et dans laquelle $R_2=R'_2$
Dans ce dernier cas, les quantités d'aldéhyde et d'agent réducteur mises en oeuvre sont doublées.

**IB Deuxième variante**

Les composés de formule

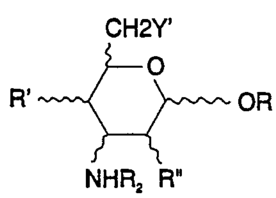

R, R', R", Y' et $R_2$ ayant les significations précédemment indiquées qui entrent dans la préparation des composés selon l'invention de formule

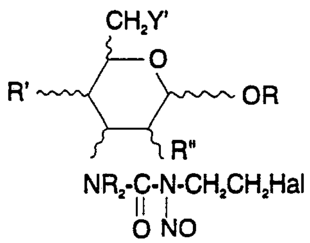

Hal = halogène

peuvent être également prépares à partir des composés de formule

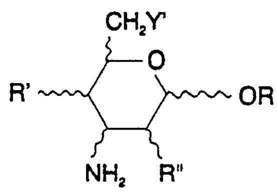

lesquels sont soumis à l'action d'un halogénioformiate d'alcoyle, de formule $Hal\underset{\underset{O}{\|}}{C}OR_2$, notamment un chloroformiate d'alcoyle pour donner les composés de formule

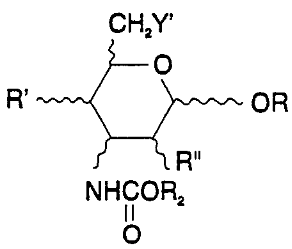

ces composés étant ensuite soumis à un agent réducteur, par exemple l'hydrure d'aluminium et de lithium, pour tranformer le groupe $NH\underset{\underset{O}{\|}}{C}OR_2$ en $NHR_2$.

Ce procédé peut également s'appliquer à l'obtention des composés de formule

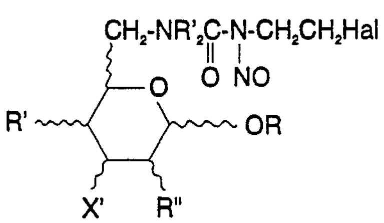

ainsi qu'à ceux de formule

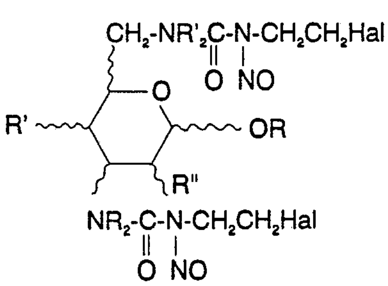

**II Préparation des composés de formule**

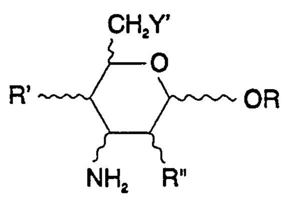
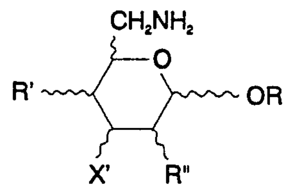
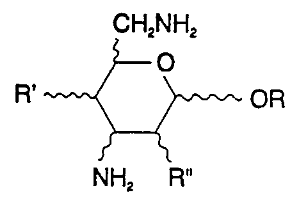

Les composés de formule

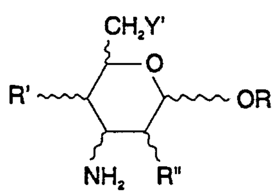

dans lesquels R, R' et R" ont les significations indiquées ci-dessus et Y' représente un groupe hydroxy peuvent être obtenus à partir de composés de formule

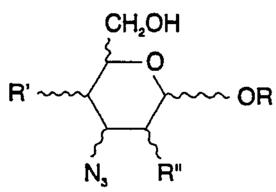

lesquels sont réduits, notamment par hydrogénation catalytique, par exemple hydrogénation en présence de charbon palladié.

Les composés de formule

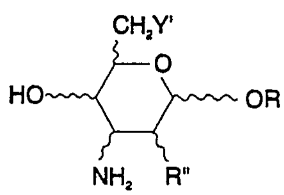

dans lesquels Ret R" ont les significations indiquées ci-dessus et Y' représente un groupe hydroxy, peuvent être obtenus à partir des composés de formule

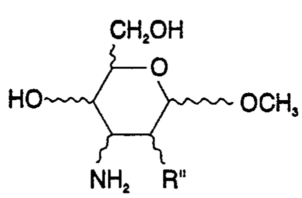

dans lesquels on protège l'un des atomes d'hydrogène du groupe $-NH_2$ en position 3, notamment en faisant réagir les composés précédemment représentés avec de l'anhydride trifluoroacétique pour donner les composés de formule

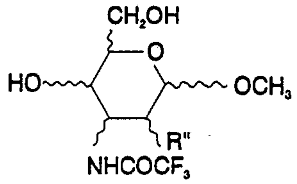

lesquels sont traités par un alcool ROH, en milieu acide, pour donner les composés de formule

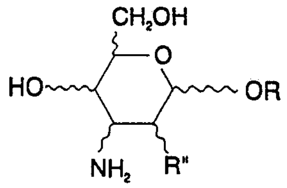

le groupe protectueur $-COCF_3$ étant simultanément hydrolysé.

Les composés de formule

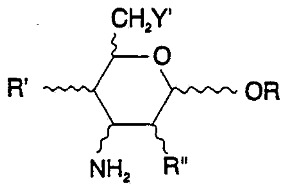

dans lesquels R, R' et R" ont les significations indiquées ci-dessus et Y' représente un atome d'hydrogène, peuvent être obtenus à partir des composés de formule

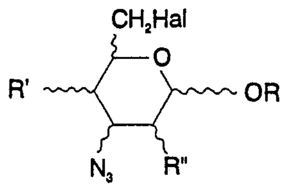

dans lesquels R, R' et R" ont les significations indiquées ci-dessus et Hal représente un halogène, notamment le brome, ces composés étant réduits, notamment par hydrogénation catalytique, par exemple en présence de charbon palladié

Les composés de formule

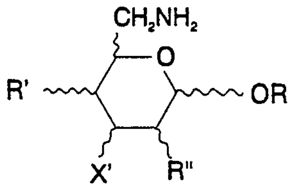

dans lesquels R, R' et R" ont les significations indiquées ci-dessus et X' représente un groupe hydroxy, peuvent être obtenus à partir de composés de formule

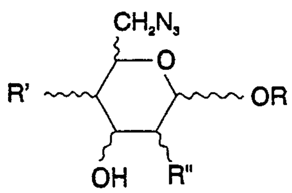

par réduction, notamment par hydrogénation catalytique, par exemple en présence de charbon palladié.

Les composés de formule

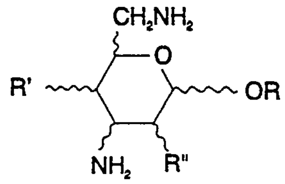

dans lesquels R, R' et R" ont les significations indiquées ci-dessus peuvent être obtenus à partir de composés de formule

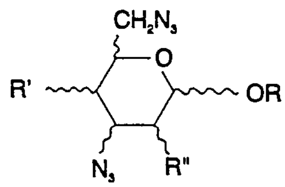

lesquels sont soumis à une réduction notamment une hydrogénation catalytique, par exemple en présence de charbon palladié.

**III Préparation des composés de formule**

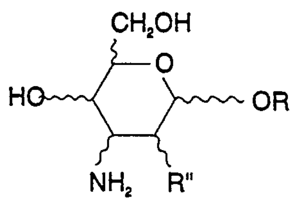

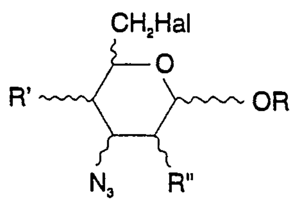

Hal = halogène, notamment Br

Les composés de formule

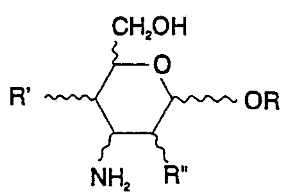

dans lesquels R, R' et R" ont les significations indiquées ci-dessus peuvent être obtenus à partir de composés de formule

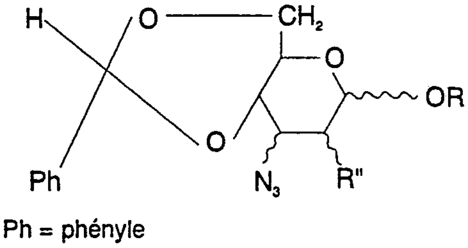

Ph = phényle

lesquels sont soumis à l'action du chlorure d'acétyle, puis à une neutralisation par de l'ammoniac pour donner les composés de formule

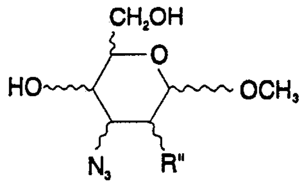

lesquels sont réduits, notamment par hydrogénation catalytique, par exemple en présence de charbon palladié, pour donner les composés de formule

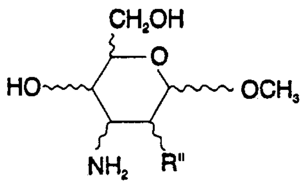

lesquels peuvent être transformés par action d'un alcool ROH, en milieu acide, en composés de formule

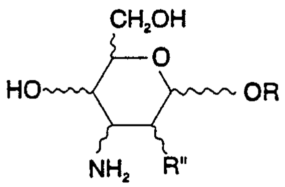

Les composés de formule

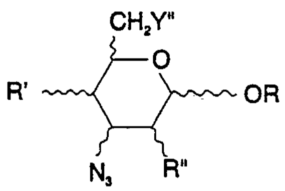

dans lesquels R, R' et R" ont les significations indiquées ci-dessus et Y" représente un atome d'halogène, notamment le brome, peuvent être obtenus à partir des composés, de formule

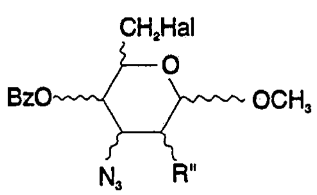

Bz = benzoyle

lesquels sont soumis à un alcool de formule ROH, en milieu acide pour donner les composés de formule

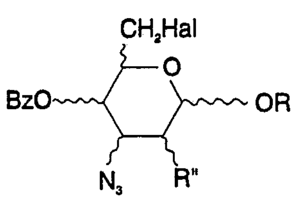

lesquels sont ensuite transformés en composés de formule

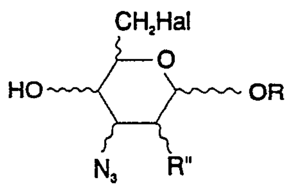

par élimination du groupe benzoyle, par exemple à l'aide d'une base, notamment un alcoolate alcalin, tel que le méthylate de sodium.

Les composés obtenus ci-dessus peuvent ensuite être traités par un agent d'alcoylation, tel que $M_2SO_4$ ou MX, X représentant un halogène, M représentant un groupe alcoyle de 1 à 30, de préférence de 1 à 12 atomes de carbone, un groupe aryle de 6 à 12 atomes de carbone, un groupe aralcoyle de 7 à 12 atomes de carbone, de préférence 7 à 9 atomes de carbone, les groupes aryle et aralcoyle étant éventuellement substitués par un ou plusieurs, notamment jusqu'à 3 atomes d'halogène, groupes $NO_2$, $NH_2$, $CF_3$, ou alcoxy de 1 à 4 atomes de carbone, ou représentant un groupe acyle de 2 à 8 atomes de carbone, de préférence 2 ou 3, un groupe aroyle de 5 à 12, de préférence 5 à 9 atomes de carbone, non substitués ou substitués par un ou plusieurs, notamment jusqu'à 3 groupes $NO_2$, $NH_2$, $CF_3$ halogène, alcoxy de 1 à 4 atomes de carbone, en présence d'une base telle que NaOH, pour conduire au composé de formule

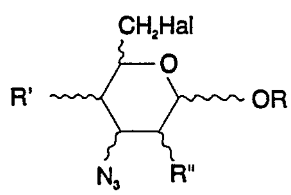

avec R'= MO M ayant la signification indiquée cidessus

**IV Préparation des composés de formule**

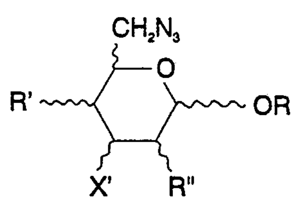

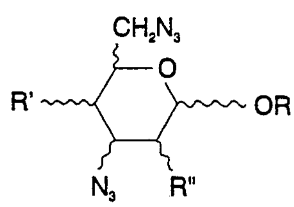

Les composés de formule:

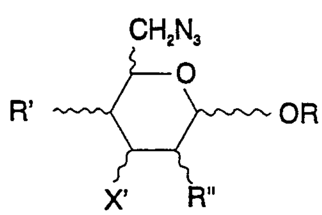

dans lesquels R, R' et R" ont les significations indiquées ci-dessus et X' représente un groupe OH, peuvent être obtenus à partir des composés de formule

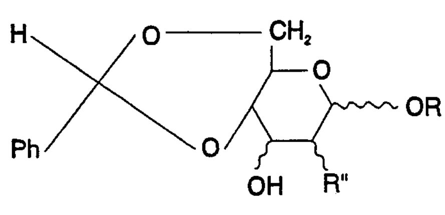

dans lesquels les fonctions OH en 4 et 6 sont protégées, en faisant réagir les composés ci-dessus décrits, par exemple

sur de l'anhydride acétique, en présence de pyridine pour protéger le groupe hydroxy en 3, pour donner les composés de formule

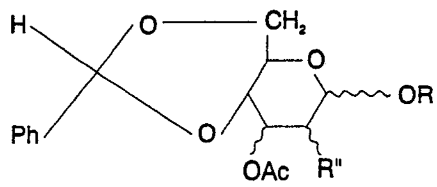

Ac = acétyle

lesquels en présence de N-halogéno succinimide, de préférence Nbromosuccinimide, et de carbonate de baryum donnent les composés de formule

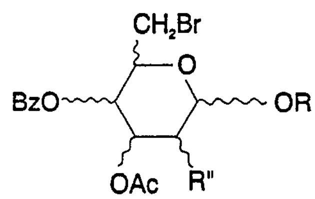

Bz = benzoyle

lesquels sont soumis à un azoture, notamment d'un métal alcalin, tel que le sodium, en présence de diméthylformamide, pour donner les composés de formule

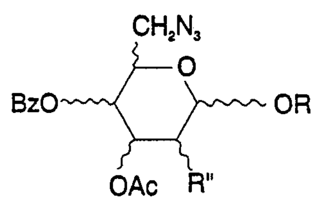

lesquels en présence d'une base, notamment un alcoolate alcalin, tel que le méthylate de sodium donnent

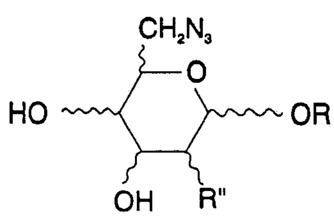

lesquels peuvent être soumis à $M_2SO_4$ ou MX (X= halogène) en présence d'une base telle que NaOH pour donner les composés de formule

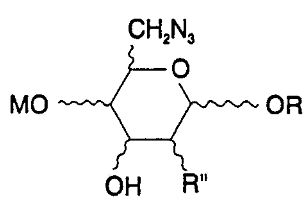

avec MO = R'
M ayant la signification indiquée ci-dessus

Les composés de formule

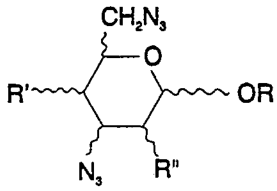

dans lesquels R, R' et R" ont les significations indiquées ci-dessus peuvent être obtenus à partir des composés de formule

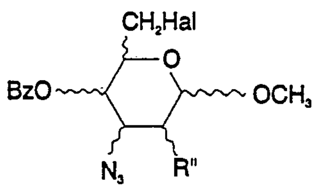

dans lesquels Hal représente un atome d'halogène notamment, le brome, lesquels sont soumis à la réaction d'un azoture, notamment, d'un métal alcalin, tel que l'azoture de sodium, pour donner les composés de formule

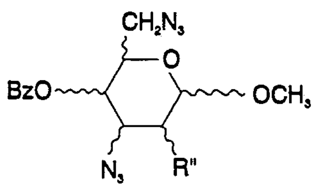

dans lesquels, le groupe benzoyle est éliminé, par exemple, par addition d'une base, notamment d'un alcoolate alcalin tel que le méthylate de sodium, pour donner les composés de formule

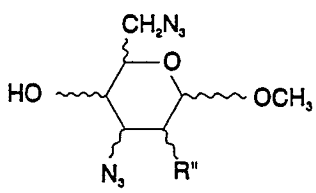

Ces composés peuvent être transformés en composés de formule:

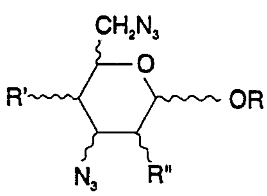

en protégeant le groupe hydroxy en 4, notamment à l'aide du chlorure de benzoyle pour obtenir les composés de formule

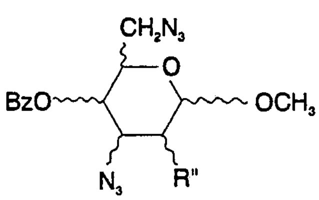

puis en additionnant un alcool de formule R OH, en milieu acide, pour obtenir les composés de formule

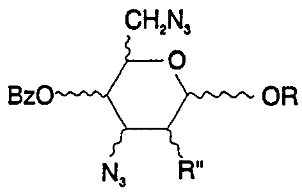

puis en éliminant le groupe protecteur de la fonction hydroxy en 4, notamment à l'aide d'une base, notamment un alcoolate alcalin tel que le méthylate de sodium, pour obtenir les composés de formule

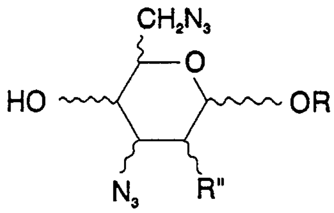

puis en alcoylant les composés ci-dessus, notamment à l'aide de $M_2SO_4$ ou MX, X représentant un halogène représentant un groupe alcoyle de 1 à 30, de préférence 1 à 12 atomes de carbone, un groupe aryle de 4 à 10 atomes de carbone, un groupe aralcoyle de 7 à 12 atomes de carbone, de préférence 7 à 9 atomes de carbone, les groupes aryle et aralcoyle étant éventuellement substitués par un ou plusieurs, notamment jusqu'à 3 atomes d'halogène, groupes $NO_2$, $NH_2$, $CF_3$ ou alcoxy de 1 à 4 atomes de carbone, ou représentant un groupe acyle de 2 à 8 atomes de carbone, de préférence 2 ou 3, un groupe aroyle de 5 à 12, de préférence 5 à 9 atomes de carbone, non substitués ou substitués par un ou plusieurs, notamment jusqu'à 3 groupes $NO_2$, $NH_2$, $CF_3$ halogène, alcoxy de 1 à 4 atomes de carbone, en présence d'une base telle que NaOH, pour conduire aux composés de formule

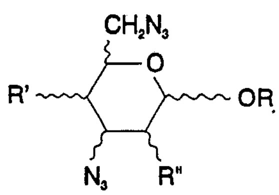

avec R' = MO
M ayant la signification indiquée ci-dessus

Les procédés qui viennent d'être décrits ci-dessus s'appliquent avantageusement à la préparation des composés susceptibles d'être utilisés pour la synthèse des composés de formule VI selon l'invention.

Plus précisément, le procédé décrit dans le paragraphe I, permet de préparer les composés de formule

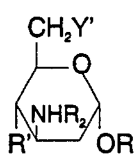

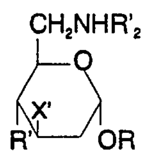

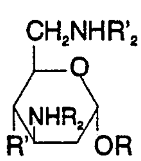

dans lesquels R, R', X', Y', $R_2$ et $R'_2$ ont les significations indiquées ci-dessus

Le procédé décrit dans le paragraphe II permet de préparer les composés de formule

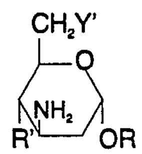

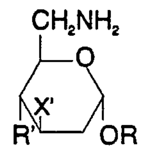

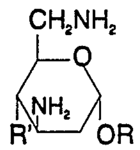

dans lesquels R, R', X' et Y" ont les significations indiquées ci-dessus

Le procédé décrit dans le paragraphe III permet de synthétiser les composés de formule

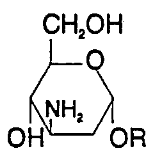

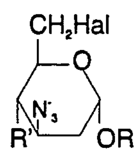

dans lesquels R, R' et Hal ont les significations indiquées ci-dessus

Le procédé décrit dans le paragraphe IV permet de préparer les composés de formule

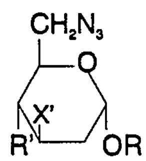

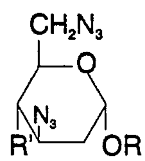

dans lesquels R, R', X' ont les significations indiquées ci-dessus

Selon un mode de réalisation préféré du procédé conforme à l'invention, on prépare les composés de formule générale VIbis, pour obtenir les composés de formule VI conformes à l'invention, en faisant réagir un α-D-arabinohexopyranoside de formule générale VIter ci-après:

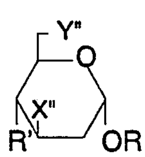

VI ter

dans laquelle R et R' sont tels que définis plus haut, X" est un groupe azide, hydroxy, $NH_2$ ou un groupe alcoylamine, dont le radical alcoyle comporte de 1 à 6 atomes de carbone,

Y" peut représenter un halogène, lorsque X" représente un groupe azide ou hydroxy, ou Y" peut représenter un hydrogène, un groupe azide, un groupe $NH_2$, un groupe hydroxy ou un groupe $NHR'_2$ où $R'_2$ est un atome d'hydrogène ou un groupe alcoyle de 1 à 6 atomes de carbone, aralcoyle de 7 à 12 atomes de carbone, aryle de 4 à 10 atomes de carbone ou cycloalcoyle de 3 à 6 atomes de carbone, les groupes aryle et aralcoyle étant éventuellement substitués par un ou plusieurs, notamment jursqu'à 3, atomes d'halogène, groupes $NO_2$, $NH_2$' $CF_3$ ou groupes alcoxy de 1 à 4 atomes de carbone,

a) soit avec un alcool en milieu acide, dans le cas où X" est un groupe azido et Y" un atome d'halogène, d'hydrogène ou un groupe hydroxy ou dans le cas où X" est un groupe hydroxy et Y" un groupe azide. R est un groupe alcoyle de 1 à 12 atomes de carbone, aralcoyle de 7 à 12 atomes de carbone ou halogénoalcoyle de 1 à 12 atomes de carbone et R' représente OM, M étant un groupe acyle de 2 à 8 atomes de carbone ou arylester de 6 à 12 atomes de carbone et où 1'α-D-arabinohexopyranoside est désoxyle en 2,3,6-, le composé obtenu étant ensuite réduit, notamment par hydrogénation catalytique, par exemple en présence de de charbon palladié pour transformer le

groupe azido en $NH_2$,

b) soit avec un agent d'alcoylation dans le cas ou X" est un groupe azido. Y" est un atome d'halogène ou d'hydrogène. R' est un groupe OH et R est un groupe alcoyle de 1 à 12 atomes de carbone ou aralcoyle de 7 à 12 atomes de carbone et où 1'α-D-arabinohexopyranoside est tridésoxylé en 2, 3, 6-, le composé obtenu étant ensuite réduit, notamment par hydrogénation catalytique, par exemple en présence de charbon palladié, pour transformer le groupe azido en groupe $NH_2$,

c) soit avec de l'anhydride trifluoroacétique, dans le cas ou X" est un groupe $NH_2$ et Y" un groupe hydroxy ou un atome d'hydrogène ou dans le cas où X" est un groupe hydroxy et Y" est un groupe $NH_2$, R est un groupe alcoyle de 1 à 12 atomes de carbone ou aralcoyle de 7 à 12 atomes de carbone et R' un groupe hydroxy, le dérivé trifluoro acétamido 3-α-D-arabinohexopyranoside obtenu étant traité par un alcool en milieu acide pour obtenir le composé amino-3 de formule générale correspondant et où 1'α-D-arabinohexopyranoside est didésoxylé en 2, 3, ou tridésoxylé en 2, 3, 6-;

d) soit avec un aldéhyde en milieu alcoolique, dans le cas où X" est un groupe $NH_2$, Y" est un atome d'hydrogène, un groupe $NH_2$ ou $NHR'_2$ ou un groupe hydroxy ou dans le cas où X" est un groupe hydroxy et Y" un groupe $NH_2$ ou $NHR'_2$ et R et R' sont tels que définis plus haut, pour obtenir respectivement le composé désoxy-3 imino-3 et/ou le compose désoxy-6 imino-6 correspondant, qui par réduction par un agent réducteur approprié tel que le borohydrure ou le cyanoborohydrure de sodium donne l'amine qui permet ensuite de conduire à 1'α-D-arabinohexopyranosyl-3-nitrosourée de formule générale VI correspondante, 2, 3 -ou 2, 6 - didésoxylée ou 2, 3, 6 - tridésoxylée.

Pour préparer les α-D-arabinohexopyranosides-nitrosourées selon l'invention, on peut procédé de préférence comme suit:

**Variante a) du procédé:**

A une solution de méthyl azido-3 O-acyl (ou arylester)-4 bromo-6 tridésoxy-2,3,6 α-D-arabinohexopyranoside, 0,01 mole, dans 200 ml d'hexane, on ajoute 10 ml d'alcool (éthanol, alcool benzylique ou autre) et 2 g d'acide paratoluènesulfonique.

La solution est chauffée au reflux durant 12 à 48 heures. Après refroidissement, le milieu réactionnel est versé sur une solution saturée de bicarbonate de sodium, puis extrait par de l'éther. La phase organique est évaporée à sec et donne un résidu brut qui est chromatographié sur silice pour donner l'éther de formule générale I correspondant à l'alcool.

**Variante b) du procédé:**

On met un alcoyl- ou aralcoyl-azido-3 bromo-6 tridésoxy-2,3,6 α-D-arabinohexopyranoside, et de préférence le méthyl-azido-3-bromo-6 tridésoxy-2,3,6–α-D-arabinohexopyranoside, 0,01 mole, en solution dans un solvant approprié tel que du tétrahydrofurane anhydre (100 ml) ou dans un autre solvant tel que dioxane, éther isopropylique, etc... On ajoute 5 -10 g de NaOH, puis 5 à 10 g d'un agent d'alcoylation tel qu'un sulfate d'alcoyle, un halogénure d'alcoyle, un halogénure d'aralcoyle, par exemple. La suspension ainsi obtenue est chauffée au reflux durant 12 à 48 heures. Après refroidissement et addition lente d'eau, on agite à 20°C pendant 2 heures. La phase organique est soutirée par décantation, puis la phase aqueuse est à nouveau extraite par 100 ml de tétrahydrofurane. La phase organique, séchée sur du sulfate de sodium, est évaporée à sec. On obtient de 60 à 90 % du composé désiré, qui est purifié par chromatographie sur silice.

**Variante c) du procédé:**

On fait agir de l'anhydride trifluoroacétique sur le méthyl amino-3 tridésoxy-2,3,6 α-D-arabinohexopyranoside [préparé suivant la méthode de J. BOIVIN et Coll., Carb. Res. 85 (1980) 223 - 42] pour obtenir le méthyl-trifluoro acétamido-3-α-D-arabinohexopyranoside (le groupe méthyle pouvant être remplacé par un autre groupe alkyle ou un groupe aralkyle, tel que défini plus haut).

A une solution de 0,01 mole du dérivé trifluoroacétamido-3 ainsi obtenu, dans 200 ml de n-hexane, on ajoute 5 à 20 ml d'alcool et 1 à 3 g d'acide p-toluènesulfonique. En poursuivant le traitement comme décrit dans la 1ère variante du procédé, on obtient l'éther de formule générale I correspondant à l'alcool, après avoir libéré l'amine protégée, à l'aide de carbonate de potassium, en milieu alcoolique aqueux.

**Variante d) du procédé:**

On fait agir un aldéhyde sur l'amine correspondante (Cf. 3ème variante du procédé) comme suit, pour obtenir l'imine correspondante: une solution de 0,01 mole d'aldéhyde dans l'éthanol ou le méthanol est ajoutée goutte à goutte, à la température ambiante, à une solution de 0,01 mole de l'amine qui constitue le composé de départ de la 3ème variante du procédé, dans de l'éthanol ou du méthanol anhydre. La solution est chauffée au reflux pendant 2 à 12 heures. Après

refroidissement, on ajoute, par petites portions, de 0,01 à 0,05 ml d'un agent réducteur approprié tel que le borohydrure ou le cyanoborohydrure de sodium, et on agite pendant 12 heures. Après évaporation du solvant, le résidu est repris dans 20 ml d'eau, le précipité est essoré, puis séché sous vide sous $P_2O_5$. L'amine secondaire obtenue est recristallisée dans des alcools.

Le processus est le même dans le cas où le groupe amino-3 est remplacé, dans le composé de départ, par un groupe hydroxy-3, le groupe amino étant en 6. Dans le cas où le composé recherché doit comporter une substitution par une amine secondaire non seulement en 3- mais également en 6-, les quantités d'aldéhyde et d'agent réducteur mises en oeuvre sont doublées.

Les quatre variantes du procédé décrites ci-dessus sont illustrées par les schémas ci-dessous:

**I - Schéma de la variante a) du procédé: Ethérification**

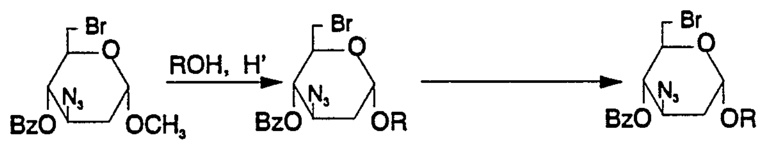


R = Ethyl, benzyl, etc ...

**II - Schéma de la variante b) du procédé:**

et {MO = R'
R et M = méthyl, éthyl, benzyl, etc...

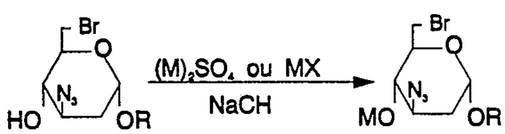


**III - Schéma de la variante c) du procédé avec blocage de l'amine en 3**

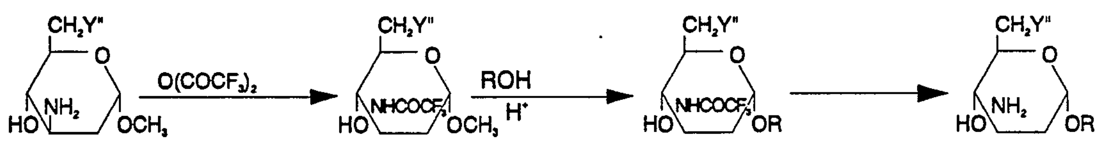


**VI - Schéma de la variante d) du procédé**

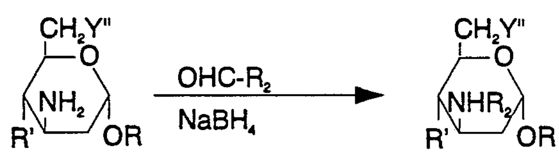


si Y = $NH_2$ :

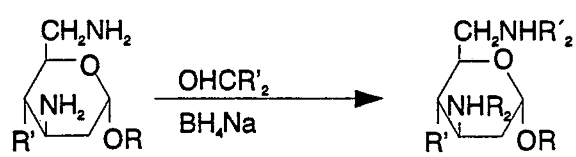


avec $R_2$ = $R'_2$

Pour préparer les α-L-arabinohexopyranosides-nitrosourées selon l'invention, de formule VII:

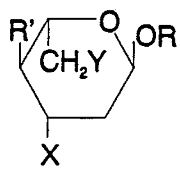

et dans lesquels le groupe nitroso est sur le carbone 3, et R, R', X et Y ont les significations indiquées ci-dessus on peut avantageusement procéder comme suit. On traite le composé de formule suivante:

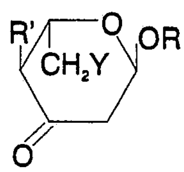

par du chlorhydrate de O-méthylhydroxylamine et de l'acétate de sodium pour obtenir le composé de formule suivante:

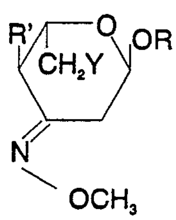

En traitant le composé précédemment obtenu par du diborane et des ions $OH^-$, on obtient le composé de formule:

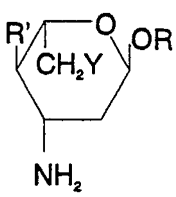

Ce composé est ensuite traité par de l'isocyanate de chloro-2 éthyle, pour donner le composé de formule:

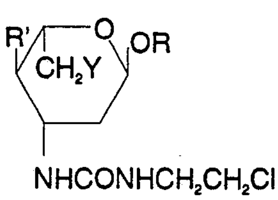

Ce composé est ensuite dissous, par exemple dans l'acide formique, puis traité avec du nitrite de sodium, pour donner le composé de formule:

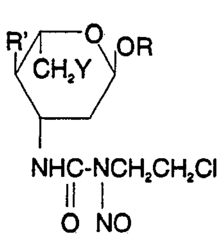

Pour préparer les α-D-xylohexopyranosides-nitrosourées de l'invention de formule III, notamment ceux comportant un groupe nitrosourée en position 3, en particulier le composé de formule suivante:

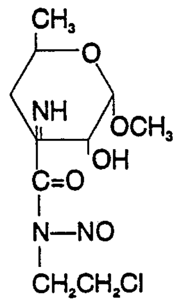

on peut utiliser le composé de formule suivante:

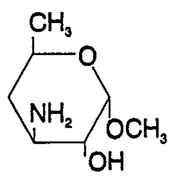

qui dans une première étape, est traité par l'isocyanate de chloro-2 éthyle, pour donner l'urée correspondante, c'est-à-dire le méthyl[(chloro-2 éthyl)-3 ureido]-3-tridésoxy-3-4-6, α-D xylohexopyranoside de formule:

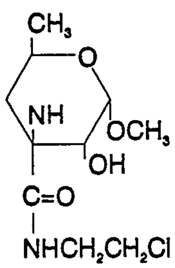

Le composé, ci-dessus indiqué, par traitement au nitrite de sodium dans l'acide formique conduit à la nitrosourée correspondante, c'est-à-dire au méthyl [(chloro-2 éthyl)-3 nitroso-3 ureido]-3 tridésoxy-3,4,6 α-D-xylohexopyranoside de formule:

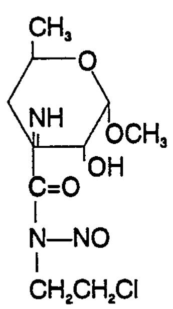

Tous les composés qui sont susceptibles d'être utilisés dans la préparation des composés de formule I, notamment de formule VI, peuvent être préparés en appliquant l'une ou l'autre des séquences réactionnelles décrites ci-dessus, ainsi que les séquences réactionnelles appropriées décrites dans les exemples donnés ci-après, à titre illustratif.

Outre les dispositions qui précèdent, l'invention comprend encore d'autres dispositions, qui ressortiront de la description qui va suivre.

L'invention vise plus particulièrement les nouveaux dérivés de nitrosourées conformes aux dispositions qui précèdent et leurs procédés de préparation, ainsi que les compositions, en particulier des compositions thérapeutiques, dans lesquelles entrent lesdits dérivés.

L'invention sera mieux comprise à l'aide du complément de description qui va suivre, qui se réfère à des exemples de mise en oeuvre du procédé objet de la présente invention.

Il doit être bien entendu, toutefois, que ces exemples de mise en oeuvre sont donnés uniquement à titre d'illustration de l'objet de l'invention, dont ils ne constituent en aucune manière une limitation.

**Exemples**

**Exemple 1**

Préparation du benzyl azido-3 O-benzoyl-4 bromo-6 tridésoxy 2,3,6 α-D-arabinohexopyranoside en mettant en oeuvre la première variante du procédé.

A une solution de méthyl azido-3 O-benzoyl-4 bromo-6 tridésoxy-2,3,6 α-D-arabinohexopyranoside (1,5 g, 4,05 mmol.) dans 125 ml de n-hexane, on ajoute 10 ml d'alcool benzylique et 1 g d'acide p-toluènesulfonique. La suspension est chauffée au reflux durant 20 heures avec élimination azéotropique du méthanol libéré.

Après refroidissement, le milieu réactionnel est versé sur une solution saturée de bicarbonate de sodium puis extrait par de l'éther. L'excès d'alcool benzylique est ensuite éliminé par entraînement azéotropique par de l'eau, puis du toluène. Le résidu brut ainsi obtenu (1,8 g) est chromatographié sur silice H avec comme éluant le mélange hexane/chlorure de méthylène, 2 : 1.

On isole 1,5 g de benzyl azido-3 O-benzoyl-4 bromo-6 tridésoxy 2,3,6 α-D-arabinohexopyranoside (84 %).

F : 77°C (hexane) - $[\alpha]^{20}_D$: + 20° (c : 1 %; $CHCl_3$)

$IR_{nujol}$: ν 2100 $cm^{-1}$ ($N_3$)
ν 1760, 1260, 1030 $cm^{-1}$ (ester)
ν 1600, 1585 $cm^{-1}$ (aromatiques)

On prépare de la même manière, par exemple:

- L'éthyl azido-3 O-benzoyl-4 bromo-6 tridesoxy-2,3,6 α-D-arabinohexopyranoside.
- Le p-chlorobenzyl azido-3 O-benzoyl-4 bromo-6 tridésoxy 2,3,6 α-D-arabinohexopyranoside.

**Exemple 2**

Mise en oeuvre de la 2ème variante du procédé. On prépare par alkoylation d'un alkyl-ou aralkyl-azido-3 bromo-6, tridésoxy-2,3,6 α-D-arabinohexopyranoside, les composés suivants, cités à titre d'exemples:

. Le méthyl azido-3 bromo-6 tridésoxy-2,3,6 O-éthyl-4 α-D-arabinohexopyranoside
. Le méthyl azido-3 bromo-6 tridésoxy-2,3,6 O-benzyl-4 α-D-arabinohexopyranoside
. L'éthyl azido-3 bromo-6 tridésoxy-2,3-6 O-éthyl-4 α-D-arabinohexopyranoside
. L'éthyl azido-3 bromo-6 tridésoxy-2,3-6 O-benzyl-4 α-D-arabinohexopyranoside
. Le benzyl azido-3 bromo-6 tridésoxy-2,3,6 O-éthyl-4 α-D-arabinohexopyranoside
. Le benzyl azido-3 bromo-6 tridésoxy-2,3,6 O-benzyl-4 α-D-arabinohexopyranoside.

**Exemple 3**

Mise en oeuvre de la 3ème variante du procédé
A une solution de (300 mg, 1,16 mmol.) méthyl trifluoroacétamido-3 tridésoxy-2,3,6 α-D-arabinohexopyranoside dans 100 ml de n-hexane, on ajoute 5 ml d'alcool benzylique et 300 mg d'acide p-toluène sulfonique sec, et on traite comme décrit à l'Exemple 1.

On isole, après chromatographie et cristallisation dans un mélange hexane-acétone, 100 mg (30 %) de produit pur.

F: 165°C - $[\alpha]_D$ : + 66° (c : 0,5 %, $CHCl_3$).

L'amine est ensuite libérée par l'action du carbonate de potassium en milieu méthanolique aqueux.

En procédant de la façon qui vient d'être décrite, on prépare, notamment, les composés suivants, cités à titre d'exemples:

. Le benzyl amino-3 tridésoxy-2,3,6 α-D-arabinohexopyranoside
. L'éthyl amino-3 tridésoxy-2,3,6 α-D-arabinohexopyranoside
. Le benzyl amino-3 didésoxy-2,3, α-D-arabinohexopyranoside
. L'éthyl amino-3 didésoxy-2,3 α-D-arabinohexopyranoside.

**Exemple 4**

Mise en oeuvre de la 4ème variante du procédé. En procédant conformément à la 4ème variante du procédé on peut préparer, entre autres, les composés suivants, donnés à titre d'exemples:

. Le méthyl éthylamino-3 tridésoxy-2,3,6, α-D-arabinohexopyranoside
. Le méthyl benzylamino-3 tridésoxy-2,3,6 α-D-arabinohexopyranoside

- Le méthyl éthylamino-3 didésoxy-2,3 α-D-arabinohexopyranoside
- Le méthyl benzylamino-3 didésoxy-2,3 α-D-arabinohéxopyranoside
- Le benzyl éthylamino-3 tridésoxy-2,3,6 α-D-arabinohexopyranoside
- Le benzyl benzylamino-3 tridésoxy-2,3,6 α-D-arabinohexopyranoside
- Le méthyl éthylamino-6 didésoxy-2,6 α-D-arabinohexopyranoside
- Le méthyl benzylamino-6 didésoxy-2,6 α-D-arabinohexopyranoside
- Le benzyl éthylamino-6 didésoxy-2,6 α-D-arabinohexopyranoside
- Le benzyl benzylamino-6 didésoxy-2,6 α-D-arabinohexopyranoside.

En doublant les quantités d'aldéhyde et d'agent réducteur mises en oeuvre, on prépare les amines secondaires suivantes substituées en 3,6:

- Le méthyl diéthylamino-3,6 tridésoxy-2,3,6 α-D-arabinohexopyranoside
- Le méthyl dibenzylamino-3,6 tridésoxy-2,3,6 α-D-arabinohexopyranoside
- Le benzyl diéthylamino-3,6 tridésoxy-2,3,6 α-D-arabinohexopyranoside
- Le benzyl dibenzylamino-3,6 tridésoxy-2,3,6 α-D-arabinohexopyranoside.

**Exemple 5**

**Méthyl [(chloro-2 éthyl)-3 uréido] 3-tridésoxy-2,3,6 α-D-arabinohexopyranoside - Composé 1**

A une solution de 0,8 g ($5.10^{-3}$ mole) de méthyl amino-3 tridésoxy-2,3,6 α-D-arabinohexopyranoside (préparé suivant la méthode de J. BOIVIN et Coll. Carb. Res. 85 (1980) 223 - 42) dans 2 ml de DMF anhydre sont ajoutés, goutte à goutte, à 0°C et sous agitation, 0,4 ml ($5.10^{-3}$ mole) d'isocyanate de chloro-2 éthyle. Après 5 heures d'agitation, le mélange réactionnel est évaporé à sec sous vide. Le résidu, après purification par chromatographie sur colonne de silice, éluant $CHCl_3$: 95, MeOH: 5, donne un produit monotache cristallisant dans l'éther éthylique anhydre. Les cristaux (0,8 g, Rendement 60 %) sont essorés puis séchés.

**Analyse** $C_{10}H_{19}Cl\ N_2O_4$: 266,5 - Calculé % C: 45,0, H: 7,1,
N: 10,5 - Trouvé % C: 44,9, H: 7,0, N: 10,5.
F: 125 - 127°
**Spectre RMN** Solvant: DMSO.$d_6$: 1,14 (d $CH_3$-6' J = 6 Hz), 1,46 (t(d) H-2'$_{ax}$J = J' = 12 Hz, J" = 4 Hz) 1,90 (dd H-2'$_{eq}$ J = 12 Hz, J' = 4 Hz) 2,78 (t H-4' J = J' = 9 Hz) 3,19 (s $OCH_3$) 3,28 (m $CH_2$-4) 3,42 (m H-5') 3,53 (m $CH_2$-5) 3,61 (m H-3') 4,58 (d H-1' J = 3 Hz) 5,99 (d NH J = 8 Hz) 6,14 (t NH J = J'= 6 Hz).

**Exemple 6**

**Méthyl [(chloro-2 éthyl)-3 nitroso-3 uréido]-3 tridésoxy-2,3,6 α-D-arabinohexopyranoside - Composé 2 - IC 81 1183**

1,2 g (4,5.10-3 mole) de méthyl [(chloro-2 éthyl)-3 uréido]-3 tridésoxy-2,3,6 α-D-arabinohexopyranoside sont dissous dans 8 ml d'acide formique. A la solution maintenue à 0°C, sont ajoutés par petites portions et sous agitation, 2,5 g (0,036 mole) de nitrite de sodium. Après 30 minutes, sont ajoutés 10 ml d'eau, l'agitation est alors maintenue une heure. Le mélange réactionnel est versé sur 100 ml d'acétate d'éthyle puis séché sur sulfate de sodium et évaporé à sec sous vide. Après purification sur colonne de chromatographie, support silice, éluant $CHCl_3$: 9, MeOH: 1, on obtient des cristaux incolores (600 mg, Rendement 45 %).

**Analyse** $C_{10}H_{18}Cl\ N_3O_5$: 295,71 - Calculé % C: 40,6, H: 6,1,
N: 14,2 - Trouvé % C: 40,9, H: 6,0, N: 13,9.
F: 100°C
$[\alpha]^{20}_D$: + 92,8° (c: 0,5 % $CHCl_3$) - $[\alpha]^{20}_{365}$: 123,6°
(c: 0, 5 % $CHCl_3$)
**Spectre RMN** (DMSO.$d_6$) 1,15 (d $CH_3$-6' J = 6 Hz) 1,78 à 1,94 (m $CH_2$ -2') 3,04.(t H-4' J = J' = 9 Hz) 3,25 (s $OCH_3$) 3,51 (m H-5') 3,60 (t $CH_2$-4 J = J' = 6 Hz) 4,10 (m $CH_2$-5, H-3 ') 4,65 (d H-1' J = 3 Hz) 8,48 (d NH J = 9 Hz).
**Spectre de Masse:** (M + 1): 296.

**Exemple 7**

**Méthyl azido-3 didésoxy-2,3 α-D-arabinohexopyranoside - Composé 3**

A 7 g (0,024 mole) de méthyl azido-3 benzylidène-4,6 didésoxy-2,3 α-D-arabinohexopyranoside préparé suivant RI-CHARDSON A.C., (Carbohyd. Res. 4 (1967), 422 - 428), en solution dans 400 ml de méthanol anhydre, on ajoute goutte à goutte et sous agitation, 8 ml de chlorure d'acétyle. Après 6 heures d'agitation à température ambiante, la solution est neutralisée par barbotage d'ammoniac gazeux. Après évaporation sous pression réduite, le résidu est repris par de l'acétone à reflux. La solution acétonique est concentrée sous pression réduite. Par addition d'hexane, puis par refroidissement, on obtient des cristaux blancs qui sont essorés, lavés avec de l'hexane, puis séchés sous vide. On obtient: 3,45 g (71 %).

**Analyse** $C_7H_{13}N_3O_4$: 203,2
F: 123° - 124°C
[α] $^{20}_D$: + 183° (c: 1 %, MeOH)
IR: νOH 3340, 3280 $cm^{-1}$; μ $N_3$ 2100 $cm^{-1}$

**Exemple 8**

**Méthyl amino-3 didésoxy-2,3 α-D-arabinohexopyranoside - Composé 4**

Une solution de 5 g (0,0225 mole) de méthyl azido-3 didésoxy-2,3 α-D-arabinohexopyranoside dans 10 ml de méthanol est agitée 12 heures sous atmosphère d'hydrogène, en présence de triéthylamine (1 ml) et de charbon palladié à 10 % (1 g). Le catalyseur est éliminé par filtration. Le filtrat, évaporé à sec sous pression réduite, donne 4,35 g de composé 4 sous forme d'une huile incolore.

Ce composé est cristallisé sous forme de chlorhydrate.

**Analyse** $C_7H_{15}NO_4$, HCl: 213,67
F: 120°C (déc) [α] $^{20}_D$: + 90° (c: 1 %, $H_2O$).

**Exemple 9**

**9 Méthyl [(chloro-2 éthyl)-3 nitroso-3 uréido]-3 didésoxy 2,3 α-D-arabinohexopyranoside - Composé 6 - IC 81 1184**

A partir du Composé 4, suivant le mode opératoire déjà décrit, on obtient par action de l'isocyanate de chloro-2 éthyle le méthyl[(chloro-2 éthyl)-3 uréido]-3 didésoxy-2,3 α-D-arabinohexopyranoside, 5.

**Analyse** $C_{10}H_{19}ClN_2O_5$: 282 -Calculé % C: 42,5, H: 6,7,N: 9,9
Trouvé % C: 42,3, H: 6,8, N: 10,0
F: 125°C [α] $^{20}_D$: + 168° (c: 0,25 %, $CHCl_3$)
Puis par nitrosation par le nitrite de sodium, dans l'acide formique, le Composé 6.

**Analyse** $C_{10}H_{18}$ Cl $N_3O_6$: 311,71 - Calculé %: C: 38,5, H: 5,8, N: 13,5
Trouvé % C: 38,4, H: 5,6, N: 13,6.
F: 118°C
[α] $^{20}_D$: + 96,2° (c: 0,5 %, $CHCl_3$) - [α] $^{20}_{365}$: 118,0° (c: 0,5 %, $CHCl_3$)
**Spectre RMN** (DMSO.$d_6$): 1,82 (t(d) H-2'$_{ax}$ J = J' = 12 Hz, J": 4 Hz) 1,88 (d(d) H-2'eq J = 12 Hz J' = 4 Hz) 3,28 (s $OCH_3$) 3,43 (t H-4' J = J' = 9 Hz) 3,35 à 3,53 (m H-5', H-3') 3,49 à 3,67 (ab $CH_2$-6' $J_{gem}$ = 12 Hz) 3,61 (t $CH_2$-4 J = J' = 6 Hz) 4,10 (m $CH_2$-5) 4,73 (d H-1' J = 4 Hz) 8,53 (d NH J = 9 Hz).
**Spectre de Masse** (ionisation chimique): m/e 312 ($Cl_{35}$, M + 1, Pic de base) et m/e 314 ($Cl_{37}$, 30 %); m/e 280 (M + 1 - 32, 30 %) et m/e 282 (10 %).

**Exemple 10**

**Méthyl azido-3 bromo-6 tridésoxy-2,3,6 α-D-arabinohexopyranoside - Composé 7**

10 g (0,026 mole) de méthyl azido-3 O-benzoyl-4 bromo-6 tridésoxy2,3,6 α-D-arabinohexopyranoside préparé suivant Hanessian, J. Org. Chem. 34 (1969) 1045 - 1053, sont mis en solution dans 100 ml de méthanolate de sodium méthanolique 1 M. Après 4 heures d'agitation à 20°C, la solution est ensuite neutralisé par passage sur résine Amberlite IRA 50, forme $H^+$. Le filtrat, évaporé à sec sous vide, est purifié par chromatographie sur silice H-60 et donne 6 g (Rendement 84 %) du composé 7

**Analyse** $C_7H_{12}Br$ $N_3O_3$: 266,22
[α] $^{20}_D$: + 124° (c: 1 %, $CHCl_3$)
**Spectre IR**: ν 3420 - 3440 $cm^{-1}$ (OH) 2100 $cm^{-1}$ ($N_3$)

**Exemple 11**

**Méthyl azido-3 bromo-6 tridésoxy-2,3,6, O-méthyl-4 α-D-arabinohexopyranoside - Composé 8**

5,5 g (0,021 mole) du composé 7 sont mis en solution dans 200 ml de tétrahydrofuranne anhydre. On ajoute 20 g de soude, puis 20 ml de sulfate de méthyle. Le mélange réactionnel est porté au reflux pendant 24 heures. Après refroidissement, 100 ml d'eau sont ajoutés. La phase organique est soutirée par décantation puis la phase aqueuse est à nouveau extraite par 100 ml de tétrahydrofuranne. La phase organique, séchée sur sulfate de sodium, est évaporée à sec. On obtient 5,7 g (Rendement 98 %) du composé 8. Il est purifié par chromatographie sur silice H (éluant hexanechlorure de méthylène 1 : 1).

**Analyse** $C_8H_{14}BrN_3O_3$: 280,25
$[\alpha]^{20}_D$: + 188° (c: 1 %, $CHCl_3$).
**Spectre IR**: ν $N_3$ 2100 $cm^{-1}$

**Exemple 12**

**Méthyl amino-3 tridésoxy-2,3,6, O-méthyl-4, α-D-arabinohexopyranoside - Composé 9**

5,15 g (0,018 mole) du composé 8 en solution dans 100 ml d'éthanol anhydre et 3 ml de triethylamine redistillée sont mis à hydrogéner en présence de 2 g de palladium sur charbon à pression ordinaire, durant 12 heures. Après élimination du catalyseur, la solution est passée sur une résine échangeuse d'ions IR 45, forme $OH^-$. La solution évaporée à sec conduit à 3,5 g de produit brut purifié par chromatographie sur silice H, éluant $CH_2Cl_2$ - MeOH 9 : 1
**Analyse** $C_8H_{17}NO_3$: 175,22
$[\alpha]^{20}_D$: + 109° (c: 1 %, $CHCl_3$)

**Exemple 13**

**Méthyl [(chloro-2 éthyl)-3 nitroso-3 uréido] -3 tridésoxy-2,3,6 0-méthyl-4 α-D-arabinohexopyranoside - Composé 10 - IC 83 1373**

Il est obtenu par la méthode habituelle à partir du composé 9.
**Analyse** $C_{11}H_{20}ClN_3O_5$: 309,75 - Calculé % C: 42,6, H: 6,5, N: 13,6 - Trouvé % C: 42,3, H: 6,2, N: 13,6.
F: 60°C
$[\alpha]^{20}_D$: + 68,8° (c: 0,5 % $CHCl_3$) - $[\alpha]^{20}_{365}$: + 113,0° (c: 0,5 %, $CHCl_3$)
**Spectre RMN** (DMSO-$d_6$): 1,19 (d $CH_3$ J = 6 Hz), 1,87 (m $CH_2$-2') 3,01 (t H-4'J = J' = 9 Hz) 3,25 (s $OCH_3$-1) 3,35 (s OCH3-4) 3,53 (m H-5) 3,63 (t $CH_2$-4 J = J' = 6 Hz) 3,61 (m $CH_2$-5) 4,18 (m H-3') 4,66 (d H-1' J = 3 Hz) 8,73 (d NH J = 9 Hz) .
**Spectre de Masse** (ionisation chimique): m/e 310 ($Cl_{35}$, (M+1, Pic de base) et m/e 312 ($Cl_{37}$, 30 %); m/e 278 (M+1-32, 90 %) et m/e 280 (27 %).

**Exemple 14**

**Méthyl diazido-3,6 0-benzyl-4 tridésoxy-2,3,6 α-D-arabinohexopyranoside - Composé 11**

A une solution de 6 g (0,016 mole) de méthyl azido-3 O-benzyl-4 bromo-6 tridésoxy-2,3,6 α-D-arabinohexopyranoside dans 50 ml de diméthylformamide anhydre, sont ajoutés 6 g (0,092 mole) d'azoture de sodium. Le milieu réactionnel est porté à 80°C pendant 8 heures. Après refroidissement et dilution par 150 ml d'eau, on extrait à plusieurs reprises avec de l'éther éthylique. La phase éthérée, après évaporation à sec, sous vide, donne une huile incolore: 5 g (98 %).
$[\alpha]^{20}_D$: + 51° (c: 2,2 %, $CHCl_3$)

**Spectre IR**: μ 3450 $cm^{-1}$ (OH) 2115 $cm^{-1}$ ($N_3$)

**Exemple 15**

**Méthyl diazido-3,6 tridésoxy-2,3,6 α-D-arabinohexopyranoside - Composé 12**

5 g (0,015 mole) du composé 11 sont mis en solution dans 50 ml de méthanol anhydre, on ajoute alors une solution molaire de méthylate de sodium (50 ml) et on agite pendant 3 heures à température ambiante. La solution est neutralisée par filtration sur résine Amberlite IR 50 $H^+$, puis évaporée à sec sous pression réduite. On obtient une huile sirupeuse (3,4 g 100 %) qui est purifiée sur colonne de silice H (éluant $CH_2Cl_2$).

**Analyse** $C_7H_{12}N_6O_3$
[α] $^{20}{}_D$: + 128° (c: 1,6 %, $CHCl_3$)
**Spectre IR**$_{film}$: ν 3450 $cm^{-1}$ (OH) 2115 $cm^{-1}$ ($N_3$)

**Exemple 16**

**Méthyl diamino-3,6 tridésoxy-2,3,6 α-D-arabinohexopyranoside - Composé 13**

3 g (0,013 mole) du composé 12 en solution dans 100 ml d'éthanol à 1 % de triéthylamine sont mis à hydrogéner à pression ordinaire pendant 12 heures en présence de palladium sur charbon comme catalyseur. Après élimination du catalyseur, le filtrat évaporé à sec donne un résidu sirupeux (2,8 g). Une chromatographie sur silice (éluant $CH_2Cl_2$, MeOH ammoniacal 80 : 20) permet d'isoler le produit 13 pur.

**Analyse** $C_7H_{16}N_2O_3$: 176,25
[α] $^{20}{}_D$: + 130° (c: 1,13 %, MeOH)
**Spectre IR**$_{film}$: ν 3700 $cm^{-1}$ (OH), 3360 $cm^{-1}$ (NH)

**Exemple 17**

**Méthyl bis [-(chloro-2 éthyl)-3 nitroso-3 uréido]-3,6 tridésoxy-2,3,6 α-D-arabinohexopyranoside. - Composé 14 - IC 83 1374)**

Il est obtenu par la méthode habituelle à partir du composé 13.
**Analyse** $C_{13}H_{22}Cl_2N_6O_7$: 445,267 - Calculé % C: 35,1, H: 5,0, N: 18,9 - Trouvé % C: 35,3, H: 5,1, N: 19,2.
F: 102°C
[α] $^{20}{}_D$: -59,0° (c: 0,5 %, $CHCl_3$)- [α] $^{20}{}_{365}$: + 74,0° (c: 0,5 %, $CHCl_3$)
**Spectre RMN** (DMSO-$d_6$): 1,84 (m $CH_2$-2') 3,15 (s $OCH_3$) 3,17 à 3,77 (m $CH_2$-6', H-3', H-4', H-5', 2$CH_2$-4) 4,06 (m 2$CH_2$-5) 4,66 (d H-1 J = 3 Hz) 8,41 (t NH J = J' = 6 Hz) 8,50 (d NH J = 9 Hz) .
**Spectre de Masse**: (M + 1): 445 100 % Pic de base
$Cl_{35}$ $Cl_{37}$
447 449
$Cl_{37}$ $Cl_{37}$

**Exemple 18**

**Méthyl O-acétyl-3 O-benzylidène-4,6 désoxy-2 α-D-arabinohexopyranoside - Composé 15**

A 10 g (0,037 mole) de méthyl O-benzylidène-4,6 désoxy-2 α-D-arabinohexopyranoside en solution dans 50 ml de pyridine anhydre, on ajoute 40 ml d'anhydride acétique redistillé. Après 48 h à 50°C, le mélange réactionnel est refroidi puis versé sur de la glace pilée, extrait par 3 fois 100 ml de dichlorométhane. La phase organique séchée sur sulfate de sodium donne après évaporation, 11,5 g (99 %) d'un produit cristallisé que l'on purifie par recristallisation dans un mélange hexane-acétone.
F: 125° - 127°C.
[α] $^{20}{}_D$: 74° (c: 1 %, chloroforme)
**Spectre IR**$_{Nujol}$: 1728, 1240 $cm^{-1}$ (C = O ester) .

**Exemple 19**

**Méthyl O-acétyl-3 O-benzoyl-4 bromo-6 didésoxy-2,6 α-D-arabinohexopyranoside Composé 16**

A 5,12 g (0,02 mole) du composé 15 en solution dans 200 ml de tétrachlorure de carbone, sont ajoutés 7,13 g (0,04 mole) de carbonate de baryum et 3,23 g (0,020 mole) de N-bromo-succinimide. La réaction est portée au reflux pendant 3 heures. Après refroidissement et élimination de l'insoluble par filtration, la phase organique est lavée par une solution saturée d'hydrogénocarbonate de sodium. On obtient, après évaporation, 6,4 g (98 %) d'un produit huileux monotache en c.c.m. [α] $^{20}{}_D$: + 70° (c: 1,45 %, $CHCl_3$)

**Spectre IR**: 1730 $cm^{-1}$, 1240 $cm^{-1}$ (CO ester) 1600, 1585 $cm^{-1}$ (aromatiques)

**Exemple 20**

**Méthyl O-acétyl-3 azido-6 O-benzoyl-4 didésoxy-2,6 α-D-arabinohexopyranoside. - Composé 17**

A une solution de 8 g (0,020 mole) du composé 16 dans 50 ml de diméthylformamide anhydre, sont ajoutés 8 g (0,12 mole) d'azoture de sodium. Le mélange réactionnel est porté à 80°C pendant 8 heures.

Après refroidissement et dilution par de l'eau, le mélange réactionnel est extrait par de l'éther. La solution lavée à plusieurs reprises par de l'eau, est séchée sur sulfate de sodium. Après évaporation sous vide, le résidu est purifié par chromatographie sur silice (éluant hexane-acétate d'éthyle,3-1). On isole 6,5 g de produit pur 95 %) .

Le produit est recristallisé dans l'hexane.

**Analyse** $C_{16}H_{19}N_3O_5$: 349,38
F: 68°C [α] $^{20}$D: +90° (c: 1 %; $CHCl_3$)
IR: 2100 $cm^{-1}$ (azide) 1725, 1280, 1050 $cm^{-1}$ (ester) 1610, 1590 $cm^{-1}$ (aromatique)

**Exemple 21**

**Méthyl azido-6 didésoxy-2,6 α-D-arabinohexopyranoside - Composé 18**

A une solution de 4,96 g (0,014 mole) du composé 17 dans 50 ml de méthanol anhydre, est ajoutée une solution méthanolique de méthylate de sodium (20 ml).

Après 12 heures d'agitation à la température ambiante, le milieu réactionnel est neutralisé par filtration sur résine Amberlite IR 50 (forme $H^+$). Après élimination du solvant, le résidu sirupeux obtenu est chromatographié sur colonne de silice pour éliminer le benzoate de méthyle. On obtient 2,76 g du composé 18 (96 %) .
**Analyse**: $C_7H_{13}N_3O_4$: 203,2
[α] $^{20}$D: + 104° (c: 1 %, chloroforme).
**Spectre IR**: 3400 $cm^{-1}$ (OH) 2120 $cm^{-1}$ (azide).

**Exemple 22**

**Méthyl amino-6 désoxy-2 α-D-arabinohexopyranoside - Composé 19**

Une solution de 2,40 g (0,011 mole) du composé 18 dans 25 ml d'éthanol anhydre est agitée sous atmosphère d'hydrogène en présence de palladium sur charbon à 10 % (500 mg) pendant 12 heures. Après élimination du catalyseur, l'évaporation du filtrat conduit à un produit huileux 2 g (95 %). Un échantillon est transformé en picrate.

**Analyse**: $C_{13}H_{18}N_4O_{11}$: 406,35
F: 156° C (éthanol)
[α] $^{20}$D: + 75° (c: 1,2 %, chloroforme).

**Exemple 23**

**Méthyl [(chloro-2 éthyl)-3 nitroso-3 uréido] -3 didésoxy-2,6 α-D-arabinohexopyranoside - Composé 20 IC 83 1350**

Il est obtenu par la méthode habituelle à partir du composé 19.

**Analyse** $C_{10}H_{18}ClN_3O_6$: 311, 728 - Calculé %: C: 38,5, H: 5,8, N: 13,5 - Trouvé %: C: 38,5, H: 5,5, N: 13,4.
F: 101°C (déc.)
[α] $^{20}$D: + 26,2° (c: 0,5 %, $CHCl_3$)
[α] $^{20}_{365}$: + 51,0° (c: 0,5 %, $CHCl_3$)
**Spectre RMN** (DMSO-$d_6$): δ 1,45 (t(d) H-2'$_{ax}$ J = J ' = 12 Hz, J" = 4 Hz), 1,85 (d(d) H-2'$_{eq}$ J = 12 Hz J' = 4 Hz), 2,95 (t H-4' J = J' = 9 Hz), 3,10 (s-$OCH_3$), 3,30 (m H-3'), 3,51 (m H-5 + $CH_2$-6'), 3,58 (t $CH_2$-4 J = J' = 6 Hz), 3,75 (d $_bCH_2$-6' $J_{gem}$ = 12 Hz), 4,06 (m $CH_2$-5), 4,15 (d H-1 'J = 3 Hz), 4,81 (OH), 5,10 (OH), 8,50 (t NH).
**Spectre de Masse**: (M + 1) = 312 $Cl_{35}$ 314 $Cl_{37}$ (Perte de MeOH -32) 280.

**Exemple 24**

**Méthyl N méthoxycarbonyl amino-3 tridésoxy 2,3,6 α-D-arabinohexopyranoside - Composé 21**

A 1,5 g (0,0093 mole) de méthyl amino-3, tridésoxy 2,3,6 α-D-arabinohexopyranoside dans 200 ml de chlorure de méthylène anhydre, sont ajoutés à 0°C et sous agitation, en 10 minutes, 6 ml de chloroformiate de méthyle. Le mélange réactionnel est maintenu 2 heures à température ordinaire, puis sont ajoutés 100 ml de soude 4 N. Après une nuit sous agitation, la phase organique est séparée par décantation, la phase aqueuse extraite par 100 ml de chlorure

de méthylène. La phase organique lavée avec de l'eau distillée est séchée sur sulfate de sodium.

Après évaporation sous vide, le résidu obtenu est recristallisé dans un mélange méthanol-chlorure de méthylène, 1,7 g (80 %).

**Analyse** $C_9H_{17}NO_5$: 219,24
F: 180°C
[α] $^{20}$D: + 157° (c: 1 %, $CHCl_3$).

**Exemple 25**

**Méthyl méthylamino-3 tridésoxy-2,3,6 α-D-arabinohexopyranoside - Composé 22**

A 1 g d'hydrure de lithium et d'aluminium dans 100 ml d'éther éthylique anhydre sont ajoutés 1,7 g (0,0077 mole) du composé 21 dans 50 ml d'éther éthylique anhydre, goutte à goutte, de façon à maintenir un léger reflux (30'). Le reflux est continué pendant 6 heures. Après refroidissement, l'excès d'hydrure est décomposé par addition très lente de 1 ml d'eau, puis 1 ml de soude 3 N, puis 3 ml d'eau. Le précipité est éliminé par filtration. La phase organique séchée sur sulfate de sodium donne, après évaporation sous vide puis recristallisation dans un mélange acétone-hexane, 1,05 g de cristaux (80 %); F: 105°C.

**Analyse**: $C_8H_{17}NO_5$: 175,23
[α] $^{20}$D: 89° (c : 1 %, $CHCl_3$)

**Exemple 26**

**Méthyl [-(chloro-2 éthyl) -3 méthyl-1 nitroso-3 uréido]-3 tridésoxy-2,3,6 (α-D-arabinohexopyranoside - Composé 23 - IC 83 1375**

Il est préparé suivant la méthode habituelle à partir du composé 22.

Les exemples 5 à 25 ci-dessus sont illustrés par les schémas réactionnels suivants:

**Examples 5 et 6**

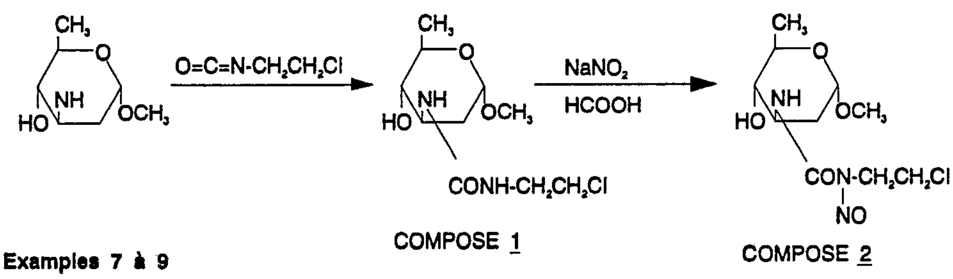


COMPOSE 1

COMPOSE 2

**Examples 7 à 9**

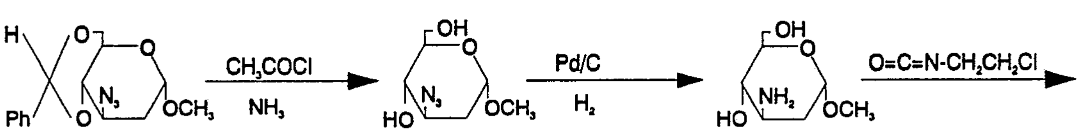


COMPOSE 3

COMPOSE 4

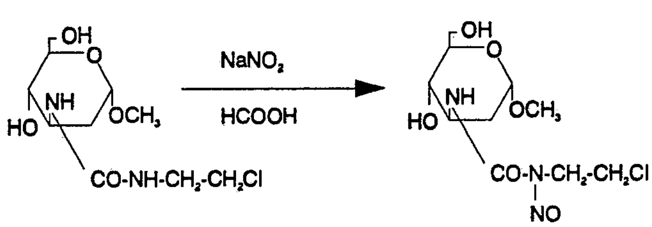


COMPOSE 5

COMPOSE 6

**Examples 10 à 17**

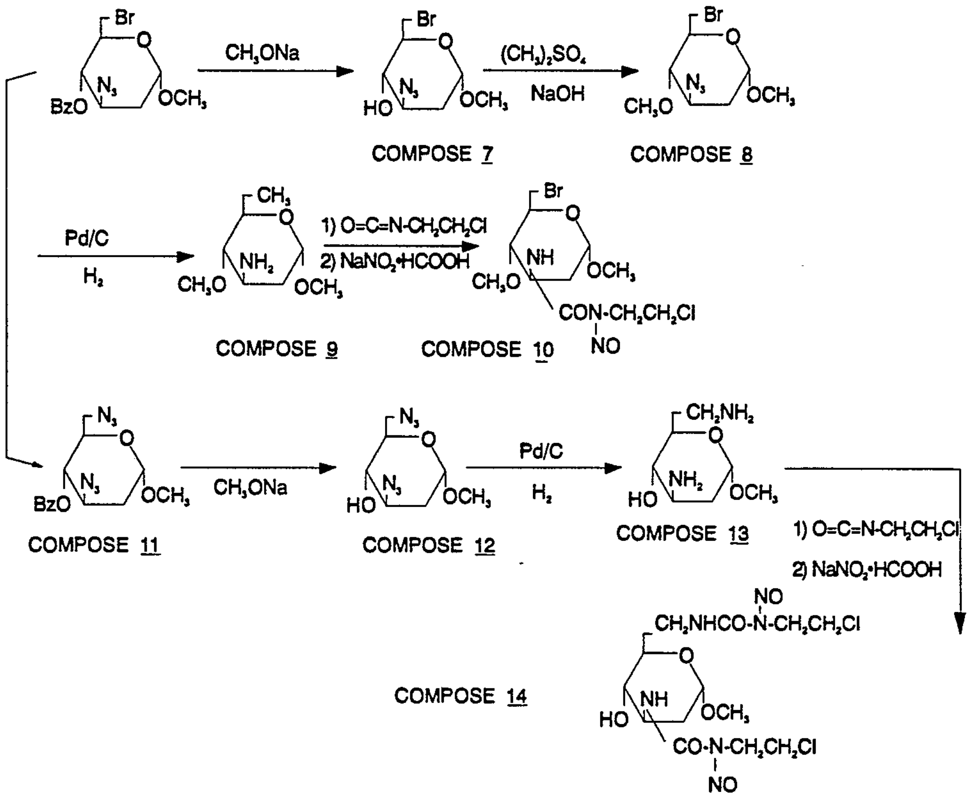


COMPOSE 7

COMPOSE 8

COMPOSE 9

COMPOSE 10

COMPOSE 11

COMPOSE 12

COMPOSE 13

COMPOSE 14

**Examples 18 à 23**

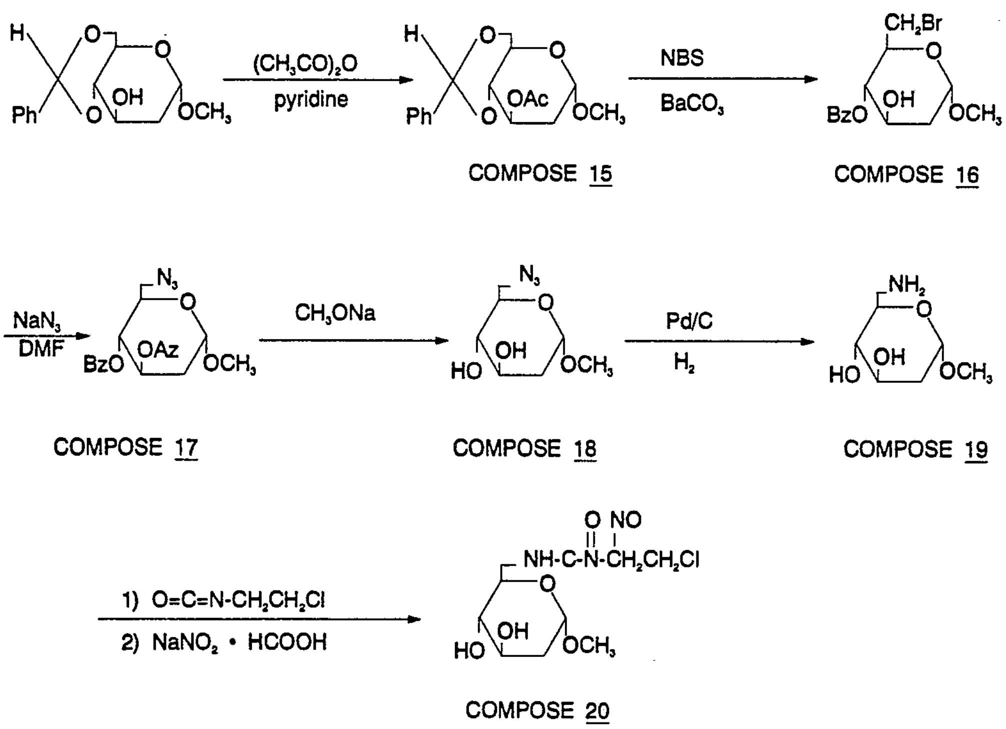


COMPOSE 15

COMPOSE 16

COMPOSE 17

COMPOSE 18

COMPOSE 19

COMPOSE 20

**Examples 24 à 26**

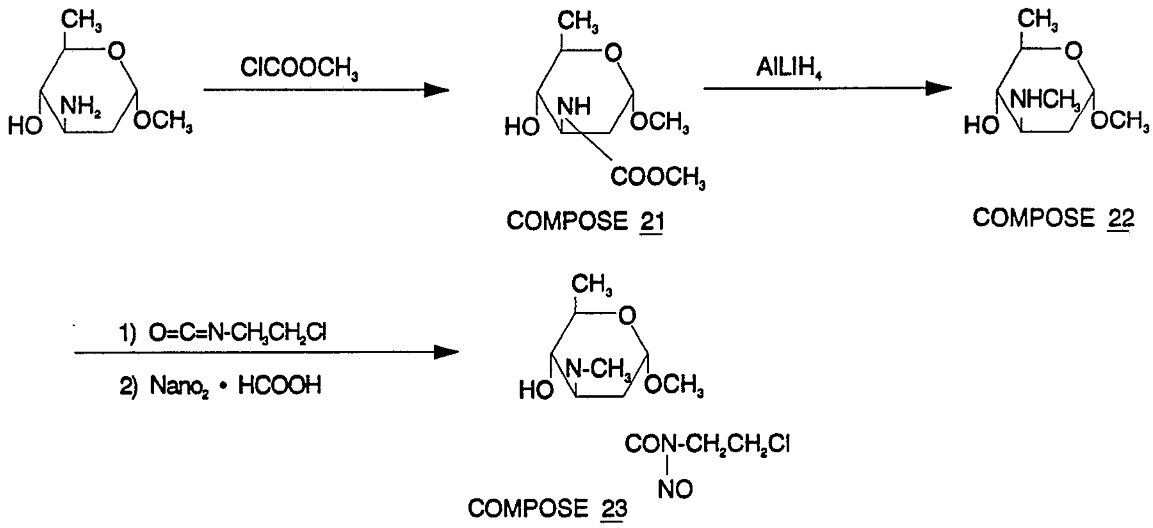


COMPOSE 21

COMPOSE 22

COMPOSE 23

**Exemple 27**

**Méthyl [(chloro-2-éthyl)-3 nitroso 3-uréido]-3-tridésoxy-2,3,6 α-L-arabinohexo-pyranoside - Composé 26 - IC 84 1530**

On effectue d'abord la préparation, comme indiqué, ci-après, du méthyl amino-3-tridésoxy-2,3,6 α-L-arabinohexopyranoside (L-Acosamine).

A 1,92 g (0,012 mole) de méthyl didésoxy-2,6 α-L-érythrohexopyranoside ulose-3 dans 25 ml d'éthanol aqueuse à 50 %, sont ajoutés 3,1 g (0,04 mole) d'acétate de sodium anhydre et 2,1 g (0,024 Mole) de chlorhydrate de O-méthyl hydroxylamine. On porte le mélange réactionnel au reflux pendant 3 heures puis on évapore l'éthanol. Après extraction au dichlorométhane et séchage sur sulfate de sodium une huile claire (2g) est obtenue dont la structure est confirmée par RMN et correspond au O-méthyloxime du composé 3.

Cette huile est solubilisée dans 20 ml de tétrahydrofuranne anhydre et on ajoute à la solution sous azote et à 0°C, 30 milli équivalents de diborane. Le mélange réactionnel est porté au reflux deux heures puis refroidi à 0°C. 5 ml d'eau puis 5 ml de potasse à 20 % sont ajoutés avec précaution. Le milieu réactionnel, porté au reflux 3 heures puis refroidi, est extrait par de l'acétate d'éthyle. La phase organique donne après évaporation un résidu qui purifié donne des cristaux de L-Acosamine.

F: 130° - 133°C

$[\alpha]^{20}_{D}$: -140°C (c: 0,6 %, MeOH)

On effectue ensuite, comme indiqué ci-après, la préparation du méthyl [(chloro-2 éthyl)-3 uréido]-3 tridésoxy-2,3,6 α-L-arabino-hexopyranoside (composé 25)

A une solution de 0,48 g ($3.10^{-3}$ mole) de méthyl amino-3 tridésoxy-2,3,6 α-L-arabinohexopyranoside dans 8 ml de diméthylformamide redistillé, sont ajoutés 0,3 ml ($4.10^{-3}$ mole) d'isocyanate de chloro-2 éthyle. Après 2 heures d'agitation, le mélange réactionnel est évaporé à sec sous vide. Les cristaux sont essorés puis lavés à l'éther: 781 mg (98 %).

F: 128°C

**Spectre RMN**: Solvant DMSO $D_6$: 1,14 (d, $CH_3$-6', J = 6 Hz); 1,46 (T(d), H-2'$_{ox}$, J = J' = 12 Hz, J" = 4 Hz) ; 1,90 (dd,H-2'eq, J = 12 Hz, J' = 4 Hz); 2,78 (T,H-4', J = J'= 9 Hz); 3,19 (s,$OCH_3$); 3,28 (m,$CH_2$-4); 3,42 (m,H-5'); 3,53 (m,$CH_2$-5); 3,61 (m,H-3'); 4,58 (d,H-1', J = 3 Hz); 5,99 (d,NH, J = 8 Hz); 6,14 (t,NH, J = J' = 6 Hz).

Le produit final désiré (composé 26), à savoir le méthyl [(chloro-2 éthyl)-3 nitroso-3 uréido]-3 tridéxoxy2,3,6 α-L-arabinohexopyranoside est obtenu à partir du produit précédemment synthétisé, comme suit:

0,66 g ($2,5.10^{-3}$ mole) de méthyl [(chloro-2 éthyl)3 uréido]-tridésoxy-2,3,6 α-L-arabinohexopyranoside sont dissous dans 5 ml d'acide formique. A la solution maintenue à 0°C, sont ajoutés par petites portions et sous agitation, 1,4 g (0,02 Mole) de nitrite de sodium. Après 3O minutes, on ajoute 5 ml d'eau et l'agitation est alors maintenue une heure. Le mélange réactionnel est versé sur 100 ml d'acétate d'éthyle puis séché sur sulfate de sodium et évaporé à sec sous vide. Après purification sur colonne support silice, éluant $CH_2Cl_2$: 98, MeOH: 2, on obtient des cristaux incolores, 210 mg (30 %).

F: 100°C

**Analyse** $C_{10}H_{18}ClN_3O_5$: 295,71.

Calculé %: C: 40,6; H: 6,1; N: 14,2.

Trouvé %: C: 40,9; H: 6,1; N: 13,90.

**Spectre RMN** (DMSO,$D_6$): 1,15 (d,$CH_3$-6' J = 6 Hz); 1,78 à 1,94 (m, CH-2'); 3,04 (t,H-4', J = 9 Hz); 3,25 (s,$OCH_3$); 3,51 (m,H-5'); 3,60 (T,$CH_2$-4, J = J' = 6 Hz); 4,10 (m,$CH_2$-5, H-3'); 4,65 (d,H-1', J = 3 Hz); 8,48 (d,NH, J = 9 Hz).

Le schéma réactionnel ci-après, résume les étapes qui viennent d'être décrites:

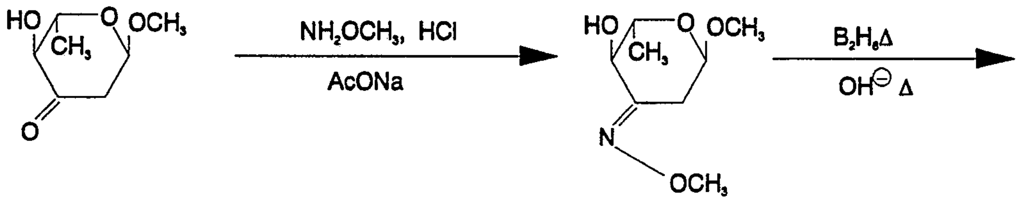


méthyl didésoxy-2,6
α-L-erythrohex opyranoside ulose-3

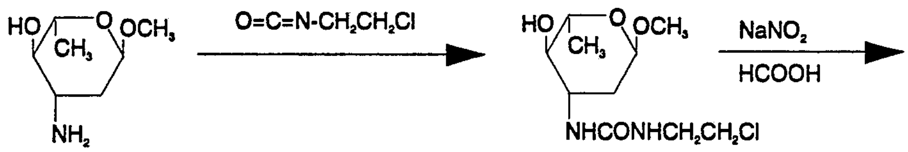


COMPOSE 24 COMPOSE 25

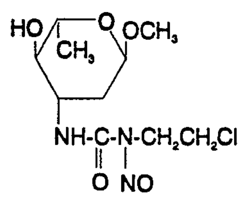

COMPOSE 26

Etude pharmacologique

Afin de tester l'activité antitumorale des composés décrits plus haut, on a utilisé en premier lieu la leucémie murine L1210. Parmi les leucémies murines,la leucémie L1210 est résistante et sélective. Une substance ayant une grande activité sur la leucémie L1210 a un potentiel d'activité en clinique humaine (J.M. Venditti, Relevance of transplantable animal tumor systems to the Selection of new agents for clinical trial in Pharmacological Basis of Cancer Chemotherapy, the University of Texas ed Williams and Wilkins Co, publ. 1975, Baltimore USA, p. 245 - 270).

Par ailleurs la tumeur expérimentale qui est la leucémie L1210 de la souris est en effet couramment utilisée pour l'évaluation de tous les composés antitumoraux actuellement mis en oeuvre en thérapeutique humaine, ainsi qu'il est décrit, par exemple par C.C. Zubrod dans Proc.Nat. Acad. Sci. USA,69 1972, p. 1042 - 1047. Le système tumoral ainsi constitué expérimentalement permet une évaluation quantitative très précise de l'activité du composé testé, et, par conséquent aussi, une comparaison objective entre les activités respectives de différents composés, par exemple selon les méthodes décrites par R.E. Skipper, F.M. Schabel, Jr. et W.S. Wilcox dans Cancer Chemother, Rep., 35, 1964, p. 1 - 111 et 45, 1965, p. 5 - 28.

Ceci a été confirmé par les résultats des travaux récents de Staquet et al. Cancer Treatment Reports, vol. 67, n° 9, Septembre 83.

En pratique, les effets biologiques des nouveaux dérivés de nitrosourées conformes à la présente invention ont été testés comme suit:

### Méthode

Le test utilisé est celui de W. J. Durkin et Al.- Cancer Research 1979, 39, 402 - 407 modifié.

Toutes les nitrosourées sont dissoutes dans de l'éthanol à 70 % à raison de 10 mg/ml.

Le test est effectué en deux étapes.

**1 - Détermination de l'indice cytotoxique 20 %**

100 µl d'une suspension de cellules L 1210 ($10^6$ cellules par ml) dans du milieu de culture R P M I 1640 additionné de 10 % de sérum de veau foetal et de 40 µg/ml de gentamycine, contenant différentes doses des produits à tester (0 à 100 µg/ml) sont mis en incubation 24 heures à 37°C. Au bout de ce temps, la viabilité cellulaire est déterminée par le test d'exclusion du bleu de trypan. L'indice cytotoxique est défini selon la formule:

Indice cytotoxique:

$$100\left[1-\frac{\%\ \text{cellules vivantes traitées}}{\%\ \text{cellules vivantes témoins}}\right]$$

La quantité d'éthanol est la même dans les cultures contenant les produits à tester et dans les cultures témoins (Cette quantité n'a aucun effet ni sur la croissance ni sur la viabilité cellulaire).

On détermine pour chaque produit la dose qui donne un indice cytotoxique de 20 %.

**2 - Détermination de l'activité potentielle "in vivo"**

Des cellules L 1210, dans des conditions analogues au protocole précédent, sont mises en contact pendant 1 heure avec une dose des différents produits testés correspondant à une valeur de l'indice cytotoxique égale à 20 %. Au bout de ce temps, les cellules sont mises dans du milieu de culture ne contenant pas de nitrosourées et mises en incubation à 37°C. Au bout de 48 heures, l'indice cytotoxique est déterminé.

W.J. Durkin et al... ont montré que, dans ces conditions, si l'indice cytotoxique est égal ou supérieur à 40 %, le produit considéré sera actif "in vivo" chez la souris.

**Résultats**

Les résultats sont rassemblés dans le Tableau I qui va suivre:

**Tableau I**

| Produit Teste | Test N° 1 Dose Correspondant a un Indice Cytotoxique 20 % µg/ml | Test N° 2 Indice Cytotoxique % | Action Potentielle "In vivo" | Activite "In vivo" |
|---|---|---|---|---|
| Comp. 2 IC 1183 | 50 | 77 | + | + |
| Comp. 6 IC 1184 | 20 à 25 | N.D. | | N.D. |
| Comp. 10 IC 1373 | 80 | 68 | + | N.D. |
| Comp. 14 IC 1374 | 5C | 71 | + | N.D. |
| Comp. 20 IC 1350 | N.S. | N.S. | - | |
| Comp. 23 IC 1375 | 6C | 69 | + | N.D. |

N.D. = non déterminé
N.S. = non significatif

**3 - Détermination de l'activité effective "in vivo" sur la souris**

L'expérimentation dont il est rendu compte ci-après a utilisé le composé 2 préparé conformément à l'exemple 6 ci-dessus (Réf. IC 81 1183).

**3.1 Protocole**

- Les souris (femelles, poids moyen 20g) utilisées sont des $F_1/DBA_2/C_{57}/B1$ (Centre de sélection et d'élevage des animaux des Laboratoires du CNRS, Orléans, La Source).
- Les souri réparties par tirage au sort dans les cages ont été inoculées le jour "O" avec $10^5$ cellules de leucémie L1210.
- Les animaux ont été traités avec le composé IC 81 1183 par voie intrapéritonéale les jours 1, 5 et 9.
- suspensions ont été préparées juste avant l'injection: produit 2 + huile d'olive neutralisée stérilisée.
- La moralité des animaux a été relevée régulièrement, l'augmentation relative de la survie (T/C x 100) a été calculée à partir de la survie moyenne des animaux traités (T) et celle des animaux de contrôle (C)
- Les doses utilisées sont en mg/kg de souris: 1,25, 2,5, 5, 10, 20, 40, 50, 60, 80.

**3.2.Résultats**

- . 1,25 mg →T/C = 118
- . 2,50 mg →T/C = 158
- . 5 mg →T/C = 170
- . 10 mg →T/C = 220
- . 20 mg →T/C = ∞
- . 40 mg →T/C = ∞
- . 50 mg →T/C = 100
- . ≥ 60 mg →Toxicité

**3.3 Remarques**

a) T/C = ∞: pour définition: plus de 50 % des animaux traités sont guéris définitivement; or dans l'expérimentation réalisée, tous les animaux aux doses de 20 et 40 mg/kg sont guéris définitivement.

b) Cette courbe d'efficacité est aussi bonne que celle obtenue avec le RFCNU et le RPCNU décrits par IMBACH et Alia (Loc. cité) et elle est très nettement supérieure à celles obtenues par CCNU et Me CCNU décrits par MATHE et KENIS (Loc. cité).

c) On observe une chute brutale du T/C après 40 mg/kg. Ce phénomène est également observé avec les nitrosourées utilisées comme produits de comparaison:
l'élévation rapide de la toxicité annule l'efficacité du produit.

**3.4.** La valeur de T/C ≤ 125 % a été recherchée afin de déterminer la dose minimale active qui se situe entre 1,25 mg/kg de poids corporel et 2,5 mg/kg; en effet, à 1,25 mg/kg on se trouve légèrement au-dessous du seuil significatif de 125 % de survie, ce qui n'est pas le cas à la dose de 2,5 mg/kg.

**4. Détermination de l'activité "in vivo" des composés selon l'invention sur les trois tumeurs respectives leucémie L 1210 IGR, tumeur de Lewis et melanome B16.**

Les composés testés sont les composés des exemples référencés par IC 1183, IC 1184, IC 1350, IC 1373 et IC 1374.

**4.1. Leucémie L1210**

- . On a utilisé des souris femelles $DBA_2$, âgées d'environ 8 semaines et pesant environ 20 g provenant du centre IFFA-CREDO, (les Oncins, 69210 Arbresles)
- . Au jour "O", chaque souris reçoit par voie intrapéritonéale un inoculum de 1 x $10^5$ cellules tumorales dans un volume de 0,2 ml.
- . Après la greffe tumorale, les souris sont réparties au hasard en 21 cages de 5 animaux, elles-mêmes réparties ensuite,par tirage au sort, en 6 séries expérimentales. A l'intérieur de ces 6 séries expérimentales, on constitue une série témoin de contrôle de 6 cages et 5 séries expérimentales, de 3 cages chacune, et dont les souris sont destinées à être traitées par les composés de l'invention.

**4.2. Melanome B16**

- . On utilise des souris femelles C57 B 1/6, âgées d'environ 8 semaines et pesant environ 20 g, provenant du centre IFFA-CREDO.
- . Au jour "O", chaque souris reçoit par voie intrapéritonéale un inoculum de 2 x $10^6$ cellules tumorales dans un volume de 0,5 ml.
- . Après la greffe tumorale, on effectue une répartition au hasard comme indiqué plus haut, pour obtenir une série témoin de contrôle de 8 cages et 5 séries expérimentales, de 4 cages chacune et dont les souris sont destinées à être traitées par les composés de l'invention.

**4.3. Tumeur de Lewis**

- . On opère dans les conditions qui viennent d'être précédemment décrites à propos du melanome B16, en injectant les cellules tumorales, à raison de 2 x $10^6$ par souris, dans un volume de 0,2 ml.

**4.4 Protocole et traitement**

- . Le protocole est identique pour les 3 tumeurs et les 5 composés de l'invention à tester.
- . En ce qui concerne les séries expérimentales à traiter, chaque souris reçoit, aux jours 1,5 et 9, par voie intrapéritonéale, une dose de 20 mg/kg de composé à tester, dans un volume de 0,2 ml d'huile d'olive neutralisée et stérilisé.
- . En ce qui concerne les séries témoins, chaque souris reçoit, par voie intrapéritonéale, 0,2 ml d'huile d'olive neutralisée et stérilisée.

### 4.5. Résultats

. Séries contrôles (témoins)
La survie moyenne des souris témoins est exprimé en jours ± 2 écarts type de la moyenne (± 2 σ m):

- L 1210: 8,9 ± 0,26
- Tumeur 3LL de Lewis: 12,87 ± 0,79
- Mélanome B16 : 14,52 ± 0,80.

. Séries traitées (voir Tableau II ci-après)

- Les résultats sont exprimés par l'augmentation relative de la survie (T/C x 100) calculée à partir de la survie moyenne des animaux traités (T) et de celle des animaux de contrôle (C)
- Les souris survivant plus de 60 jours sont considérées comme guéries.
- Les chiffres entre parenthèses indiquent le pourcentage de souris guéries.
- Le signe ∞ signifie que 50 % au moins des souris sont guéries.
- Lorsque le pourcentage de souris guéries est inférieur à50%, les souris guéries sont considérées comme mortes à 60 jours pour le calcul du T/C x 100 (dans ce cas le T/C x 100 est donc plus ou moins sous évalué).
- Parmi les souris ayant vécu au moins 60 jours, et donc considérées comme guéries, certaines ont été sacrifiées au 60ème jour. Il n'a pas été relevé d'anomalie lors des examens macroscopiques des organes prélevés.
- Les autres souris ont été conservées et étaient encore en vie au 200ème jour; elles ne manifestent aucun trouble apparent dans leur comportement.
- Les animaux décédés au cours de l'expérimentation ont été autopsiées et il n' y a pas de mort par toxicité.

**Tableau II**

| Composé testé / Tumeur | I.C. 1183 | I.C 1184 | I.C. 1350 | I.C. 1373 | I.C. 1374 |
|---|---|---|---|---|---|
| Leucémie 1210 | ∞ (100) | 556 (47) | 362 (20) | ∞ (100) | ∞ (67) |
| Tumer 3LL De Lewis | ∞ (75) | ∞ (90) | 325 (40) | 316 (30) | ∞ (75) |
| Melanome B 16 | 234 (5) | 235 (15) | 225 (5) | 165 (0) | 246 (10) |

### 5. Détermination de l'activité "in vivo" des composés selon l'invention sur les tumeurs L1210 USA et L1210 IGR.

. La tumeur L1210 USA est plus résistante que la tumeur L1210 IGR.
. Les composés testés sont les composés IC 1183, IC 1184, IC 1350, IC 1373 et IC 1374.
. L'expérimentation est identique à celle décrite dans le paragraphe 4 précédent mais des doses plus faible ont été utilisées.
. On a inoculé aux souris, $10^5$ cellules tumorales de L1210 USA par voie intrapéritonéale dans un volume de 0,2 ml.
. Aux jours 1,5 et 9, les composés de l'invention à tester ont été injectés par voie intrapéritonéale, à une dose plus faible que celle utilisée dans l'expérimentation décrite en 4, c'est-à-dire à raison de 5 mg/kg.
. On a effectué le même protocole avec la tumeur L1210 IGR, en inoculant $10^5$ cellules tumorales de L1210 IGR par voie intrapéritonéale dans un volume de 0,2 ml puis, aux jours 1,5 et 9, on a injecté par voie intrapéritonéale 5 mg/kg de chacun des composés à tester.
Les résultats figurent dans les tableaux III et IV ci-après.

**Tableau III**

**Tumeur L1210 USA**

| | Témoins | IC 1350 | IC 1373 | IC 1374 | IC 1183 | IC 1184 |
|---|---|---|---|---|---|---|
| Survie <u>moyenne</u> ± 2 σ m | 8,6 ± 0,22 | 10,5 ± 0,74 | 10,6 ± 0,44 | 12,9 ± 0,55 | 13,5 ± 0.91 | 18 ± 1,19 |
| Variance ($\zeta^2_m$ | 0,013 | 0,0139 | 0,049 | 0,077 | 0,205 | 0,355 |
| T/C x 100 | | 126 | 123 | 150 | 157 | 209 |
| Survie <u>médiane</u> | 8,7 | 10,15 | 10,6 | 13,1 | 14,0 | 17,8 |
| T/C x 100 | | 118 | 122 | 151 | 161 | 205 |

**Tableau IV**

**Tumeur L 1210 IGR**

| | Témoins | I.C.1350 | I.C.1373 | I.C.1374 | I.C.1183 | I.C.1184 |
|---|---|---|---|---|---|---|
| Survie <u>moyens</u> ± 2 σ m | 9,05 ± 0,18 | 14,3 ± 1,23 | 12,9 ± 0,55 | 17,1 ± 2,10 | 18,7 ± 1,30 | |
| Variance ($ζ^2_m$ | 0,008 | 0,379 | 0,077 | 1,10 | 0,423 | |
| T/C x 100 | | 158 | 142 | 189 | 207 | 378 |
| Survie <u>médiane</u> | 9,05 | 14,25 | 13,1 | 16,75 | 19 | 24 |
| T/C x 100 | | 157 | 145 | 185 | 210 | 265 |

**6. Etude de la toxicité des composés selon l'invention.**

. Des examens histologiques ont été effectués sur les organes de souris $DBA_2$ inoculées par la tumeur L1210 IGR et traitées pendant 60 jours avec les produits IC 1183, IC 1184, IC 1350, IC 1373 et IC 1374, ainsi que sur des organes de souris $DBA_2$ témoins' c'est-à-dire inoculées par la tumeur L1210 IGR.

. Les organes soumis à ces examens histologiques sont le foie, les reins, la rate, les glandes surénales et les poumons.

. Les examens ont montré que les animaux témoins inoculés par la L1210 IGR présentent une désorganisation cellulaire des hépatocytes.

. Par contre, la structure hépatique de souris inoculées par la L1210 TGR, puis traitées par les produits selon l'invention, ne montre pas de bouleversement aussi important que celui observée chez les souris témoins inoculées avec la L1210 IGR, et l'hypertrophie nucléaire hépatique ne semble donc pas liée à la toxicité propre des produits de l'invention

. Une étude hématologique a été faite sur certains des animaux traités avec les produits de l'invention. Il s'agit en particulier des souris $DBA_2$ inoculées par la leucémie L1210 et des souris C57/B16 ayant recu la tumeur de Lewis.

. L'étude comporte, à partir d'un prélèvement sanguin par ponction cardiaque, recueilli sur héparine, une numération globulaire (rouges et blancs), hématocrite, numération des plaquettes, formule sanguine. A partir d'étalements de moelle, d'origine fémorale et d'empreinte de rate, une étude succinte de ces centres hématopoietiques a également été faite.

. Une étude histologiques a également été pratiquée sur foie, rate, reins, surrénales, poumons, prélevés au moment du sacrifice.

. Dans l'ensemble, il n'a pas été constaté de perturbation importante ni dans la numération globulaire ni dans la formule sanguine. IL n'existe pas d'aplasie médullaire et les moelles observées sont riches en cellules de toutes sortes. Les rates apparaissent sensiblement normales.

**Conclusion générale**

. L'expérimentation animale effectuée avec le produit selon l'invention donne d'intéressants résultats lorsque le modèle choisi est le mélanome B16, et d'excellents résultats lorsque les modèles choisis sont la tumeur 3LL de Lewis, la tumeur L1210 IGR ainsi que la tumeur L1210 USA, plus résistante que L1210 IGR

Les composés selon l'invention sont donc particulièrement appropriés pour le traitement de divers cancers humains, notamment ceux qui sont sensibles à la chimiothérapie. Les composés de l'invention sont particulièrement appropriés pour le traitement de diverses formes de cancer répondant à cette condition et qui se trouvent identifiées dans les publications déjà mentionnées. Les composés de l'invention sont également appropriés au traitement des tumeurs cérébrales primitives et secondaires, des tumeurs broncho-pulmonaires, des tumeurs de la sphère ORL, des tumeurs digestives (gastrique, pancréatique, colique et rectale), des tumeurs du sein, des tumeurs génitales chez la femme, des tumeurs osseuses (ostéosarcomes, réticulo-sarcomes), des mélanomes, des hemato-sarcomes (lymphomes hodgkiniens et non hodgkiniens) des myélomes multiples.

L'invention concerne aussi les compositions pharmaceutiques comprenant les composés nouveaux susdits en association avec un véhicule pharmaceutique approprié au mode d'administration choisi.

L'invention concerne particulièrement des solutions stériles ou stérilisables, injectables ou propres à être utilisées pour la préparation, notamment extemporanée de solutions injectables aptes à être administrées par injections ou perfusions intraveineuses. Elle concerne, en particulier, des solutions hydroalcooliques physiologiquement acceptables.

Les produits selon l'invention, peuvent par exemple être présentés sous forme de poudre lyophilisée, laquelle, pour être administrée, est préparée extemporanement par solubilisation à l'aide de solvant alcoolique stérile. La solution ainsi obtenue est ensuite diluée avec de l'eau stérile apyrogène puis avant d'être administrée par perfusion intraveineuse, la solution est à nouveau rediluée dans du sérum salé isotonique à 9 ‰ ou glucose, isotonique à 5 %.

Les doses administrées quotidiennement doivent être suffisantes pour qu'une action puisse se manifester au

moins dans une proportion relativement importante de patients atteints de l'une ou l'autre des diverses formes de cancer qui sont ou seront accessibles à la chimiothérapie, cependant sans pour autant excéder celles pour lesquelles les composés deviendraient trop toxiques.

Plus particulièrement, les doses à administrer sont déterminées selon les modèles classiquement utilisés dans ce domaine et qui sont, par exemple, décrits dans les deux articles suivants:

- Cancer Research, 37, 1934 - 1937, juin 1977, PS SCHEIN.
- Cancer Chimiotherapy Reports, vol. 50, No 4. May 1966, E.J. FREIREICH.

Le modèle pour déterminer les doses appropriées pour un composé donné, consiste à déterminer la dose qui est tolérée par l'animal et qui correspond à environ 1/10 de la dose léthale (DL10) exprimée en $mg/m^2$ de surface corporelle. Les doses qui peuvent être utilisées chez l'homme correspondent de 1/3 à 1/10 de la dose DL10 mentionnée ci-dessus (Cf. Cancer Research, 37, 1935, colonne 1, Juin 1977).

A titre d'exemple, les doses quotidiennes administrées par voie générale, notamment par perfusion, et exprimées en mg/kg pourront varier d'environ 1 à environ 50 mg/kg, par exemple d'environ 3 mg/kg.

L'invention concerne également les autres formes d'administration, notamment, par voie orale (compositions solides ou liquides) ou par voie rectale (compositions glycérinées appropriées à cette dernière voie).

Ces intervalles de doses n'ont évidemment qu'une valeur d'indication.

Il est naturellement entendu que, dans ce type de thérapie, les doses administrées doivent, dans chaque cas, être évaluées par le clinicien, compte tenu de l'état du malade et de sa réactivité personnelle à l'égard des médicaments.

Un exemple de préparation pharmaceutique des produits selon l'invention comprend 100 mg de l'un au moins des produits selon l'invention, présenté sous forme de poudre lyophilisée stérile, en association avec une ampoule de solvant physiologiquement acceptable, notamment d'alcool, tel que l'éthanol, à raison de 5 ml par ampoule.

Du fait de leur activité particulièrement importante, les composés de l'invention sont également utiles comme produits de référence dans des études pharmacologiques, notamment, aux fins de comparaison de propriétés antitumorales de produits étudiés par rapport à un prodiut de référence.

**Revendications** pour les Etats Contracants: BE, CH, DE, FR, GB, IT, LI, LU, NL, SE

1. Dérivés de nitrosourées, caractérisé en ce qu'ils répondent a la formule (I) suivante:

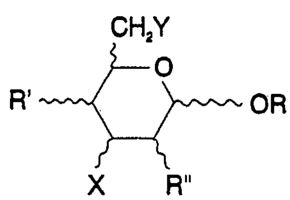

(I)

dans laquelle:

- R représente un atome d'hydrogène, un groupe alcoyle de 1 à 30, de préférence de 1 à 12 atomes de carbone ou un groupe aralcoyle de 7 à 12, de préférence de 7 à 9 atomes de carbone, éventuellement substitué ——— par un ou plusieurs, notamment jusqu'à 3 atomes d'halogène groupes $NO_2$, $NH_2$, $CF_3$ ou groupes alcoxy de 1 à 4 atomes de carbone
- X représente un groupe hydroxy ou un groupe $-NR_1R_2$
- Y représente un atome d'hydrogène un groupe hydroxy ou un groupe $-N$ (R'₁ / R'₂)

où $R_1$ et/ou $R'_1$ représentent chacun un atome d'hydrogène ou un groupe $-C(=O)-N(NO)-CH_2-CH_2$ Hal, Hal étant un halogène, de préférence Cl.

et $R_2$ et/ou $R'_2$ représentent chacun un atome d'hydrogène, un groupe alcoyle comprenant de 1 à 6 atomes de carbone, un groupe aralcoyle comprenant de 7 à 12, de préférence de 7 à 9 atomes de carbone, un groupe cycloalcoyle comprenant de 3 à 6 atomes de carbone, un groupe aryle de 4 à 10 atomes de carbone, les groupes aryle et aralcoyle étant éventuellement substitués par un ou plusieurs notamment jusqu'à 3, atomes d'halogène, groupes $NO_2$, $NH_2$, $CF_3$ ou groupes alcoxy de 1 à 4 atomes de carbone

- R' et R" représentent l'hydrogène, OH, OM, M représentant un groupe alcoyle comprenant de 1 à 30, de préférence de 1 à 12 atomes de carbone, un groupe aryle de 4 à 10 atomes de carbone, un groupe aralcoyle comprenant de 7

à 12, de préférence de 7 à 9 atomes de carbone, les groupes aryle et aralcoyle étant éventuellement substitués par un ou plusieurs notamment jusqu'à 3, atomes d'halogène, groupes $NO_2$, $NH_2$, $CF_3$ ou groupes alcoxy de 1 à 4 atomes de carbone, ou représentant un groupe acyle de 2 à 8 atomes de carbone, de préférence 2 ou 3, un groupe aroyle de 5 à 12, de préférence de 5 à 9 atomes de carbone, non substitué ou substitué par un ou plusieurs, notamment jusqu'à 3 atomes d'halogène, groupes $NO_2$, $NH_2$, $CF_3$, halogène, alcoxy, de 1 à 4 atomes de carbone, sous réserves qu'au moins

```
            R1
            /
X représente –N avec R1 représentant –C–N–CH2CH2Hal ou
            \                         ||  |
            R2                        O  NO
```

```
            R'1
            /
Y représente –N avec R'1 représentant –C–N–CH2CH2Hal
            \                          ||  |
            R'2                        O  NO
```

et sous réserve que soit R' représente l'hydrogène, soit R" représente l'hydrogène R' et R" ne pouvant pas représenter simultanément l'hydrogène.

2. Dérivés de nitrosourées selon la revendication 1, caractérisés en ce qu'ils répondent à la formule (II) suivante:

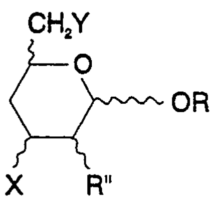

(II)

dans laquelle

- R représente un atome d'hydrogène, un groupe alcoyle de 1 à 30, de préférence de 1 à 12 atomes de carbone ou un groupe aralcoyle de 7 à 12, de préférence de 7 à 9 atomes de carbone, éventuellement substitué par un ou plusieurs, notamment jusqu'à 3, atomes d'halogène, groupes $NO_2$, $NH_2$, $CF_3$, ou groupes alcoxy de 1 à 4 atomes de carbone,
- X représente un groupe hydroxy ou un groupe $-NR_1R_2$
- Y représente un atome d'hydrogène ou un groupe

```
    R'1
    /
  –N
    \
    R'2
```

où $R_1$ et/ou $R'_1$ représentent chacun un atome d'hydrogène ou un groupe –C(=O)–N(NO)–$CH_2CH_2$Hal, Hal étant un halogène, de préférence Cl.

```
–C–N–CH2CH2Hal
 ||  |
 O  NO
```

et $R_2$ et/ou $R'_2$ représentent chacun un atome d'hydrogène, un groupe alcoyle comprenant de 1 à 6 atomes de carbone, un groupe aralcoyle comprenant de 7 à 12, de préférence de 7 à 9 atomes de carbone, un groupe cycloalcoyle comprenant de 3 à 6 atomes de carbone, un groupe aryle de 4 à 10 atomes de carbone, les groupes aryle et aralcoyle étant éventuellement substitués par un ou plusieurs notamment jusqu'à 3, atomes d'halogène, groupe $NO_2$, $NH_2$, $CF_3$ ou groupe alcoxy de 1 à 4 atomes de carbone.

R" représente de préférence OH, mais peut être remplacé par OM. M représentant un groupe alcoyle comprenant de 1 à 30, de préférence de 1 à 12 atomes de carbone, un groupe aryle de 4 à 10 atomes de carbone, un groupe aralcoyle comprenant de 7 à 12, de préférence de 7 à 9 atomes de carbone, les groupes aryle et aralcoyle étant éventuellement substitués par un ou plusieurs, notamment jusqu'à 3 atomes d'halogène, groupes $NO_2$, $NH_2$, $CF_3$ ou groupes alcoxy de 1 à 4 atomes de carbone, ou M représentant un groupe acyle de 2 à 8 atomes de carbone, de préférence 2 ou 3, un groupe aroyle de 5 à 12, de préférence de 5 à 9 atomes de carbone, non substitué ou substitué par un ou plusieurs, notamment jusqu'à 3, groupes $NO_2$, $NH_2$, $CF_3$ halogène alcoxy de 1 à 4 atomes de carbone,

$R_1$<br>
/<br>
sous réserve qu'au moins X représente –N avec $R_1$ représentant<br>
\\<br>
$R_2$

$R'_1$<br>
/<br>
–C–N–$CH_2CH_2$Hal ou Y représente –N avec $R'_1$ représentant<br>
|| |　　　　　　　　　　　　\\<br>
O NO　　　　　　　　　　　$R'_2$

–C–N–$CH_2CH_2$Hal<br>
|| |<br>
O NO

3. Dérivés de nitrosourées selon la revendication 2, caractérisés en ce qu'ils répondent à la formule III suivante:

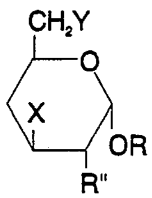

III

dans laquelle R, R", X et Y ont les significations indiquées à la revendication 2.

4. Dérivés de nitrosourées selon la revendication 2, caractérisés en ce qu'ils répondent à la formule IV suivante:

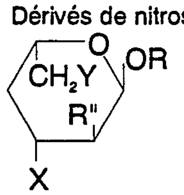

IV

dans laquelle R, R", X et Y ont les significations indiquées à la revendication 2.

5. Dérivés de nitrosourées selon la revendication 1, caractérisés en ce qu'ils répondent la formule IV suivante:

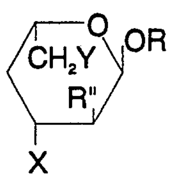

IV

dans laquelle R, R", X ont les significations indiquées à la revendication 1 et Y représente un groupe hydroxy.

6. Dérivés de nitrosourées selon la revendication 1, caractérisés en ce qu'ils répondent à la formule (V) suivante:

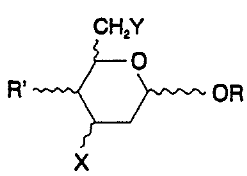

(V)

dans laquelle

- R représente un atome d'hydrogène, on groupe alcoyle de 1 à 30, de préférence de 1 à 12 atomes de carbone ou un groupe aralcoyle de 7 à 12, de préférence de 7 à 9 atomes de carbone, éventuellement substitué par un ou plusieurs, notamment jusqu'à 3, atomes d'halogène, groupes $NO_2$, $NH_2$, $CF_3$ ou groupes alcoxy de 1 à 4 atomes de carbone,

– X représente un groupe hydroxy ou un groupe $-NR_1R_2$

```
                                                                          R'1
                                                                         /
– Y représente un atome d'hydrogène un groupe hydroxy ou un groupe –N
                                                                         \
                                                                          R'2
```

où $R_1$ et/ou $R'_1$ représentent chacun un atome d'hydrogène ou
ou un groupe $-C-N-CH_2CH_2Hal$, Hal étant un halogène, de préférence Cl,

```
              ||  |
              O  NO
```

et $R_2$ et/ou $R'_2$ représentent chacun un atome d'hydrogène, un groupe alcoyle comprenant de 1 à 6 atomes de carbone, un groupe aralcoyle comprenant de 7 à 12, de préférence de 7 à 9 atomes de carbone, on groupe cycloalcoyle comprenant de 3 à 6 atomes de carbone, un groupe aryle de 4 à 10 atomes de carbone, les groupes aryle et aralcoyle étant éventuellement susbtitués par un ou plusieurs notamment jusqu'à 3, atomes d'halogène, groupes $NO_2$ $NH_2$, $CF_3$ ou groupes alcoxy de 1 à 4 atomes de carbone.

R' représente de préférence OH, mais OH peut être remplacé par OM M représentant on groupe alcoyle comprenant de 1 à 30, de préférence de 1 à 12 atomes de carbone, on groupe aryle de 4 à 10 atomes de carbone, un groupe aralcoyle comprenant de 7 à 12, de préférence de 7 à 9 atomes de carbone les groupes aryle et aralcoyle étant éventuellement substitués par ou plus notamment jusqu'à 3, atomes d'halogène, groupes $NO_2$, $NH_2$, $CF_3$ ou groupes alcoxy de 1 à 4 atomes de carbone, ou représentant un groupe acyle de 2 à 8 atomes de carbone, de préférence 2 ou 3, un groupe aroyle de 5 à 12, de préférence de 5 à 9 atomes de carbone non substitués ou substitué par un ou plusieurs, notamment jusqu'à 3, groupes $NO_2$, $NH_2$, $CF_3$, halogène, alcoxy de 1 à 4 atomes de carbone, sous réserve qu'au roi

```
               R1
              /
X représente –N avec R1 représentant –C–N–CH2CH2Hal ou
              \                       ||  |
               R2                     O  NO

               R'1
              /
Y représente –N avec R'1 représentant –C–N–CH2CH2Hal
              \                        ||  |
               R'2                     O  NO
```

7. Dérivés de nitrosourées selon la revendication 6, caractérisés en ce qu'ils répondent à la formule (VI) suivante:

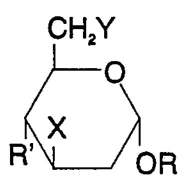

(VI)

dans laquelle R, R', X et Y ont les significations indiquées à la revendication 6.

8. Dérivés de nitrosourées selon la revendication 6, caractérisés en ce qu'ils répondent à la formule (VII) suivante:

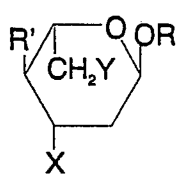

(VII)

dans laquelle R, R', X et Y ont les significations indiquées à la revendication 3.

9. Dérivés de nitrosourées selon les revendications 1 et 6 à 8, caractérisés en ce que

– R représente un groupe alcoyle de 1 à 12 atomes de carbone, aralcoyle de 7 à 12 atomes de carbone:
– R' ou R" représentent un groupe OM, M étant un groupe alcoyle comprenant de 1 à 12 atomes de carbone, un

groupe aryle comprenant de 4 à 10 atomes de carbone:

- X représente on groupe $NR_1R_2$, $R_1$ représentant $-C-N-CH_2CH_2Hal$,
  || |
  O NO
  Hal étant on halogène, de préférence Cl.
- Y représente un atome hydrogène ou un groupe hydroxy.

10. Dérivés de nitrosourées selon les revendications 1 et 6 à 8, caractérisés en ce que

- R représente un groupe alcoyle de 1 à 12 atomes de carbone, halogénoaralcoyle de 7 à 12 atomes de carbone;
- R' ou R" représente un groupe OM, M étant un groupe acyle de 2 à 8 atomes de carbone, un groupe aroyle de 5 à 12 atomes de carbone;
- X représente un groupe $-NR_1R_2$ R1 représentant $-C-N-CH_2CH_2Hal$,
  || |
  O NO
  Hal étant un halogène, notamment Cl.

- Y représente un atome d'hydrogène ou un groupe hydroxy.

11. Dérivés de nitrosourées selon les revendications 1 à 4 et 6 à 8 caractérisés en ce que

- R représente un groupe alcoyle de 1 à 12 atomes de carbone, un groupe aralcoyle de 7 à 12 atomes de carbone;
- R' ou R" représentent OH;
- X représente un groupe $NR_1R_2$, $R_1$ représentant $-C-N-CH_2CH_2Hal$,
  || |
  O NO
  Hal représentant un halogène, notamment Cl

- Y représente un atome d'hydrogène

12. Dérivés de nitrosourées selon les revendications 1 à 4 et 6 à 8, caractérisés en ce que

- R représente un groupe alcoyle de 1 à 12 atomes de carbone, un groupe aralcoyle de 7 à 12 atomes de carbone;
- R' ou R" représentent OH;
- X représente un groupe
  alcoylamino, dans lequel le groupe alcoyle a de 1 à 6 atomes de carbone, ou arylamino dans lequel le groupe aryle a de 4 à 10 atomes de carbone, et

- Y représente $NR'_1R'_2$, $R'_1$ représentant $-C-N-CH_2CH_2Hal$, Hal étant
  || |
  O NO
  un halogène, notamment Cl.

13. Dérivés de nitrosourées selon les revendications 1 à 4 et 6 à 8 caractérisés en ce que

- R représente un groupe alcoyle de 1 à 12 atomes de carbone, un groupe aralcoyle de 7 à 12 atomes de carbone;
- R' ou R" représentent OH;
- X représente un groupe hydroxy,

- Y représente $-NR'_1R'_2$, $R'_1$ représentant $-C-N-CH_2CH_2Hal$,
  || |
  O NO
  Hal représentant un atome d'halogène, notamment Cl.

14. Dérivés de nitrosourées selon les revendications 2 à 4, caractérisés en ce que:

- R représente un groupe alcoyle de 1 à 12 atomes de carbone, aralcoyle de 7 à 12 atomes de carbone;
- R' ou R" représente un groupe OM, M étant un groupe alcoyle comprenant de 1 à 12 atomes de carbone, un groupe aryle comprenant de 4 à 10 atomes de carbone;
- X représente un groupe $NR_1R_2$, $R_1$ représentant $-C-N-CH_2CH_2Hal$, Hal étant un halogène, de préférence Cl;
  || |
  O NO

- Y représente un atome d'hydrogène.

15. Dérivés de nitrosourées selon les revendications 2 à 4, caractérisés en ce que:

- R représente un groupe alcoyle de 1 à 12 atomes de carbone, halogénoaralcoyle de 7 à 12 atomes de carbone;
- R' ou R" représente un groupe OM, M étant un groupe acyle de 2 à 8 atomes de carbone, un groupe aroyle de 5 à 12 atomes de carbone;
- X représente un groupe $-NR_1R_2$, $R_1$ représentant $-\underset{\underset{O}{\|}}{C}-\underset{\underset{NO}{|}}{N}-CH_2CH_2Hal$, Hal étant un halogène, notamment Cl;
- Y représente un atome d'hydrogène.

16. Dérivés de nitrosourées selon la revendication 5, caractérisés en ce que

- R représente un groupe alcoyle de 1 à 12 atomes de carbone, aralcoyle de 7 à 12 atomes de carbone;
- R' ou R" représente un groupe OM, M étant un groupe alcoyle comprenant de 1 à 12 atomes de carbone, un groupe aryle comprenant de 4 à 10 atomes de carbone;
- X représente un groupe $NR_1R_2$, $R_1$ représentant $-\underset{\underset{O}{\|}}{C}-\underset{\underset{NO}{|}}{N}-CH_2CH_2Hal$, Hal étant un halogène, de préférence Cl;
- Y représente un groupe hydroxy.

17. Dérivés de nitrosourées selon la revendication 5, caractérisés en ce que

- R représente un groupe alcoyle de 1 à 12 atomes de carbone, halogénoaralcoyle de 7 à 12 atomes de carbone;
- R' ou R" représente un groupe OM, M étant un groupe acyle de 2 à 8 atomes de carbone, un groupe aroyle de 5 à 12 atomes de carbone;
- X représente un groupe $-NR_1R_2$, $R_1$ représentant $-\underset{\underset{O}{\|}}{C}-\underset{\underset{NO}{|}}{N}-CH_2CH_2Hal$, Hal étant un halogène, notamment Cl;
- Y représente un groupe hydroxy.

18. Composés selon la revendication 1, de formule

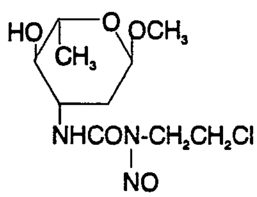

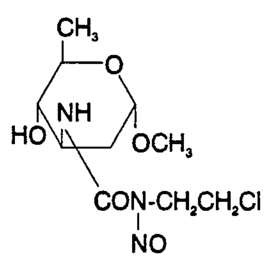

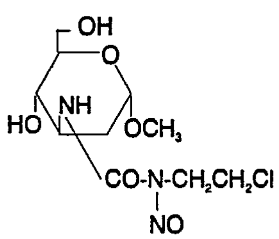

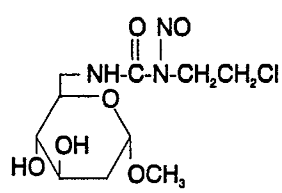

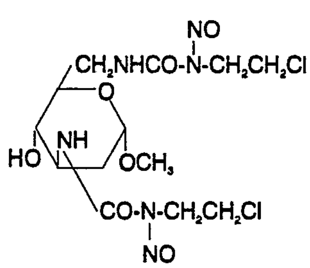

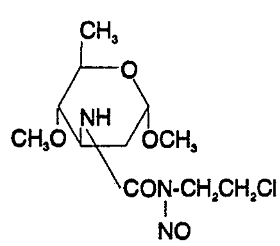

19. Procédé de préparation de composés selon l'une quelconque des revendications 1 à 18, caractérisé en ce qu'il consiste à faire réagir dans une première étape un groupe oside de formule générale Ibis ci-après:

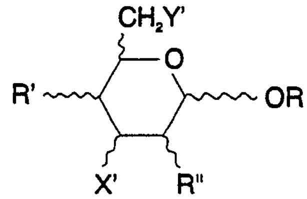

(I bis)

dans laquelle:

- R, R' et R" ont les significations indiquées aux revendications 1 à 18,
- X' représente un groupe hydroxy, ou $-NHR_2$:
- Y' représente un atome d'hydrogène, un groupe hydroxy ou $-NHR'_2$;
- $R_2$ et $R'_2$ identiques ou différents, représentant un groupe alcoyle de 1 à 12 atomes de carbone, un groupe aralcoyle de 7 à 12 atomes de carbone, cycloalcoyle de 3 à 6 atomes de carbone, les groupes aryle et aralcoyle étant éventuellement substitués par un ou plusieurs notamment jusqu'à 3 atomes d'halogène, groupes $NO_2$, $NH_2$, $CF_3$ ou groupes alcoxy de 1 à 4 atomes de carbone, et dans laquelle l'un au moins des groupes X' et Y' représente $-NHR_2$ ou $-NHR'_2$ sur un isocyanate d'halogéno–2–éthyle pour transformer le groupe $NHR_2$ ou $NHR'_2$ du composés de formule Ibis respectivement en $-NR_2CNHCH_2Hal$ ou $-NR'_2CNHCH_2CH_2Hal$,
  ||   ||
  O   O

  Hal étant un atome d'halogène, notamment le chlore et dans une deuxième étape, à soumettre le composé obtenu à l'issue de la première étape à une nitrosation, à l'aide d'un nitrite d'un métal alcalin, de préférence le nitrite de sodium pour transformer les groupes $-NR_2CNHCH_2CH_2Hal$
  ||
  O

  ou $-NR'_2CNHCH_2CH_2Hal$ respectivement en $-NR_2CNCH_2CH_2Hal$ ou
  ||   || |
  O   O NO

  $-NR'_2CNCH_2CH_2Hal$.
  || |
  O NO

20. Procédé de préparation selon la revendication 19, pour préparer des composés de formule VI:

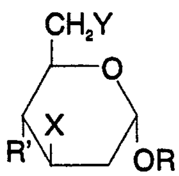

(VI)

caractérisé en ce qu'il consiste à faire réagir un composé de formule VIbis:

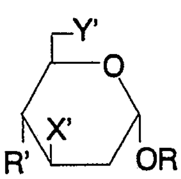

(VI bis)

ou R et R' sont tels que définis plus haut,

- X' représente un groupe $-NHR_2$ ou hydroxy;
- Y' représente un atome d'hydrogène, un groupe hydroxy ou $-NHR'_2$ et peut représenter on atome d'halogène lorsque X' représente un groupe hydroxy;
- $R_2$ et $R'_2$ identiques ou différents, représentent indépendamment l'un de l'autre un atome d'hydrogène, un groupe alcoyle de 1 à 30 atomes de carbone, un groupe aralcoyle de 7 à 12 atomes de carbone, aryle de 4 à 10 atomes de carbone, cycloalcoyle de 3 à 6 atomes de carbone, les groupes aryle et aralcoyle étant éventuellement substitués par un ou plusieurs notamment jusqu'à 3 atomes d'halogène, groupes $NO_2$, $NH_2$, $CF_3$ ou groupes alcoxy de 1 à

4 atomes de carbone, et dans laquelle l'un au moins des groupes X' et Y' représente $-NHR_2$ ou $-NHR'_2$ sur un isocyanate d'halogéno–2–éthyle pour transformer le groupe $NHR_2$ ou $NHR'_2$ du composé de formule VI bis respectivement en $-NR_2CNHCH_2CH_2Hal$ ou $-NR'_2CNHCH_2CH_2Hal$,
|| ||
O O

Hal étant un atome d'halogène, notamment le chlore, le composé obtenu à l'issue de l'étape précédente étant ensuite soumis à une nitrosation, à l'aide d'un nitrite d'un métal alcalin, de préférence le nitrite de sodium pour transformer les groupes $-NR_2CNHCH_2CH_2Hal$ et
||
O

$-NR'_2CNHCH_2CH_2Hal$ respectivement en $-NR_2CNCH_2CH_2Hal$ ou
|| || |
O O NO

$-NR'_2CNCH_2CH_2Hal$.
|| |
O NO

21. Procédé de préparation selon la revendication 19, pour préparer des composés de formule VII:

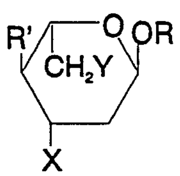

VII

caractérisé en ce qu'il consiste à faire réagir un composé de formule VIIbis:

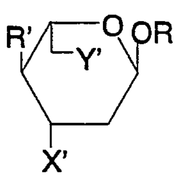

VII bis

où R et R' sont tels que définis plus haut,

- X' représente un groupe $-NHR_2$ ou hydroxy;
- Y' représente un atome d'hydrogène, un groupe hydroxy ou $-NHR'_2$ et peut représenter un atome d'halogène lorsque X' représente un groupe hydroxy,
- $R_2$ et $R'_2$ identiques ou différents, représentent un indépendamment l'un de l'autre un atome d'hydrogène, groupe alcoyle de 1 à 30 atomes de carbone, un groupe aralcoyle de 7 à 12 atomes de carbone, aryle de 4 à 10 atomes de carbone, cycloalcoyle de 3 à 6 atomes de carbone, les groupes aryle et aralcoyle étant éventuellement substitués par un ou plusieurs notamment jusqu'à 3 atomes d'halogène groupes $NO_2$, $NH_2$, $CF_3$ ou groupes alcoxy de 1 à 4 atomes de carbone, et dans laquelle l'un au moins des groupes X' et Y' représente $-NHR_2$ ou $-NHR'_2$ sur un isocyanate d'halogéno–2–éthyle pour transformer le groupe $-NHR_2$ ou $-NHR'_2$ du composé de formule VIIbis respectivement en $-NR_2CNHCH_2CH_2Hal$ ou $-NR'_2CNHCH_2CH_2Hal$,
|| ||
O O

Hal étant un atome d'halogène, notamment le chlore, le composé obtenu à l'issue de l'étape précédente étant ensuite soumis à une nitrosation, à l'aide d'un nitrite d'un métal alcalin, de préférence le nitrite de sodium pour transformer les groupes $-NR_2CNHCH_2CH_2Hal$ et
||
O

$-NR'_2CNHCH_2CH_2Hal$ respectivement en $-NR_2CNCH_2CH_2Hal$ et
|| || |
O O NO

$-NR'_2CNCH_2CH_2Hal$.
|| |
O NO

22. Procédé de préparation selon la revendication 19 pour préparer le composé de formule suivante:

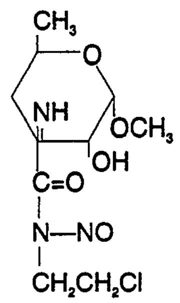

caractérisés en ce que on fait réagir un composé de formule:

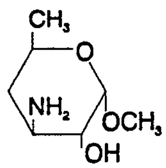

sur l'isocyanate de chloro–2 éthyle pour transformer le groupe $NH_2$ en $NHCONHCH_2CH_2Cl$, pour donner le composé de formule suivante:

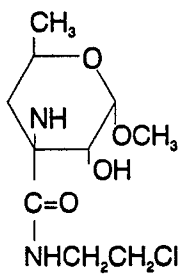

lequel est traité au nitrite de sodium dans l'acide formique pour transformer le groupe $NHCONHCH_2CH_2Cl$ en $NHCONHCH_2CH_2Cl$.
|
NO

23. Composition pharmaceutique, caractérisée en ce qu'elle comprend, en association avec un véhicule pharmaceutique, un composé selon l'une quelconque des revendications 1 à 18.

24. Composition pharmaceutique selon la revendication 23, caractérisée en ce qu'elle comprend 100 mg de l'un au moins des composés selon l'une quelconque des revendications 1 à 18, présenté sous forme de poudre lyophilisée stérile, en association avec une ampoule d'un solvant physiologiquement acceptable, notamment d'un alcool tel que l'éthanol à raison de 5 ml par ampoule.

**Revendications** pour l'Etat Contracant: AT

1. Procédé de préparation de dérivés de nitrosourées, de formule I suivante:

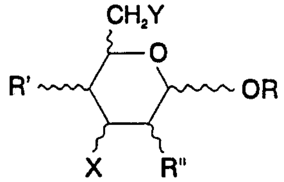

(I)

dans laquelle:

- R représente un atome d'hydrogène, un groupe alcoyle de 1 à 30, de préférence de 1 à 12 atomes de carbone ou un groupe aralcoyle de 7 à 12, de préférence de 7 à 9 atomes de carbone, éventuellement substitué par un ou plusieurs notamment jusqu'à 3, atomes d'halogène groupes $NO_2$, $NH_2$, $CF_3$ ou groupes alcoxy de 1 à 4 atomes de carbone

– X représente un groupe hydroxy ou un groupe $NR_1R_2$

– Y représente un atome d'hydrogène un groupe hydroxy ou un groupe N(R'$_1$)(R'$_2$)

où $R_1$ et/ou $R'_1$ représentent chacun un atome d'hydrogène ou un groupe $-C(O)-N(NO)-CH_2-CH_2Hal$, Hal étant un halogène, de préférence Cl.

et $R_2$ et/ou $R'_2$ représentent chacun un atome d'hydrogène, un groupe alcoyle comprenant de 1 à 6 atomes de carbone, un groupe aralcoyle comprenant de 7 à 12, de préférence de 7 à 9 atomes de carbone, un groupe cycloalcoyle comprenant de 3 à 6 atomes de carbone, un groupe aryle de 4 à 10 atomes de carbone, les groupes aryle et aralcoyle étant éventuellement substitués par un ou plusieurs notamment jusqu'à 3, atomes d'halogène, groupes $NO_2$, $NH_2$, $CF_3$ ou groupes alcoxy de 1 à 4 atomes de carbone

R' et R" représentent l'hydrogène, OH, OM, M représentant un groupe alcoyle comprenant de 1 à 30, de préférence de 1 à 12 atomes de carbone, un groupe aryle de 4 à 10 atomes de carbone, un groupe aralcoyle comprenant de 7 à 12, de préférence de 7 à 9 atomes de carbone, les groupes aryle et aralcoyle étant éventuellement substitués par un ou plusieurs notamment jusqu'à 3, atomes d'halogène, groupes $NO_2$, $NH_2$, $CF_3$ ou groupes alcoxy de 1 à 4 atomes de carbone, ou M représentant un groupe acyle de 2 à 8 atomes de carbone, de préférence 2 ou 3, un groupe aroyle de 5 à 12, de préférence de 5 à 9 atomes de carbone, non substitué ou substitué par un ou plusieurs, notamment jusqu'à 3, groupes $NO_2$, $NH_2$, $CF_3$, halogène, alcoxy de 1 à 4 atomes de carbone, sous réserve qu'au moins

X représente - N(R$_1$)(R$_2$) avec $R_1$ représentant $-C(O)-N(NO)-CH_2CH_2Hal$ ou

Y représente - N(R'$_1$)(R'$_2$) avec $R'_1$ représentant $-C(O)-N(NO)-CH_2CH_2Hal$

et sous réserve que soit R' représente l'hydrogène, soit R" représente l'hydrogène, R' et R" ne pouvant pas simultanément représenter l'hydrogène, caractérisé en ce qu'il consiste à faire réagir dans une première étape un groupe oside de formule 1 bis suivante:

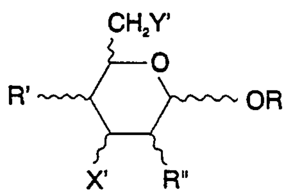

(I bis)

dans laquelle:

– R, R' et R" ont les significations indiquées ci-dessus,
– X' représente un groupe hydroxy, ou $-NHR_2$;
– Y' représente un atomes d'hydrogène, un groupe hydroxy, $NHR'_2$
– $R_2$ et $R'_2$ identiques ou différents, représentent, un groupe alcoyle de 1 à 12 atomes de carbone, un groupe aralcoyle de 7 à 12 atomes de carbone, cycloalcoyle de 3 à 6 atomes de carbone, les groupes aryle et aralcoyle étant éventuellement substitués par un ou plusieurs notamment jusqu'à 3, atomes d'halogène, groupes $NO_2$, $NH_2$, $CF_3$ ou groupes alcoxy de 1 à 4 atomes de carbone, et dans laquelle l'un au moins des groupes X'ou Y' représente $-NHR_2$ ou $-NHR'_2$ sur un isocyanate d'halogéno–2–éthyle pour transformer le groupe $NHR_2$ ou $NHR'_2$ du composé de formule I bis respectivement en $NR_2C(O)NHCH_2CH_2Hal$ ou $NR'_2C(O)NHCH_2CH_2Hal$,

Hal étant un atome d'halogène, notamment le chlore, et dans une seconde étape, à soumettre le composé obtenu à l'étape précédente a une nitrosation, à l'aide d'un nitrite d'un métal alcalin, de préférence, le nitrite de sodium pour

transformer les groupes $NR_2CNHCH_2CH_2Hal$ ou $NR'_2CNHCH_2CH_2Hal$
(with ‖ O under the C of each group)

respectivement en $NR_2C{-}NCH_2CH_2Hal$ où $NR'_2C{-}N{-}CH_2CH_2Hal$.
(with ‖ O under the C and ‖ NO under the N of each group)

2. Procédé selon la revendication 1, de préparation de dérivés de nitrosourées de formule II suivante:

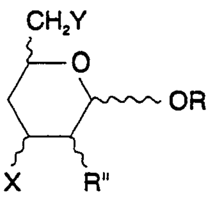

(II)

dans laquelle

- R représente un atome d'hydrogène un groupe alcoyle de 1 à 30, de préférence de 1 à 12 atomes de carbone ou un groupe aralcoyle de 7 à 12, de préférence de 7 à 9 atomes de carbone éventuellement substitué par un ou plusieurs, notamment jusqu'à 3, atomes d'halogène, groupes $NO_2$, $NH_2$, $CF_3$ ou groupes alcoxy de 1 á 4 atomes de carbone.
- X représente un groupe hydroxy ou un groupe $NR_1R_2$
- Y représente un atome d'hydrogène ou un groupe N(R'₁)(R'₂)

où $R_1$ et/ou $R'_1$ représente chacun un atome d'hydrogène ou un groupe $-C{-}N{-}CH_2CH_2Hal$ (‖ O, | NO), Hal étant un halogène de préférence Cl.

et $R_2$ et/ou $R'_2$ représente chacun un atome d'hydrogène un groupe alcoyle comprenant de 1 à 6 atomes de carbone un groupe aralcoyle comprenant de 7 à 12, de préférence de 7 à 9 atomes de carbone un groupe cycloalcoyle comprenant de 3 à 6 atomes de carbone les groupes aryle et aralcoyle étant éventuellement substitués par un ou plusieurs notamment jusqu'à 3, atomes d'halogène groupes $NO_2$, $NH_2$, $CF_3$ ou groupes alcoxy de 1 à 4 atomes de carbone.

R" représente de préférence OH, mais peut représenter OM, M représenter un groupe alcoyle comprenant de 1 à 30 de préférence de 1 à 12 atomes de carbone un groupe aryle de 4 à 10 atomes de carbone, un groupe aralcoyle comprenant de 7 à 12, de préférence de 7 à 9 atomes de carbone les groupes aryle et aralcoyle étant éventuellement substiués par un ou plusieurs, notamment jusqu'à 3 atomes d'halogène, groupes $NO_2$, $NH_2$, $CF_3$ ou groupes alcoxy de 1 à 4 atomes de carbone, ou M représentant un groupe acyle de 2 à 8 atomes de carbone de préférence 2 ou 3 un groupe aroyle de 5 à 12, de préférence 5 à 9 atomes de carbone, non substitué ou substitué par un ou plusieurs, notamment jusqu'à 3 groupes $NO_2$, $NH_2$, $CF_3$, halogène, alcoxy de 1 à 4 atomes de carbone,

sous réserve qu'au moins X représente - N(R₁)(R₂) avec $R_1$ représentant

$-C{-}N{-}CH_2CH_2Hal$ (‖ O, | NO) ou Y représente - N(R'₁)(R'₂) avec $R'_1$ représentant

$-C{-}N{-}CH_2CH_2Hal$. (‖ O, | NO)

3. Procédé selon la revendication 2 de préparation de dérivés de nitrosourées de formule III suivante:

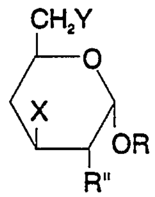

III

dans laquelle R, R", X et Y ont les significations indiquées à la revendication 2.

4. Procédé selon la revendication 2 de préparation de dérivés de nitrosourées de formule IV suivante:

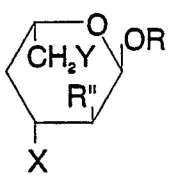

IV

dans laquelle R, R", X et Y ont les significations indiquées à la revendication 2.

5. Procédé selon la revendication 1 de préparation de dérivés de nitrosourées de formule IV suivante:

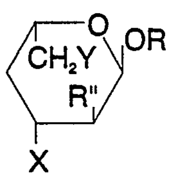

IV

dans laquelle

R, R", X ont les significations indiquées à la revendication 1 et Y représente un groupe hydroxy.

6. Procédé selon la revendication 1, de prépation de dérivés de nitrosourées de formule V suivante:

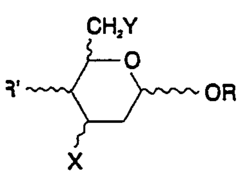

(V)

dans laquelle

- R représente un atome d'hydrogène, un groupe alcoyle de 1 à 30, de préférence de 1 à 12 atomes de carbone ou un groupe aralcoyle de 7 à 12, de préférence de 7 à 9 atomes de carbone, éventuellement substitué par un ou plusieurs, notamment jusqu'à 3, atomes d'halogène, groupes $NO_2$, $NH_2$, $CF_3$ ou groupes alcoxy de 1 à 4 atomes de carbone,
- X représente un groupe hydroxy ou un groupe $NR_1R_2$
- Y représente un atome d'hydrogène un groupe hydroxy ou un groupe $N(R'_1)(R'_2)$

où $R_1$ et/ou $R'_1$ représentent chacun un atome d'hydrogène ou un groupe $-C(=O)-N(NO)-CH_2CH_2Hal$, Hal étant un halogène, de préférence Cl,

et $R_2$ et/ou $R'_2$ représentent chacun un atome d'hydrogène, un groupe alcoyle comprenant de 1 à 6 atomes de carbone, un groupe aralcoyle comprenant de 7 à 12, de préférence de 7 à 9 atomes de carbone, un groupe

cycloalcoyle comprenant de 3 à 6 atomes de carbone, un groupe aryle de 4 à 10 atomes de carbone, les groupes aryle et aralcoyle étant éventuellement susbtitués par un ou plusieurs notamment jusqu'à 3, atomes d'halogène, groupes $NO_2$, $NH_2$, $CF_3$ ou groupes alcoxy de 1 à 4 atomes de carbone,

R' représente de préférence OH, mais peut représenter OM, M représentant un groupe alcoyle comprenant de 1 à 30, de préférence de 1 à 12 atomes de carbone, un groupe aryle de 4 à 10 atomes de carbone, un groupe aralcoyle comprenant de 7 à 12, de préférence de 7 à 9 atomes de carbone, les groupes aryle et aralcoyle étant éventuellement substitué par ou plusieurs notamment jusqu'à 3, atomes d'halogène, groupes $NO_2$, $NH_2$, $CF_3$ ou groupes alcoxy de 1 à 4 atomes de carbone, ou représentant un groupe acyle de 2 à 8 atomes de carbone, de préférence de 2 ou 3, un groupe aroyle de 5 à 12, de préférence de 5 à 9 atomes de carbone, non substitué ou substitué par un ou plusieurs, notamment jusqu'à 3, groupes $NO_2$, $NH_2$, $CF_3$, halogène, alcoxy de 1 à 4 atomes de carbone,

```
                                              R1
                                             /
sous réserve qu'au moins X représente - N avec R1 représentant
                                             \
                                              R2

                                         R'1
                                        /
–C–N–CH2CH2Hal ou Y représente - N avec R'1 représentant
 || |                                   \
 O NO                                    R'2

–C–N–CH2CH2Hal.
 || |
 O NO
```

7. Procédé selon la revendication 6, de préparation de composés de formule VI suivante:

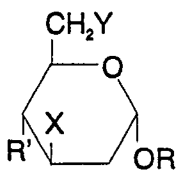

dans laquelle R, R', X et Y ont les significations indiquées à la revendication 6.

8. Procédé selon la revendication 6, de préparation de composés de formule VII suivante:

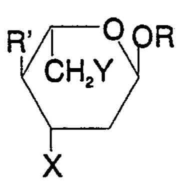

(VII)

dans laquelle R, R', X et Y ont les significations indiquées à la revendication 6.

9. Procédé selon les revendications 1 et 6 à 8 de préparation de composés de formule I, ,V, VI ou VII dans lesquels

- R représente un groupe alcoyle de 1 à 12 atomes de carbone, aralcoyle de 7 à 12 atomes de carbone;
- R' ou R" représentent un groupe OM, M étant un groupe alcoyle comprenant de 1 à 12 atomes de carbone, un groupe aryle comprenant de 4 à 10 atomes de carbone;
- X représente un groupe $NR_1R_2$, $R_1$ représentant –C–N–CH2CH2Hal,
```
                                         || |
                                         O NO
```
  Hal étant un halogène de préférence Cl.
- Y représente un atome d'hydrogène ou un groupe hydroxy.

10. Procédé selon les revendications 1 et 6 à 8, de préparation de composés de formule I, V, VI ou VII dans lesquels

- R représente un groupe alcoyle de 1 à 12 atomes de carbone, halogénoaralcoyle de 7 à 12 atomes de carbone;
- R' ou R" représente un groupe OM, M étant un groupe acyle de 2 à 8 atomes de carbone, un groupe aroyle de 7 à

12 atomes de carbone;

- X représente un groupe $-NR_1R_2$, $R_1$ représentant $-C-N-CH_2CH_2Hal$,
  || |
  O NO
  Hal étant un halogène, notamment Cl
- Y représente un atome d'hydrogène ou un groupe hydroxy

11. Procédé selon les revendications 1 à 4 et 6 à 8, de préparation de composés de formule I, II, V, VI ou VII dans lesquels

- R représente un groupe alcoyle de 1 à 12 atomes de carbone, un groupe aralcoyle de 7 à 12 atomes de carbone;
- R' ou R" représentent OH;
- X représente un groupe $NR_1R_2$, $R_1$ représentant $-C-N-CH_2CH_2Hal$,
  || |
  O NO
  Hal représentant un halogène, notamment Cl
- Y représente un atome d'hydrogène.

12. Procédé selon les revendications 1 à 4 et 6 à 8 de préparation de composés de formule I, II, V, VI ou VII dans lesquels

- R représente un groupe alcoyle de 1 à 12 atomes de carbone, un groupe aralcoyle de 7 à 12 atomes de carbone;
- R' ou R" représentent OH;
- X représente un groupe alcoylamino, dans lequel le groupe alcoyle a de 1 à 6 atomes de carbone, ou arylamino dans lequel le groupe aryle a de 4 à 10 atomes de carbone, et
- Y représente $NR'_1R'_2$, $R'_1$ représentant $C-N-CH_2CH_2Hal$, Hal étant un halogène, notamment Cl.
  || |
  O O

13. Procédé selon les revendications 1 à 4 et 6 à 8 de préparation de composés de formule I, II, V, VI ou VII dans lesquels

- R représente un groupe alcoyle de 1 à 12 atomes de carbone, un groupe aralcoyle de 7 à 12 atomes de carbone;
- R' ou R" représentent OH;
- X représente un groupe hydroxy, et
- Y représente $NR'_1R'_2$, $R'_1$ représentant $-C-N-CH_2CH_2Hal$,
  || |
  O NO
  Hal représentant un atome d'halogène, notamment Cl.

14. Procédé selon les revendications 2 à 4 de préparation de dérivés de nitrosourées caractérisé en ce que

- R représente un groupe alcoyle de 1 à 12 atomes de carbone, aralcoyle de 7 à 12 atomes de carbone;
- R' ou R" représente un groupe OM, M étant un groupe alcoyle comprenant de 1 à 12 atomes de carbone, un groupe aryle comprenant de 4 à 10 atomes de carbone;
- X représente un groupe $NR_1R_2$, $R_1$ représentant $-C-N-CH_2CH_2Hal$, Hal étant un halogène, de préférence Cl;
  || |
  O NO
- Y représente un atome d'hydrogène.

15. Procédé selon les revendications 2 à 4 de préparation de dérivés de nitrosourées caractérisé en ce que:

- R représente un groupe alcoyle de 1 à 12 atomes de carbone, halogénoaralcoyle de 7 à 12 atomes de carbone;
- R' ou R" représente un groupe OM, M étant un groupe acyle de 2 à 8 atomes de carbone, un groupe aroyle de 5 à 12 atomes de carbone;
- X représente un groupe $-NR_1R_2$, $R_1$ représentant $-C-N-CH_2CH_2Hal$, Hal étant un halogène, notamment Cl;
  || |
  O NO
- Y représente un atome d'hydrogène.

16. Procédé selon la revendication 5, de préparation de dérivés de nitrosourées caractérisé en ce que:

- R représente un groupe alcoyle de 1 à 12 atomes de carbone, aralcoyle de 7 à 12 atomes de carbone;
- R' ou R" représente un groupe OM, M étant un groupe. alcoyle comprenant de 1 à 12 atomes de carbone, un groupe aryle comprenant de 4 à 10 atomes de carbone;
- X représente un groupe $NR_1R_2$, $R_1$ représentant –C–N–$CH_2CH_2$Hal, Hal étant un halogène, de préférence Cl;
  || |
  O NO
- Y représente un groupe hydroxy.

17. Procédé selon la revendication 5, de préparation de dérivés de nitrosourées caractérisé en ce que:

- R représente un groupe alcoyle de 1 à 12 atomes de carbone, halogénoaralcoyle de 7 à 12 atomes de carbone;
- R' ou R" représente un groupe OM, M étant un groupe acyle de 2 à 8 atomes de carbone, un groupe aroyle de 5 à 12 atomes de carbone;
- X représente un groupe –$NR_1R_2$, $R_1$ représentant –C–N–$CH_2CH_2$Hal, Hal étant un halogène, notamment Cl;
  || |
  O NO
- Y représente un groupe hydroxy.

18. Procédé selon la revendication 1 de préparation de dérivé de nitrosourée de formule:

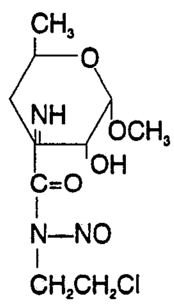

caractérisés en ce que on fait réagir un composé de formule:

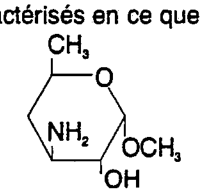

sur l'isocyanate de chloro–2 éthyle pour transformer le groupe $NH_2$ en $NHCONHCH_2CH_2Cl$, pour donner le composé de formule suivante:

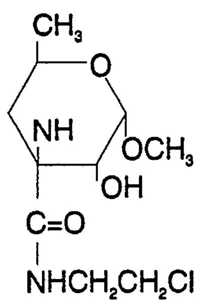

lequel est traité au nitrite de sodium dans l'acide formique pour transformer le groupe $NHCONHCH_2CH_2Cl$ en $NHCONHCH_2CH_2Cl$.
|
NO

19. Procédé selon la revendication 1, de préparation des composés de formule:

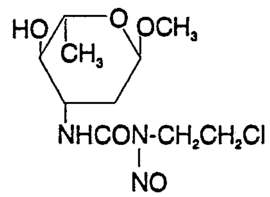

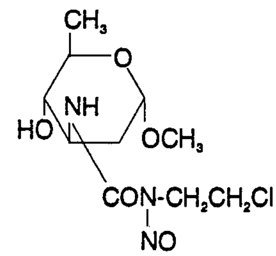

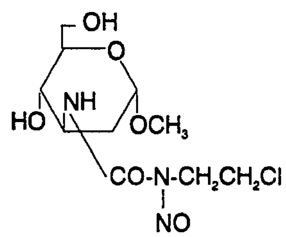

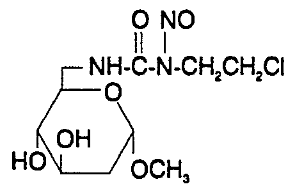

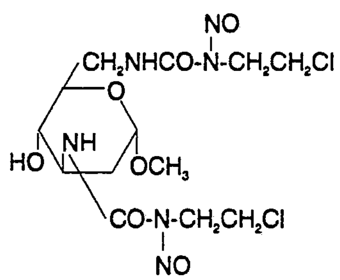

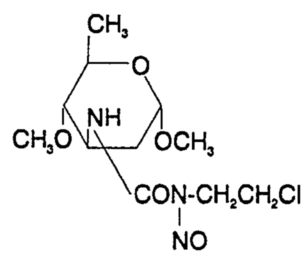

20. Procédé de préparation d'une composition pharmaceutique, caractérisé en ce que l'on associe l'un quelconque des composés obtenus à l'issue du procédé selon l'une quelconque des revendications 1 à 19, avec un véhicule pharmaceutiquement acceptable dans des conditions permettant la production de ladite composition pharmaceutique.

**Patentansprüche** für die Vertragsstaaten: BE, CH, DE, FR, GB, IT, LI, LU, NL, SE

1. Nitrosoharnstoffderivate, dadurch gekennzeichnet, daß sie der folgenden Formel (I) entsprechen:

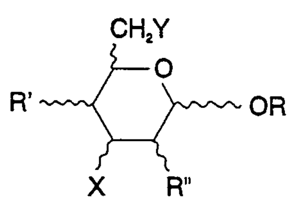

(I)

in der:

- R ein Wasserstoffatom, eine Alkylgruppe von 1 bis 30, vorzugsweise von 1 bis 12 Kohlenstoffatomen, oder eine Aralkylgruppe von 7 bis 12, vorzugsweise von 7 bis 9 Kohlenstoffatomen, repräsentiert, eventuell durch ein oder mehrere, insbesondere bis zu 3, Halogenatome, $NO_2$–, $NH_2$–, $CF_3$–Gruppen oder Alkoxygruppen von 1 bis 4 Kohlenstoffatomen substituiert;
- X eine Hydroxygruppe oder eine $-NR_1R_2-$Gruppe repräsentiert;
- Y ein Wasserstoffatom, eine Hydroxygruppe oder eine $N-R'_1$–Gruppe

  |
  $R'_2$

  repräsentiert,

worin $R_1$ und/oder $R'_1$ jeweils ein Wasserstoffatom oder eine $-C-N-CH_2-CH_2-$Hal-Gruppe repräsentiert, wobei Hal
||  |
O NO
ein Halogen, vorzugsweise Cl ist, und $R_2$ und/oder $R'_2$ jeweils ein Wasserstoffatom, eine Alkylgruppe mit von 1 bis 6 Kohlenstoffatomen, eine Aralkylgruppe mit von 7 bis 12, vorzugsweise von 7 bis 9 Kohlenstoffatomen, eine Cycloalkylgruppe mit von 3 bis 6 Kohlenstoffatomen, eine Arylgruppe mit von 4 bis 10 Kohlenstoffatomen repräsentiert, wobei die Aryl- und Aralkylgruppen eventuell durch ein oder mehrere, insbesondere bis zu 3, Halogenatome, $NO_2-$, $NH_2-$, $CF_3-$Gruppen oder Alkoxygruppen von 1 bis 4 Kohlenstoffatomen substituiert sind;

- R' und R" Wasserstoff, OH, OM repräsentieren, wobei M eine Alkylgruppe mit von 1 bis 30, vorzugsweise von 1 bis 12 Kohlenstoffatomen, eine Arylgruppe von 4 bis 10 Kohlenstoffatomen, eine Aralkylgruppe mit von 7 bis 12, vorzugsweise von 7 bis 9 Kohlenstoffatomen repräsentiert, wobei die Aryl- und Aralkylgruppen eventuell durch ein oder mehrere, insbesondere bis zu 3, Halogenatome, $NO_2-$, $NH_2-$, $CF_3-$Gruppen oder Alkoxygruppen von 1 bis 4 Kohlenstoffatomen substituiert sind, oder eine Acylgruppe von 2 bis 8 Kohlenstoffatomen, vorzugsweise 2 oder 3, eine Aroylgruppe von 5 bis 12, vorzugsweise von 5 bis 9 Kohlenstoffatomen, repräsentiert, und substituiert oder durch eine oder mehrere, insbesondere bis zu 3, $NO_2-$, $NH_2-$, $CF_3-$Gruppen, Halogen, Alkoxy von 1 bis 4 Kohlenstoffatomen substituiert, vorausgesetzt, daß zumindest:

- X $-N-R_1$ repräsentiert, wobei $R_1$ $-C-N-CH_2CH_2$Hal
  |  ||  |
  $R_2$  O NO
  repräsentiert, oder

- Y $-N-R'_1$ darstellt, wobei $R'_1$ $-C-N-CH_2CH_2$Hal dar
  |  ||  |
  $R'_2$  O NO
  darstellt,
  und vorausgesetzt, daß entweder R' Wasserstoff repräsentiert oder R" Wasserstoff repräsentiert, wobei R' und R" nicht gleichzeitig Wasserstoffatome sind.

2. Nitrosoharnstoffderivate nach Anspruch 1, dadurch gekennzeichnet, daß sie der folgenden Formel (II) entsprechen:

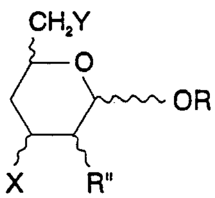

(II)

wobei:

- R ein Wasserstoffatom, eine Alkylgruppe von 1 bis 30, vorzugsweise von 1 bis 12 Kohlenstoffatomen, oder eine Aralkylgruppe von 7 bis 12, vorzugsweise von 7 bis 9 Kohlenstoffatomen, repräsentiert, eventuell durch ein oder mehrere, insbesondere bis zu 3, Halogenatome, $NO_2-$, $NH_2-$, $CF_3-$Gruppen oder Alkoxygruppen von 1 bis 4 Kohlenstoffatomen, substituiert;

- X eine Hydroxygruppe oder eine $-NR_1R_2-$Gruppe repräsentiert;
- Y ein Wasserstoffatom;
  oder eine $-N-R'_1-$Gruppe repräsentiert,
  |
  $R'_2$
  wobei $R_1$ und/oder $R'_1$ jeweils ein Wasserstoffatom oder eine $-C-N-CH_2CH_2$Hal-Gruppe repräsentieren, wobei Hal
  ||  |
  O NO
  ein Halogen, vorzugsweise Cl ist, und $R_2$ und/oder $R'_2$ jeweils ein Wasserstoffatom, eine Alkylgruppe mit von 1 bis 6 Kohlenstoffatomen, eine Aralkylgruppe mit von 7 bis 12, vorzugsweise von 7 bis 9 Kohlenstoffatomen, eine Cycloalkylgruppe mit von 3 bis 6 Kohlenstoffatomen, eine Arylgruppe von 4 bis 10 Kohlenstoffatomen repräsentiert, wobei die Aryl- und Aralkylgruppen eventuell durch ein oder mehrere, insbesondere bis zu 3, Halogenatome, $NO_2-$, $NH_2-$, $CF_3-$Gruppen oder Alkoxygruppen von 1 bis 4 Kohlenstoffatomen substituiert sind;

- R" vorzugsweise OH repräsentiert, aber auch durch OM ersetzt sein kann, wobei M eine Alkylgruppe mit von 1 bis 30, vorzugsweise von 1 bis 12 Kohlenstoffatomen, eine Arylgruppe von 4 bis 10 Kohlenstoffatomen, eine Aralkylgruppe mit von 7 bis 12, vorzugsweise von 7 bis 9 Kohlenstoffatomen repräsentiert, wobei die Aryl- und Aralkylgruppen eventuell durch ein oder mehrere, insbesondere bis zu 3, Halogenatome, $NO_2-$, $NH_2-$, $CF_3-$Gruppen oder

Alkoxygruppen von 1 bis 4 Kohlenstoffatomen substituiert sind, oder M eine Acylgruppe von 2 bis 8 Kohlenstoffatomen, vorzugsweise 2 oder 3, eine Aroylgruppe von 5 bis 12, vorzugsweise von 5 bis 9, Kohlenstoffatomen repräsentiert und substituiert oder durch ein oder mehrere, insbesondere bis zu 3, $NO_2$–, $NH_2$–, $CF_3$–Gruppen, Halogen, Alkoxy von 1 bis 4 Kohlenstoffatomen substituiert, vorausgesetzt, daß zumindest

X –N—$R_1$ repräsentiert, wobei $R_1$–C–N–$CH_2CH_2Hal$
   |                                   ‖ |
   $R_2$                                 O NO
repräsentiert, oder
Y –N—$R'_1$ repräsentiert, wobei $R'_1$ –C–N–$CH_2CH_2Hal$
   |                                   ‖ |
   $R'_2$                                O NO

repräsentiert.

3. Nitrosoharnstoffderivate nach Anspruch 1, *dadurch gekennzeichnet,* daß sie der folgenden Formel (III) entsprechen:

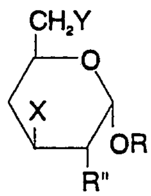

(III)

worin R, R", X und Y die im Anspruch 2 angegebene Bedeutung haben.

4. Nitrosoharnstoffderivate nach Anspruch 2, *dadurch gekennzeichnet,* daß sie der folgenden Formel (IV) entsprechen:

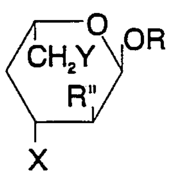

(IV)

worin R, R", X und Y die im Anspruch 2 angegebene Bedeutung haben.

5. Nitrosoharnstoffderivate nach Anspruch 1, *dadurch gekennzeichnet,* daß sie der folgenden Formel (IV) entsprechen:

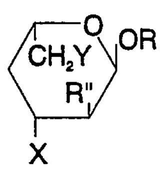

(IV)

worin R, R" und X die im Anspruch 1 angegebene Bedeutung haben und Y eine Hydroxygruppe repräsentiert.

6. Nitrosoharnstoffderivate nach Anspruch 1, *dadurch gekennzeichnet,* daß sie der folgenden Formel (V) entsprechen:

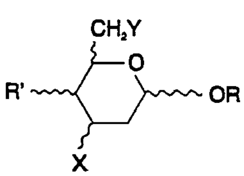

wobei:

- R ein Wasserstoffatom, eine Alkylgruppe mit von 1 bis 30, vorzugsweise von 1 bis 12 Kohlenstoffatomen, oder eine Aralkylgruppe mit von 7 bis 12, vorzugsweise von 7 bis 9 Kohlenstoffatomen repräsentiert, eventuell durch ein oder mehrere, insbesondere bis zu 3, Halogenatome, $NO_2$–, $NH_2$– oder $CF_3$–Gruppen oder Alkoxygruppen mit von 1 bis 4 Kohlenstoffatomen substituiert,

- X eine Hydroxygruppe oder eine $-NR_1R_2-$Gruppe repräsentiert,
- Y ein Wasserstoffatom, eine Hydroxygruppe oder eine N—R'$_1$–Gruppe
  |
  R'$_2$

repräsentiert, wobei $R_1$ und/oder $R'_1$ jeweils ein Wasserstoffatom oder eine –C–N–$CH_2$–$CH_2$Hal-Gruppe
|| |
O NO

wobei Hal Halogen, vorzugsweise Cl ist, und $R_2$ und/oder $R'_2$ jeweils ein Wasserstoffatom, eine Alkylgruppe mit von 1 bis 6 Kohlenstoffatomen, eine Aralkylgruppe mit von 7 bis 12, vorzugsweise von 7 bis 9 Kohlenstoffatomen, eine Cycloalkylgruppe mit von 3 bis 6 Kohlenstoffatomen, eine Arylgruppe mit von 4 bis 10 Kohlenstoffatomen repräsentiert, wobei die Aryl- und Aralkylgruppen eventuell durch ein oder mehrere, insbesondere bis zu 3, Halogenatome, $NO_2$–, $NH_2$– oder $CF_3$–Gruppen oder Alkoxygruppen von 1 bis 4 Kohlenstoffatomen substituiert sind,

- R' vorzugsweise OH repräsentiert, OH jedoch auch durch OM ersetzt sein kann, wobei M eine Alkylgruppe mit von 1 bis 30, vorzugsweise von 1 bis 12 Kohlenstoffatomen, eine Arylgruppe mit von 4 bis 10 Kohlenstoffatomen, eine Aralkylgruppe mit von 7 bis 12, vorzugsweise von 7 bis 9 Kohlenstoffatomen repräsentiert, wobei die Aryl- und Aralkylgruppen eventuell durch ein oder mehrere, insbesondere bis zu 3, Halogenatome, $NO_2$–, $NH_2$– oder $CF_3$–Gruppen oder Alkoxygruppen von 1 bis 4 Kohlenstoffatomen substituiert sind, oder M eine Acylgruppe von 2 bis 8 Kohlenstoffatomen, vorzugsweise 2 oder 3, eine Aroylgruppe von 5 bis 12, vorzugsweise von 5 bis 9, Kohlenstoffatomen repräsentiert, unsubstituiert oder durch eine oder mehrere, insbesondere bis zu 3, $NO_2$–, $NH_2$– oder $CF_3$–Gruppen, Halogenatome oder Alkoxygruppen von 1 bis 4 Kohlenstoffatomen substituiert, vorausgesetzt, daß zumindest

X –N—$R_1$ repräsentiert, wobei $R_1$ –C–N–$CH_2$–$CH_2$Hal
|  || |
$R_2$  O NO

repräsentiert,
oder Y –N—R'$_1$ repräsentiert, wobei R'$_1$
|
R'$_2$

–C–N–$CH_2$-$CH_2$Hal repräsentiert.
|| |
O NO

7. Nitrosoharnstoffderivate nach Anspruch 6, *dadurch gekennzeichnet,* daß sie der folgenden Formel (VI) entsprechen:

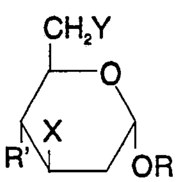

(VI)

worin R, R', X und Y die im Anspruch 6 angegebene Bedeutung haben.

8. Nitrosoharnstoffderivate nach Anspruch 6, *dadurch gekennzeichnet,* daß sie der folgenden Formel (VII) entsprechen:

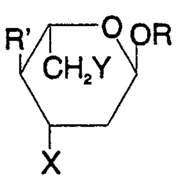

(VII)

worin R, R', X und Y die im Anspruch 3 angegebene Bedeutung haben.

9. Nitrosoharnstoffderivate nach Anspruch 1 und 6 bis 8, *dadurch gekennzeichnet,* daß

- R eine Alkylgruppe von 1 bis 12 Kohlenstoffatomen, eine Aralkylgruppe von 7 bis 12 Kohlenstoffatomen repräsentiert,

- R' oder R" eine OM-Gruppe repräsentieren, wobei M eine Alkylgruppe mit von 1 bis 12 Kohlenstoffatomen, eine Arylgruppe mit von 4 bis 10 Kohlenstoffatomen repräsentiert;
- X eine $NR_1R_2$–Gruppe repräsentiert, wobei $R_1$ –C–N–$CH_2CH_2$Hal repräsentiert, wobei Hal Halogen,
  ‖ |
  O NO
  vorzugsweise Cl ist,

- Y ein Wasserstoffatom oder eine Hydroxygruppe repräsentiert.

10. Nitrosoharnstoffderivate nach den Ansprüchen 1 und 6 bis 8, *dadurch gekennzeichnet,* daß:

- R eine Alkylgruppe von 1 bis 12 Kohlenstoffatomen, eine Halogenaralkylgruppe von 7 bis 12 Kohlenstoffatomen repräsentiert,
- R' oder R" eine OM-Gruppe repräsentieren, wobei M eine Acylgruppe von 2 bis 8 Kohlenstoffatomen, eine Aroylgruppe von 5 bis 12 Kohlenstoffatomen darstellt,
- X eine –$NR_1R_2$–Gruppe repräsentiert, wobei $R_1$ –C–N–$CH_2CH_2$Hal repräsentiert, worin Hal ein Halogen,
  ‖ |
  O NO
  vorzugsweise Cl ist,

- Y ein Wasserstoffatom oder eine Hydroxygruppe repräsentiert.

11. Nitrosoharnstoffderivate nach den Ansprüchen 1 bis 4 und 6 bis 8, *dadurch gekennzeichnet,* daß:

- R eine Alkylgruppe von 1 bis 12 Kohlenstoffatomen, eine Aralkylgruppe von 7 bis 12 Kohlenstoffatomen repräsentiert,
- R' oder R" OH repräsentieren,
- X eine $NR_1R_2$–Gruppe repräsentiert, wobei $R_1$ –C–N–$CH_2CH_2$Hal repräsentiert, wobei Hal ein Halogen,
  ‖ |
  O NO
  vorzugsweise Cl repräsentiert,

- Y ein Wasserstoffatom repräsentiert.

12. Nitrosoharnstoffderivate nach den Ansprüchen 1 bis 4 und 6 bis 8, *dadurch gekennzeichnet,* daß:

- R eine Alkylgruppe mit von 1 bis 12 Kohlenstoffatomen, eine Aralkylgruppe von 7 bis 12 Kohlenstoffatomen repräsentiert,
- R' oder R" OH repräsentieren,
- X eine Alkylaminogruppe, worin die Alkylgruppe von 1 bis 6 Kohlenstoffatomen aufweist, oder eine Arylaminogruppe repräsentiert, worin die Arylgruppe von 4 bis 10 Kohlenstoffatomen aufweist, und
- Y $NR'_1R'_2$ repräsentiert, wobei $R'_1$ –C–N–$CH_2CH_2$Hal
  ‖ |
  O NO
  repräsentiert, wobei Hal ein Halogen, vorzugsweise Cl ist.

13. Nitrosoharnstoffderivate nach den Ansprüchen 1 bis 4 und 6 bis 8, *dadurch gekennzeichnet,* daß:

- R eine Alkylgruppe von 1 bis 12 Kohlenstoffatomen, eine Aralkylgruppe von 7 bis 12 Kohlenstoffatomen repräsentiert,
- R' oder R" OH repräsentieren,
- X eine Hydroxygruppe repräsentiert,
- Y eine $NR'_1R'_2$–Gruppe repräsentiert, wobei $R'_1$ –C–N–$CH_2CH_2$Hal repräsentiert, worin Hal ein Halogen-
  ‖ |
  O NO
  atom, vorzugsweise Cl ist.

14. Nitrosoharnstoffderivat nach den Ansprüchen 2 bis 4, *dadurch gekennzeichnet,* daß:

- R eine Alkylgruppe mit von 1 bis 12 Kohlenstoffatomen, eine Aralkylgruppe mit von 7 bis 12 Kohlenstoffatomen repräsentiert,
- R' oder R" eine OM-Gruppe repräsentieren, wobei M eine Alkylgruppe mit von 1 bis 12 Kohlenstoffatomen, eine Arylgruppe mit von 4 bis 10 Kohlenstoffatomen ist,

- X eine $NR_1R_2$–Gruppe repräsentiert, wobei $R_1$ –C–N–$CH_2CH_2$Hal repräsentiert, worin Hal ein Halogen,
  || |
  O NO
  vorzugweise Cl ist,
- Y ein Wasserstoffatom repräsentiert.

15. Nitrosoharnstoffderivate nach den Ansprüchen 2 bis 4, *dadurch gekennzeichnet,* daß:

- R eine Alkylgruppe von 1 bis 12 Kohlenstoffatomen, eine Halogenaralkylgruppe von 7 bis 12 Kohlenstoffatomen repräsentieren,
- R' oder R" eine OM-Gruppe repräsentieren, wobei M eine Acylgruppe mit von 2 bis 8 Kohlenstoffatomen, eine Aroylgruppe mit 5 bis 12 Kohlenstoffatomen ist,
- X eine –$NR_1R_2$–Gruppe repräsentiert, wobei $R_1$ –C–N–$CH_2CH_2$Hal repräsentiert, wobei Hal ein Halogen,
  || |
  O NO
  vorzugsweise Cl ist,
- Y ein Wasserstoffatom repräsentiert.

16. Nitrosoharnstoffderivate nach Anspruch 5, *dadurch gekennzeichnet,* daß:

- R eine Alkylgruppe von 1 bis 12 Kohlenstoffatomen, eine Aralkylgruppe von 7 bis 12 Kohlenstoffatomen repräsentiert,
- R' oder R" eine OM-Gruppe repräsentieren, wobei M eine Alkylgruppe mit von 1 bis 12 Kohlenstoffatomen, eine Arylgruppe mit von 4 bis 10 Kohlenstoffatomen ist,
- X eine $NR_1R_2$–Gruppe repräsentiert, wobei $R_1$ –C–N–$CH_2CH_2$Hal repräsentiert, wobei Hal ein Halogen,
  || |
  O NO
  vorzugsweise Cl ist, und
- Y eine Hydroxygruppe repräsentiert.

17. Nitrosoharnstoffderivate nach Anspruch 5, *dadurch gekennzeichet,* daß:

- R eine Alkylgruppe von 1 bis 12 Kohlenstoffatomen, eine Halogenaralkylgruppe von 7 bis 12 Kohlenstoffatomen repräsentiert,
- R' oder R" eine OM-Gruppe repräsentieren, wobei M eine Acylgruppe mit von 2 bis 8 Kohlenstoffatomen, eine Aroylgruppe von 5 bis 12 Kohlenstoffatomen ist,
- X eine –$NR_1R_2$–Gruppe repräsentiert, wobei $R_1$ –C–N–$CH_2CH_2$Hal repräsentiert, worin Hal ein Halogen,
  || |
  O NO
  vorzugsweise Cl ist,
- Y eine Hydroxygruppe repräsentiert.

18. Verbindungen nach Anspruch 1 der Formeln:

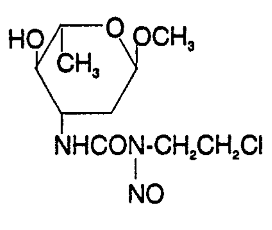

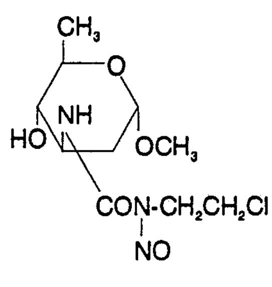

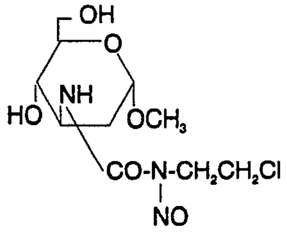

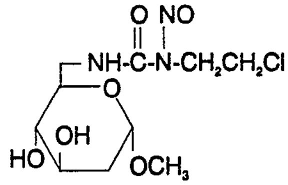

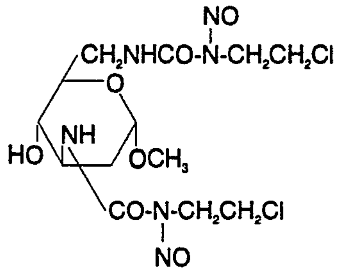

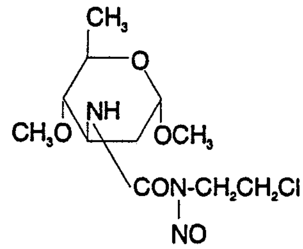

19. Verfahren zur Herstellung der Verbindungen nach einem der Ansprüche 1 bis 18, dadurch *gekennzeichnet,* daß es aus der Reaktion einer -osid-Gruppe der allgemeinen Formel (Ibis) in einer ersten Stufe:

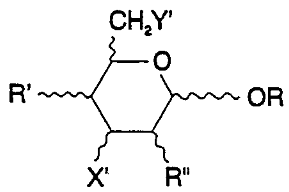

(I bis)

worin:

- R, R' und R" die in den Ansprüchen 1 bis 18 angegebene Bedeutung haben,
- X' eine Hydroxygruppe oder $-NHR_2$ repräsentiert,
- Y' ein Wasserstoffatom, eine Hydroxygruppe oder $-NHR'_2$ repräsentiert,

$-R_2$ und $R'_2$ identisch oder verschieden sind, eine Alkylgruppe von 1 bis 12 Kohlenstoffatomen, eine Aralkylgruppe von 7 bis 12 Kohlenstoffatomen, eine Cycloalkylgruppe von 3 bis 6 Kohlenstoffatomen repräsentieren, wobei die Aryl- und Aralkylgruppen eventuell durch ein oder mehrere, insbesondere bis zu 3, Halogenatome, $NO_2$–, $NH_2$–, $CF_3$–Gruppen oder Alkoxygruppen von 1 bis 4 Kohlenstoffatomen substituiert sind,
und worin zumindest eine der X'- und Y'–Gruppen $-NHR_2$ oder $-NHR'_2$ repräsentierten, auf 2–Halogenethylisocyanat, um die $-NHR_2$– bzw. $-NHR'_2$–Gruppe der Verbindung der Formel (Ibis) in $-NR_2CNHCH_2CH_2Hal$ oder –
||
O

$NR'_2CNHCH_2CH_2Hal$ umzuwandeln,
||
O

wobei Hal ein Halogenatom, vorzugsweise Chlor, ist, und aus der Unterziehung der am Ende der ersten Stufe erhaltenen Verbindung in einer zweiten Stufe einer Nitrosierung durch ein Alkalimetallnitrit, vorzugsweise Natriumnitrit, besteht, um die $-NR_2CNHCH_2CH_2Hal$–
||
O

bzw. $-NR'_2CNHCH_2CH_2Hal$-Gruppen in $-NR_2CNCH_2CH_2Hal$ oder
|| (O)    || | (O NO)

$NR'_2CNCH_2CH_2Hal$ umzuwandeln.
|| |
O NO

20. Herstellungsverfahren nach Anspruch 19, um die Verbindungen der Formel (VI) herzustellen:

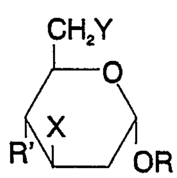

(VI)

*dadurch gekennzeichnet,* daß es aus der Reaktion einer Verbindung der Formel (VIbis):

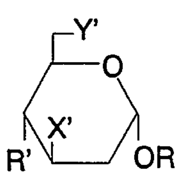

(VI bis)

worin:

- R und R' wie oben definiert sind,
- X' eine $-NHR_2$- oder eine Hydroxygruppe repräsentiert,
- Y' ein Wasserstoffatom, eine Hydroxygruppe oder eine $-NHR'_2$-Gruppe repräsentiert und ein Halogenatom repräsentieren kann, wenn X' eine Hydroxygruppe repräsentiert,
- $R_2$ und $R'_2$ identisch oder verschieden sind, unabhängig voneinander ein Wasserstoffatom, eine Alkylgruppe von 1 bis 30 Kohlenstoffatomen, eine Aralkylgruppe von 7 bis 12 Kohlenstoffatomen, eine Arylgruppe von 4 bis 10 Kohlenstoffatomen, eine Cycloalkylgruppe von 3 bis 6 Kohlenstoffatomen repräsentieren, wobei die Aryl- und Aralkylgruppen eventuell durch ein oder mehrere, insbesondere bis zu 3, Halogenatome, $NO_2-$, $NH_2-$, $CF_3-$ Gruppen oder Alkoxygruppen von 1 bis 4 Kohlenstoffatomen substituiert sind,
  und worin zumindest eine der X'- und Y'-Gruppen $-NHR_2$ oder $-NHR'_2$ repräsentieren, über 2-Halogenethylisocyanat besteht, um die $-NHR_2$- bzw. $-NHR'_2$-Gruppe der Verbindung der Formel (VIbis) in $-NHR_2C(=O)NHCH_2CH_2Hal$ oder $-NR'_2C(=O)NHCH_2CH_2Hal$ umzuwandeln, wobei Hal ein Halogenatom, insbesondere Chlor, ist, wobei die am Ende des vorhergehenden Schrittes erhaltene Verbindung der Nitrosierung durch ein Alkalimetallnitrit, vorzugsweise Natriumnitrit, unterzogen wird, um die $-NR_2C(=O)NHCH_2CH_2Hal$- bzw. $-NR'_2C(=O)NHCH_2CH_2Hal$-Gruppen in $-NR_2C(=O)N(NO)CH_2CH_2Hal$ oder $-NR'_2C(=O)N(NO)CH_2CH_2Hal$ umzuwandeln.

21. Herstellungsverfahren nach Anspruch 19, um Verbindungen der Formel (VII) herzustellen:

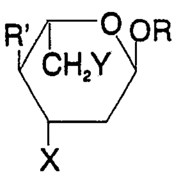

(VII)

*dadurch gekennzeichnet,* daß es aus der Reaktion einer Verbindung der Formel (VIIbis):

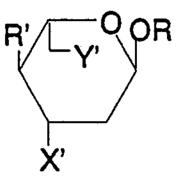

(VII bis)

worin:

- R und R' wie oben definiert sind,
- X' eine $-NHR_2$- oder eine Hydroxygruppe repräsentiert,
- Y' ein Wasserstoffatom, eine Hydroxygruppe oder eine $-NHR'_2$-Gruppe repräsentiert und ein Halogenatom repräsentieren kann, wenn X' eine Hydroxygruppe repräsentiert,
  $-R_2$ und $R'_2$ identisch oder verschieden sind, unabhängig voneinander ein Wasserstoffatom, eine Alkylgruppe von 1 bis 30 Kohlenstoffatomen, eine Aralkylgruppe von 7 bis 12 Kohlenstoffatomen, eine Arylgruppe von 4 bis 10 Kohlenstoffatomen, eine Cycloalkylgruppe von 3 bis 6 Kohlenstoffatomen repräsentieren, wobei die Aryl- und Aralkylgruppen eventuell durch ein oder mehrere, insbesondere bis zu 3, Halogenatome, $NO_2-$, $NH_2-$, $CF_3$-Gruppen oder Alkoxygruppen von 1 bis 4 Kohlenstoffatomen substituiert sind, und worin zumindest eine der X'- und Y'-Gruppen $-NHR_2$ oder $-NHR'_2$ repräsentieren, über 2-Halogenethylisocyanat besteht, um die $-NHR_2$- bzw. $-NHR'_2$-Gruppe der Verbindung der Formel (VIIbis) in $-NR_2C(=O)NHCH_2CH_2Hal$ oder $-NR'_2C(=O)NHCH_2CH_2Hal$ umzuwandeln, worin Hal ein Halogenatom, insbesondere Chlor, ist, wobei die am Ende des vorhergehenden Schrittes erhaltene Verbindung der Nitrosierung durch ein Alkalimetallnitrit, vorzugsweise Natriumnitrit, unterzogen wird, um die -$NR_2C(=O)NHCH_2CH_2Hal$- bzw.

$-NR'_2CNHCH_2CH_2Hal$–Gruppen in $-NR_2CNCH_2CH_2Hal$ oder
||                                        || |
O                                        O NO

$-NR'_2CNCH_2CH_2Hal$ umzuwandeln.
|| |
O NO

22. Herstellungsverfahren nach Anspruch 19 zur Herstellung der Verbindungen der folgenden Formel

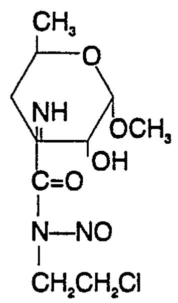

durch die Tatsache *gekennzeichnet,* daß eine Verbindung der Formel

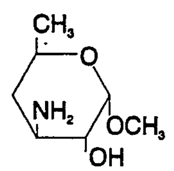

mit 2-Chlorethylisocyanat reagiert, um die $NH_2$–Gruppe in $NHCONHCH_2CH_2Cl$ umzuwandeln, um die Verbindung der folgenden Formel zu ergeben:

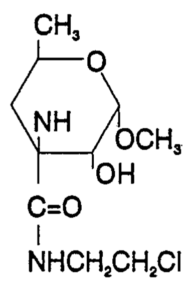

die mit Natriumnitrit in Ameisensäure behandelt wird, um die Gruppe $NHCONHCH_2CH_2Cl$ in $NHCONHCH_2CH_2Cl$ umzu-
||
NO

wandeln.

23. Pharmazeutische Zusammensetzung, *dadurch gekennzeichnet,* daß sie eine Verbindung nach einem der Ansprüche 1 bis 18 in Verbindung mit einem pharmazeutischen Träger umfaßt.

24. Pharmazeutische Zusammensetzung nach Anspruch 23, *dadurch gekennzeichnet,* daß sie 100 mg von zumindest einer der Verbindungen nach den Ansprüchen 1 bis 18 umfaßt, in Form eines sterilen gefriergetrockneten Pulvers in Verbindung mit einer Ampulle eines physiologisch verträglichen Lösungsmittels, insbesondere eines Alkohols, wie Ethanol, in einer Menge von 5 ml pro Ampulle angeboten.

**Patentansprüche** für den Vertragsstaat: AT

1. Verfahren zur Herstellung von Nitrosoharnstoffderivaten mit der folgenden Formel I:

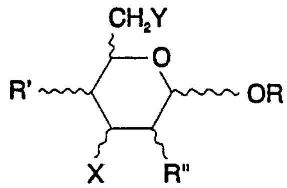

(I)

in der :

- R ein Wasserstoffatom, eine Alkylgruppe von 1 bis 30, vorzugsweise von 1 bis 12 Kohlenstoffatomen, oder eine Aralkyl
- Gruppe von 7 bis 12, vorzugsweise von 7 bis 9 Kohlenstoffatomen, repräsentiert, eventuell substituiert durch ein oder mehrere, insbesondere bis 3 Halogenatome, $NO_2$–, $NH_2$–, $CF_3$–Gruppen oder Alkoxygruppen von 1 bis 4 Kohlenstoffatomen;
- X eine Hydroxygruppe oder eine $NR_1R_2$–Gruppe repräsentiert;
- Y ein Wasserstoffatom,
  eine Hydroxygruppe oder eine N–$R'_1$–Gruppe repräsentiert
  |
  $R'_2$ .

  wobei $R_1$ und/oder $R'_1$ jeweils ein Wasserstoffatom oder eine

  C–N–$CH_2$–$CH_2$Hal–Gruppe
  || |
  O NO
  repräsentiert, wobei Hal ein Halogen ist, vorzugsweise Cl;
  und wobei $R_2$ und/oder $R'_2$
  jeweils ein Wasserstoffatom, eine Alkylgruppe mit 1 bis 6 Kohlenstoffatomen, eine Aralkylgruppe mit 7 bis 12, vorzugsweise 7 bis 9 Kohlenstoffatomen, eine Cycloalkylgruppe mit 3 bis 6 Kohlenstoffatomen, eine Arylgruppe mit 4 bis 10 Kohlenstoffatomen repräsentiert, wobei die Aryl- und Aralkylgruppen eventuell durch ein oder mehrere, insbesondere bis zu 3 Halogenatomen, $NO_2$–, $NH_2$–, $CF_3$–Gruppen oder Alkoxygruppen von 1 bis 4 Kohlenstoffatomen substituiert werden;

- R' und R" Wasserstoff, OH, OM repräsentieren, wobei M eine Alkylgruppe mit 1 bis 30, vorzugsweise 1 bis 12 Kohlenstoffatomen, eine Arylgruppe von 4 bis 10 Kohlenstoffatomen, eine Aralkylgruppe mit 7 bis 12, vorzugsweise 7 bis 9 Kohlenstoffatomen, repräsentiert, wobei die Aryl- und Aralkylgruppen eventuell durch ein oder mehrere, insbesondere bis zu 3 Halogenatomen, $NO_2$–, $NH_2$–, $CF_3$–Gruppen oder Alkoxygruppen von 1 bis 4 Kohlenstoffatomen, oder durch M ersetzt werden, wobei M eine Acylgruppe von 2 bis 8 Kohlenstoffatomen, vorzugsweise 2 oder 3, eine Aroylgruppe von 5 bis 12, vorzugsweise 5 bis 9 Kohlenstoffatomen, repräsentiert, nicht substituiert oder substituiert durch eine oder mehrere insbesondere bis zu 3, $NO_2$–, $NH_2$–, $CF_3$–, Halogen-, Alkoxygruppen von 1 bis 4 Kohlenstoffatomen, vorausgesetzt, daß zumindest
- X steht für
  –N—$R_1$, wobei $R'_1$ steht für –C–N–$CH_2CH_2$ Hal
  | || |
  $R_8$ O NO
  oder

- Y steht für
  –N—$R'_1$, wobei $R'_1$ steht für –C–N–$CH_2CH_2$Hal
  | || |
  $R'_2$ O NO

  und vorausgesetzt, daß entweder R' für Wasserstoff steht oder R" für Wasserstoff steht, wobei R' und R" nicht gleichzeitig für Wasserstoff stehen können, dadurch gekennzeichnet, daß es darin besteht, in einem ersten Schritt eine Osidgruppe der folgenden Formel Ia :

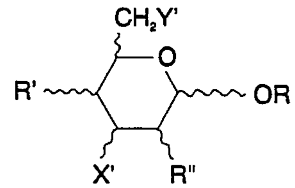

(ia)

wobei

- R, R' und R" die oben angegebenen Bedeutungen haben,
- X' eine Hydroxy- oder eine $NHR_2$–Gruppe repräsentiert;
- Y' ein Wasserstoffatom, eine Hydroxy-, eine $NHR'_2$–Gruppe repräsentiert;

wobei $R_2$ und $R'_2$ , identisch oder verschieden von einander, eine Alkylgruppe von 1 bis 12 Kohlenstoffatomen, eine Aralkylgruppe von 7 bis 12 Kohlenstoffatomen, eine Cycloalkylgruppe mit 3 bis 6 Kohlenstoffatomen repräsentiert, wobei die Aryl- und Aralkylgruppen eventuell durch ein oder mehrere, insbesondere bis zu 3 Halogenatomen, $NO_2$–, $NH_2$–, $CF_3$ –Gruppen oder Alkoxygruppen von 1 bis 4 Kohlenstoffatomen ersetzt werden;
und wobei mindestens eine der X'- oder Y'–Gruppen für $-NHR_2$ oder $-NHR'_2$ steht,
reagieren zu lassen auf ein Halogeno–2–Äthyl–Isocyanat, um die $NHR_2$– oder $NHR'_2$–Gruppe der Verbindung der Formel Ia in

$-NR_2CNHCH_2CH_2Hal$ bzw. $-NR'_2CNHCH_2CH_2Hal$ umzuwandeln,
 ||                                   ||
 O                                   O

wobei Hal ein Halogenatom ist, insbesondere Chlor, und in einem zweiten Schritt die im vorherigen Schritt erhaltene Verbindung einer Nitrosierung zu unterziehen, unter Verwendung eines Nitrits eines alkalischen Metalls, vorzugsweise Natriumnitrit,
um die $-NR_2CNHCH_2CH_2Hal$ oder $-NR'_2CNHCH_2CH_2Hal$-Gruppen
 ||                                   ||
 O                                   O

in $NR_2C$-$NCH_2CH_2Hal$ bzw. $-NR'_2C$–N–$CH_2CH_2Hal$ umzuwandeln.
 || |                                 || |
 O NO                               O NO

2. Verfahren nach Anspruch 1 zur Herstellung von Nitrosoharnstoffderivaten mit der folgenden Formel II:

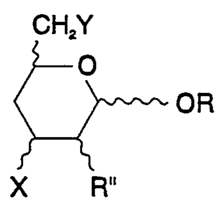

wobei

- R ein Wasserstoffatom, eine Alkylgruppe von 1 bis 30, vorzugsweise von 1 bis 12 Kohlenstoffatomen, oder eine Aralkylgruppe von 7 bis 12, vorzugsweise von 7 bis 9 Kohlenstoffatomen, repräsentiert, eventuell substituiert durch ein oder mehrere, insbesondere bis 3 Halogenatome, $NO_2$–, $NH_2$–, $CF_3$–Gruppen oder Alkoxygruppen von 1 bis 4 Kohlenstoffatomen;

- eine Hydroxygruppe oder eine $NR_1R_2$–Gruppe repräsentiert;
- Y ein Wasserstoffatom
  oder eine N—$R'_1$–Gruppe repräsentiert,
   |
   $R'_2$

  wobei $R_1$ und/oder $R'_1$
  jeweils ein Wasserstoffatom oder eine
  –C–N–$CH_2$–$CH_2Hal$-Gruppe
  || |
  O NO

  repräsentiert, wobei Hal ein Halogen ist, vorzugsweise Cl;
  und wobei $R_2$ und/oder $R'_2$

jeweils ein Wasserstoffatom, eine Alkylgruppe mit 1 bis 6 Kohlenstoffatomen, eine Aralkylgruppe mit 7 bis 12, vorzugsweise 7 bis 9 Kohlenstoffatomen, eine Cycloalkylgruppe mit 3 bis 6 Kohlenstoffatomen repräsentiert, wobei die Arylund Aryl-Alkylgruppen eventuell durch ein oder mehrere, insbesondere bis zu 3 Halogenatomen, $NO_2$–, $NH_2$–, $CF_3$–Gruppen oder Alkoxy-gruppen von 1 bis Kohlenstoffatomen substituiert werden;

- R" vorzugsweise OH präsentiert, aber auch OM repräsentieren kann, wobei M eine Alkylgruppe mit 1 bis 30, vorzugsweise 1 bis 12 Kohlenstoffatomen, eine Arylgruppe von 4 bis 10 Kohlenstoffatomen, eine Aralkylgruppe mit 7 bis 12, vorzugsweise 7 bis 9 Kohlenstoffatomen, repräsentiert, wobei die Aryl- und Aralkylgruppen eventuell durch ein oder mehrere, insbesondere bis zu 3 Halogenatomen, $NO_2$–, $NH_2$–, $CF_3$ –Gruppen oder Alkoxygruppen von 1 bis 4 Kohlenstoffatomen oder durch M ersetzt werden, wobei M eine Acylgruppe von 2 bis 8 Kohlenstoffatomen, vorzugsweise 2 oder 3, eine Aroylgruppe von 5 bis 12, vorzugsweise 5 bis 9 Kohlenstoffatomen, repräsentiert, nicht substituiert oder substituiert durch eine oder mehrere, insbesondere bis zu 3, $NO_2$–, $NH_2$, $CF_3$, Halogen- Alkoxygruppen von 1 bis 4 Kohlenstoffatomen, vorausgesetzt, daß zumindest
- X steht für
  –N—$R_1$, wobei $R_1$ steht für –C–N–$CH_2CH_2$Hal
  | || |
  $R_2$ O NO
  oder
- Y steht für
  –N—$R'_1$, wobei $R'_1$ steht für –C–N–$CH_2CH_2$Hal
  | || |
  $R'_2$ O NO

3. Verfahren nach Anspruch 2 zur Herstellung von Nitrosoharnstoffderivaten mit der folgenden Formel III:

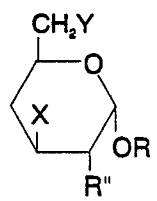

(III)

wobei R, R", X und Y die in Anspruch 2 angegebenen Bedeutungen haben.

4. Verfahren nach Anspruch 2 zur Herstellung von Nitrosoharnstoffderivaten mit der folgenden Formel IV:

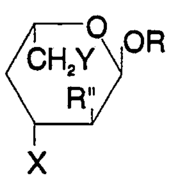

(IV)

wobei R, R", X und Y die in Anspruch 2 angegebenen Bedeutungen haben.

5. Verfahren nach Anspruch 1 zur Herstellung von Nitrosoharnstoffderivaten mit der folgenden Formel IV:

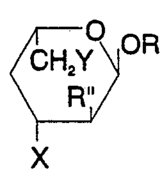

(IV)

wobei R, R", X die in Anspruch 1 angegebenen Bedeutungen haben und Y eine Hydroxygruppe repräsentiert.

6. Verfahren nach Anspruch 1 zur Herstellung von Nitrosoharnstoffderivaten mit der folgenden Formel V:

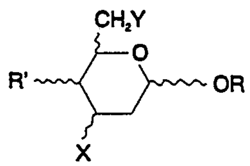

(V)

wobei

- R ein Wasserstoffatom, eine Alkylgruppe von 3 bis 30, vorzugsweise von 1 bis 12 Kohlenstoffatomen, oder eine AralkylGruppe von 7 bis 12, vorzugsweise von 7 bis 9 Kohlenstoffatomen, repräsentiert, eventuell substituiert durch ein oder mehrere, insbesondere bis 3 Halogenatome, $NO_2$–, $NH_2$–.
  $CF_3$–Gruppen oder Alkoxygruppen von 1 bis 4 Kohlenstoffatomen:
- X eine Hydroxygruppe oder eine $NR_1R_2$–Gruppe repräsentiert;
- Y ein Wasserstoffatom,
  eine Hydroxygruppe oder eine N—$R'_1$–Gruppe repräsentiert
  |
  $R'_2$
  wobei $R_1$ und/oder $R'_1$
  jeweils Wasserstoffatom oder eine –C–N–$CH_2$–$CH_2$ Hal-Gruppe
  || |
  O NO
  repräsentiert, wobei Hal ein Halogen ist, vorzugsweise Cl;
  und wobei $R_2$ und/oder $R'_2$
  jeweils ein Wasserstoffatom, eine Alkylgruppe mit 1 bis 6 Kohlenstoffatomen, eine Aralkylgruppe mit 7 bis 12, vorzugsweise 7 bis 9 Kohlenstoffatomen, eine Cycloalkylgruppe mit 3 bis 6 Kohlenstoffatomen, eine Arylgruppe mit 4 bis 10 Kohlenstoffatomen repräsentiert, wobei die Aryl- und Aralkylgruppen eventuell durch ein oder mehrere, insbesondere bis zu 3 Halogenatomen, $NO_2$–, $NH_2$–, $CF_3$–Gruppen oder Alkoxygruppen von 1 bis 4 Kohlenstoffatomen substituiert werden;
- R' vorzugsweise OH repräsentiert, aber auch OM repräsentieren kann, wobei M eine Alkylgruppe mit 1 bis 30, vorzugsweise 1 bis 12 Kohlenstoffatomen, eine Arylgruppe von 4 bis 10 Kohlenstoffatomen, eine Aralkylgruppe mit 7 bis 12, vorzugsweise 7 bis 9 Kohlenstoffatomen, repräsentiert, wobei die Aryl- und Aralkylgruppen eventuell durch ein oder mehrere, insbesondere bis zu 3 Halogenatomen, $NO_2$–, $NH_2$–, $CF_3$–Gruppen oder Alkoxygruppen von 1 bis 4 Kohlenstoffatomen, oder durch M ersetzt werden, wobei M eine Acylgruppe von 2 bis 8 Kohlenstoffatomen, vorzugsweise oder eine Aroylgruppe von 5 bis 12, vorzugsweise 5 bis 9 Kohlenstoffatomen, repräsentiert, nicht substituiert oder substituiert durch eine oder mehrere, insbesondere bis zu 3, $NO_2$–, $NH_2$–, $CF_3$, Halogen-, Alkoxygruppen von 1 bis 4 Kohlenstoffatomen, vorausgesetzt, daß zumindest

- X für –N—$R_1$ steht, wobei $R_1$ für –C–N–$CH_2CH_2$Hal steht
  | || |
  $R_2$ O NO
  oder
- Y für –N—$R'_1$ steht, wobei $R'_1$ für –C–N–$CH_2CH_2$Hal steht
  | || |
  $R'_2$ O NO

7. Verfahren nach Anspruch 6 zur Herstellung von Derivaten mit der folgenden Formel VI:

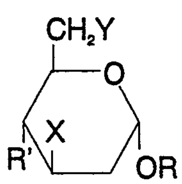

(VI)

wobei R, R', X und Y die in Anspruch 6 angegebenen Bedeutungen haben.

8. Verfahren nach Anspruch 6 zur Herstellung von Derivaten mit der folgenden Formel VII:

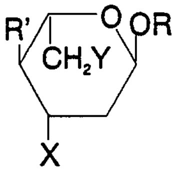

(VII)

wobei R, R', X und Y die in Anspruch 6 angegebenen Bedeutungen haben.

9. Verfahren nach den Ansprüchen 1 und 6 bis 8 zur Herstellung von Derivaten mit den Formeln I, V, VI oder VII, wobei

- R eine Alkylgruppe von 1 bis 12 Kohlenstoffatomen, eine Aralkyl-Gruppe von 7 bis 12 Kohlenstoffatomen repräsentiert:
  wobei R' oder R" eine OM-Gruppe repräsentieren, wobei M eine Alkylgruppe mit 1 bis 12 Kohlenstoffatomen, eine Arylgruppe von 4 bis 10 Kohlenstoffatomen ist;
- X $NR_1R_2$–Gruppe repräsentiert,
  wobei $R_1$ für C–N–$CH_2$–$CH_2$Hal- steht,
  || |
  O NO
  wobei Hal ein Halogen ist, vorzugsweise Cl;
- Y ein Wasserstoffatom oder eine Hydroxygruppe repräsentiert.

10. Verfahren nach den Ansprüchen 1 und 6 bis 8 zur Herstellung von Derivaten mit den Formeln I, V. VI oder VII, wobei

- R eine Alkylgruppe von 1 bis 12 Kohlenstoffatomen, eine Halogen-Aralkylgruppe von 7 bis 12 Kohlenstoffatomen repräsentiert;
  wobei R' oder R" eine OM-Gruppe repräsentieren, wobei M eine Acylgruppe mit 2 bis 8 Kohlenstoffatomen, eine Aroylgruppe von 7 bis Kohlenstoffatomen ist;
- X eine $NR_1R_2$-Gruppe repräsentiert,
  wobei $R_1$ für C–N–$CH_2$–$CH_2$Hal- steht,
  || |
  O NO
  wobei Hal ein Halogen ist, vorzugsweise Cl;
- Y ein Wasserstoffatom oder eine Hydroxygruppe repräsentiert.

11. Verfahren nach den Ansprüchen 1 bis 4 und 6 bis 8 zur Herstellung von Derivaten mit den Formeln I, II, V, VI oder VII, wobei

- R eine Alkylgruppe von 1 bis 12 Kohlenstoffatomen, eine Aralkylgruppe von 7 bis 12 Kohlenstoffatomen repräsentiert;
  wobei R' oder R" für OH stehen;
- X eine $NR_1R_2$–Gruppe repräsentiert,
  wobei $R_1$ für C–N–$CH_2$–$CH_2$Hal- steht,
  || |
  O NO
  wobei Hal ein Halogen ist, vorzugsweise Cl;
- Y ein Wasserstoffatom repräsentiert.

12. Verfahren nach den Ansprüchen 1 bis 4 und 5 bis 8 zur Herstellung von Derivaten mit den Formeln I, II, V, VI oder VII, wobei

- R eine Alkylgruppe von 1 bis 12 Kohlenstoffatomen, eine Aralkyl-Gruppe von 7 bis 12 Kohlenstoffatomen repräsentiert;
  wobei R' oder R" für OH stehen;
- X eine Alkylamino-Gruppe repräsentiert, wobei die Alkylgruppe von 1 bis 6 Kohlenstoffatome hat, oder eine Arylamino-Gruppe, wobei die Arylgruppe von 4 bis 13 Kohlenstoffatome hat, und
- Y für $NR'_1R'_2$ steht, wobei $R_1$ für C–N–$CH_2$–$CH_2$Hal- steht,
  | |
  O NO
  wobei Hal ein Halogen ist, vorzugsweise Cl:

13. Verfahren nach den Ansprüchen 1 bis 4 und 6 bis 8 zur Herstellung von Derivaten mit den Formeln I, II, V, VI oder VII, wobei

- R eine Alkylgruppe von 1 bis 12 Kohlenstoffatomen, eine Aralkyl-Gruppe von 7 bis 12 Kohlenstoffatomen repräsentiert;
  wobei R' oder R" für OH stehen;
- X eine Hydroxygruppe repräsentiert, und
- Y für $NR'_1R'_2$ steht, wobei $R_1$ für $C–N–CH_2–CH_2Hal$- steht,
  || |
  O NO
  wobei Hal ein Halogen ist, vorzugsweise Cl:

14. Verfahren nach den Ansprüchen 2 bis 4 zur Herstellung von Nitrosoharnstoffderivaten dadurch gekennzeichnet daß

- R eine Alkylgruppe von 1 bis 12 Kohlenstoffatomen, eine Aralkyl-Gruppe von 7 bis 12 Kohlenstoffatomen repräsentiert;
  wobei R' oder R" eine OM-Gruppe repräsentieren, wobei M eine Alkylgruppe mit 1 bis 12 Kohlenstoffatomen, eine Arylgruppe von 4 bis 10 Kohlenstoffatomen ist;
- X eine $NR_1R_2$–Gruppe repräsentiert,
  wobei R für $C–N–CH_2–CH_2Hal$- steht,
  || |
  O NO
  wobei Hal ein Halogen ist, vorzugsweise Cl;
- Y ein Wasserstoffatom repräsentiert.

15. Verfahren nach den Ansprüchen 2 bis 4 zur Herstellung von Nitrosoharnstoffderivaten dadurch gekennzeichnet, daß

- R eine Alkylgruppe von 1 bis 12 Kohlenstoffatomen, eine Halogen- Aralkyl-Gruppe von 7 bis 12 Kohlenstoffatomen repräsentiert;
  wobei R' oder R" eine OM-Gruppe repräsentieren, wobei M eine Acylgruppe mit 2 bis 8 Kohlenstoffatomen, eine Aroylgruppe von 5 bis 12 Kohlenstoffatomen ist;
- X eine $NR_1R_2$–Gruppe repräsentiert,
  wobei $R_1$ für $–C–N–CH_2–CH_2Hal$ steht,
  || |
  O NO
  wobei Hal ein Halogen ist, vorzugsweise Cl;
- Y ein Wasserstoffatom repräsentiert.

16. Verfahren nach Anspruch 5 zur Herstellung von Nitrosoharnstoffderivaten, dadurch gekennzeichnet daß

- R eine Alkylgruppe von 1 bis 12 Kohlenstoffatomen, eine Aralkyl-Gruppe von 7 bis 12 Kohlenstoffatomen repräsentiert:
  wobei R' oder R" eine OM-Gruppe repräsentieren, wobei M eine Alkylgruppe mit 1 bis 12 Kohlenstoffatomen, eine Arylgruppe von 4 bis 10 Kohlenstoffatomen ist;
- X eine $NR_1R_2$–Gruppe repräsentiert, wobei $R_1$, für $C–N–CH_2–CH_2Hal$ steht,
  || |
  O NO
  wobei Hal ein Halogen ist, verzugsweise Cl;
- Y eine Hydroxygruppe repräsentiert.

17. Verfahren nach Anspruch 5 zur Herstellung von Nitrosoharnstoffderivaten, dadurch gekennzeichnet, daß

- R eine Alkylgruppe von 1 bis Kohlenstoffatomen, eine Halogen Aralalkyl-Gruppe von 7 bis 12 Kohlenstoffatomen repräsentiert;
  wobei R' oder R" eine OM-Gruppe repräsentieren, wobei M eine Acyl-Gruppe mit 2 bis 8 Kohlenstoffatomen, eine Aroylgruppe von 5 bis 12 Kohlenstoffatomen ist;
- X eine $NR_1R_2$–Gruppe repräsentiert,
  wobei $R_1$ für $-C–N–CH_2–CH_2Hal$- steht,
  || |
  O NO
  wobei Hal ein Halogen ist, vorzugsweise Cl;
- Y eine Hydroxygruppe repräsentiert.

18. Verfahren nach Anspruch 1 zur Herstellung von Nitrosoharnstoffderivaten der Formel:

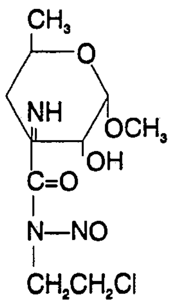

dadurch gekennzeichnet, daß man eine Verbindung mit der Formel:

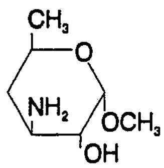

reagieren läßt auf 2–Chlor–Äthyl-Isocyanat, um die $NH_2$-Gruppe in $NHCONHCH_2CH_2Cl$ umzuwandeln, um die Verbindung mit folgender Formel zu erhalten:

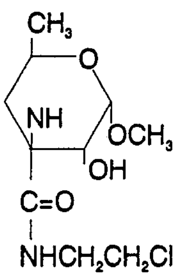

die mit Natriumnitrit in Ameisensäure behandelt wird, um die $NHCONHCH_2CH_2Cl$–Gruppe in $NHCON(NO)HCH_2CH_2Cl$ umzuwandeln.

19. Verfahren nach Anspruch 1 zur Herstellung von Verbindungen der Formel:

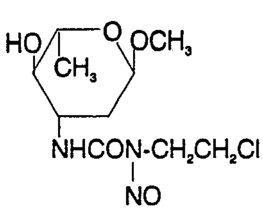

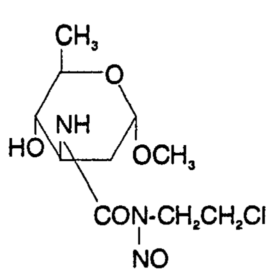

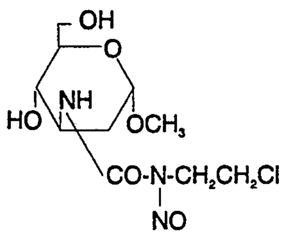

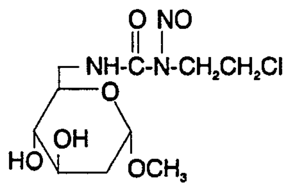

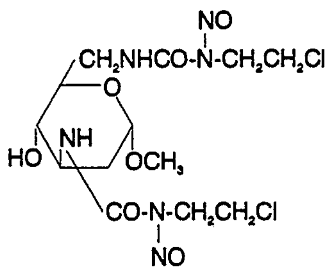

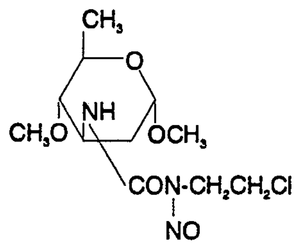

20. Verfahren zur Herstellung einer pharmazeutischen Zusammensetzung, dadurch gekennzeichnet, daß man eine beliebige der am Ende des Prozesses nach einer der Ansprüche 1 bis 19 erhaltenen Verbindungen mit einem pharmazeutisch akzeptablen Arzneistoffträger zu Bedingungen assoziiert, die die Produktion der genannten pharmazeutischen Zusammensetzung ermöglichen.

**Claims** for the Contracting States: BE, CH, DE, FR, GB, IT, LI, LU, NL, SE

1. Nitrosourea derivatives, characterized in that they correspond to the following formula (I):

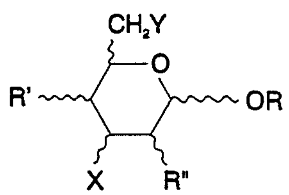

(I)

in which:

- R represents a hydrogen atom, an alkyl group from 1 to 30, preferably 1 to 12 carbon atoms or an aralkyl group from 7 to 12, preferably from 7 to 9 carbon atoms, optionally substituted by one or several, particularly up to 3, halogen atoms, $NO_2$, $NH_2$, $CF_3$ groups or alkoxy groups from 1 to 4 carbon atoms;
- X represents a hydroxy group or a $-NR_1R_2$ group;
- Y represents:
  - a hydrogen atom;
  - a hydroxy group or a $N-R'_1$ group where $R_1$
    $|$
    $R'_2$

  and/or $R'_1$ each represents a hydrogen atom or a
  $-C-N-CH_2-CH_2Hal$ group, Hal being a halogen,
  $\|\ \ |$
  O NO
  preferably Cl
  and $R_2$ and/or $R'_2$ each represents a hydrogen atom, an alkyl group comprising from 1 to 6 carbon atoms, an aralkyl group comprising from 7 to 12, preferably from 7 to 9 carbon atoms, a cycloalkyl group comprising from 3 to 6 carbon atoms, an aryl group from 4 to 10 carbon atoms, the aryl and aralkyl groups being optionally substituted by one or several, particularly up to 3, halogen atoms, $NO_2$, $NH_2$, $CF_3$ groups or alkoxy groups from 1 to 4 carbon atoms;
- R' and R" represent hydrogen, OH, OM, M representing an alkyl group comprising from 1 to 30, preferably from 1 to 12 carbon atoms, an aryl group from 4 to 10 carbon atoms, an aralkyl group comprising from 7 to 12, preferably from 7 to 9 carbon atoms, the aryl and aralkyl groups being optionally substituted by one or several, particularly up to 3, halogen atoms, $NO_2$, $NH_2$, $CF_3$ groups or alkoxy groups from 1 to 4 carbon atoms, or representing an acyl

group from 2 to 8 carbon atoms, preferably 2 or 3, an aroyl group from 5 to 12, preferably from 5 to 9 carbon atoms, unsubstituted or substituted by one or several, particularly up to 3, $NO_2$, $NH_2$, $CF_3$ groups, halogen, alkoxy from 1 to 4 carbon atoms;
provided that at least:

– X represents –N—$R_1$ with $R_1$ representing
\
$R_2$

–C–N–$CH_2CH_2$Hal or
|| |
O NO

– Y represents –N—$R'_1$ with $R'_1$ representing
|
$R'_2$

–C–N–$CH_2CH_2$Hal
|| |
O NO

and provided that either R' represents hydrogen or R" represents hydrogen, R' and R" being not simultaneously hydrogen atoms.

2. Nitrosourea derivatives according to claim 1, characterized in that they correspond to the following formula (II):

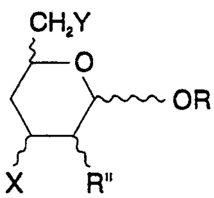

(II)

in which:

- R represents a hydrogen atom, an alkyl group of 1 from 30, preferably from 1 to 12 carbon atoms or an aralkyl group from 7 to 12, preferably from 7 to 9 carbon atoms, optionally substituted by one or several, particularly up to 3, halogen atoms, $NO_2$, $NH_2$, $CF_3$ groups or alkoxy groups from 1 to 4 carbon atoms;
- X represents a hydroxy group or a $-NR_1R_2$ group;
- Y represents:
  - a hydrogen atom;
  - or a –N—$R'_1$ group where
    |
    $R'_2$

  $R_1$ and/or $R'_1$ each represents a hydrogen atom or a –C–N–$CH_2CH_2$Hal group, Hal being a halogen,
  || |
  O NO

  preferably Cl,
  and $R_2$ and/or $R'_2$ each represents a hydrogen atom, an alkyl group comprising from 1 to 6 carbon atoms, an aralkyl group comprising from 7 to 12, preferably from 7 to 9 carbon atoms, a cycloalkyl group comprising from 3 to 6 carbon atoms, an aryl group of 4 to 10 carbon atoms, the aryl and aralkyl groups being optionally substituted by one or several, particularly up to 3, halogen atoms, $NO_2$, $NH_2$, $CF_3$ groups or alkoxy groups from 1 to 4 carbon atoms;
- R" represents preferably OH, but can be replaced by OM, M representing an alkyl group comprising from 1 to 30, preferably from 1 to 12 carbon atoms, an aryl group from 4 to 10 carbon atoms, an aralkyl group comprising from 7 to 12, preferably from 7 to 9 carbon atoms, the aryl and aralkyl groups being optionally substituted by one or several, particularly up to 3 halogen atoms, $NO_2$, $NH_2$, $CF_3$ groups or alkoxy groups from 1 to 4 carbon atoms, or M representing an acyl group from 2 to 8 carbon atoms, preferably 2 or 3, an aroyl group from 5 to 12, preferably from 5 to 9 carbon atoms, unsubstituted or substituted by one or several, particularly up to 3, $NO_2$, $NH_2$, $CF_3$ groups, halogen, alkoxy from 1 to 4 carbon atoms;
  provided that at least X represents –N—$R_1$ with $R_1$
  |
  $R_2$

  representing –C–N–$CH_2CH_2$Hal or
  || |
  O NO

Y represents –N—$R'_1$ with $R'_1$
|
$R'_2$

representing –C–N–$CH_2CH_2Hal$
|| |
O NO

3. Nitrosourea derivatives according to claim 2, characterized in that they correspond to the following formula (III):

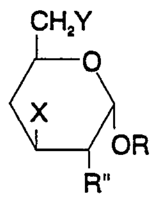

(III)

in which R, R", X and Y have the meanings mentioned in claim 2.

4. Nitrosourea derivatives according to claim 2, characterized in that they correspond to the following formula (IV):

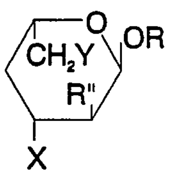

(IV)

in which R, R", X and Y have the meanings mentioned in claim 2.

5. Nitrosourea derivatives according to claim 1, characterized in that they correspond to the following formula (IV):

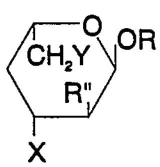

(IV)

in which R, R" and X have the meanings above mentioned in claim 1 and Y represents a hydroxy group.

6. Nitrosourea derivatives according to claim 1, characterized in that they correspond to the following formula (V):

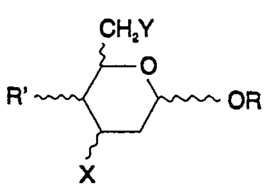

(V)

in which:

- R represents a hydrogen atom, an alkyl group from 1 to 30, preferably from 1 to 12 carbon atoms or an aralkyl group from 7 to 12, preferably from 7 to 9 carbon atoms, optionally substituted by one or several, particularly up to 3, halogen atoms, $NO_2$, $NH_2$ or CF3 groups or alkoxy groups having from 1 to 4 carbon atoms,
- X represents a hydroxy group or a $-NR_1R_2$ group
- Y represents a hydrogen atom,
  a hydroxy group or an N—$R'_1$
  |
  $R'_2$
  group where $R_1$ and/or $R'_1$ each represents a hydrogen atom or a

$-C-N-CH_2-CH_2Hal$ group, Hal being a halogen,
‖ |
O NO

preferably Cl,
and $R_2$ and/or $R'_2$ each represents a hydrogen atom, an alkyl group comprising from 1 to 6 carbon atoms, an aralkyl group comprising from 7 to 12, preferably from 7 to 9 carbon atoms, a cycloalkyl group comprising from 3 to 6 carbon atoms, an aryl group from 4 to 10 carbon atoms, the aryl and aralkyl groups being optionally substituted by one or several, particularly up to 3, halogen atoms, $NO_2$, $NH_2$ or $CF_3$ groups or alkoxy groups from 1 to 4 carbon atoms,

- R' represents preferably OH, but OH can be replaced by OM, M representing an alkyl group comprising from 1 to 30, preferably from 1 to 12 carbon atoms, an aryl group comprising from 4 to 10 carbon atoms, an aralkyl group comprising from 7 to 12, preferably from 7 to 9 carbon atoms, the aryl and aralkyl groups being optionally substituted by one or several, particularly up to 3, halogen atoms, $NO_2$, $NH_2$ or $CF_3$ groups or alkoxy groups from 1 to 4 carbon atoms, or M representing an acyl group from 2 to 8 carbon atoms, preferably 2 or 3, an aroyl group from 5 to 12, preferably from 5 to 9 carbon atoms, unsubstituted or substituted by one or several, particularly up to 3, $NO_2$, $NH_2$ or $CF_3$ groups, halogen atoms or alkoxy groups from 1 to 4 carbon atoms, provided that at least
X represents $-N-R_1$ with $R_1$ representing
|
$R_2$

$-C-N-CH_2-CH_2Hal$
‖ |
O NO

or Y represents $-N-R'_1$ with $R'_1$ representing
|
$R'_2$

$-C-N-CH_2CH_2Hal$
‖ |
O NO

7. Nitrosourea derivatives according to claim 6, characterized in that they correspond to the following formula (VI):

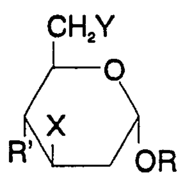

(VI)

in which R, R', X and Y have the meanings indicated in claim 6.

8. Nitrosourea derivatives according to claim 6, characterized in that they correspond to the following formula (VII):

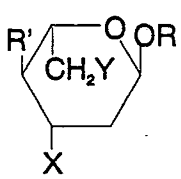

(VII)

in which R, R', X and Y have the meanings indicated in claim 3.

9. Nitrosourea derivatives according to claims 1 and 6 to 8, characterized in that:

- R represents an alkyl group from 1 to 12 carbon atoms, an aralkyl group from 7 to 12 carbon atoms;
- R' or R" represents an OM group, M being an alkyl group comprising from 1 to 12 carbon atoms, an aryl group comprising from 4 to 10 carbon atoms;
- X represents a $NR_1R_2$ group, $R_1$ representing
$-C-N-CH_2CH_2Hal$, Hal being a halogen, preferably Cl,
‖ |
O NO

– Y represents a hydrogen atom or a hydroxy group.

10. Nitrosourea derivatives according to claims 1 and 6 to 8, characterized in that:

– R represents an alkyl group from 1 to 12 carbon atoms, halogenoaralkyl from 7 to 12 carbon atoms;
– R' or R" represents an OM group, M being an acyl group from 2 to 8 carbon atoms, an aroyl group from 5 to 12 carbon atoms;
– X represents a $-NR_1R_2$ group, $R_1$ representing
$-C-N-CH_2CH_2Hal$, Hal being a halogen,
|| |
O NO
preferably Cl,
– Y represents a hydrogen atom or a hydroxy group.

11. Nitrosourea derivatives according to claims 1 to 4 and 6 to 8, characterized in that:

– R represents an alkyl group from 1 to 12 atoms, an aralkyl group from 7 to 12 carbon atoms;
– R' or R" represents OH;
– X represents a $NR_1R_2$ group, $R_1$ representing
$-C-N-CH_2CH_2Hal$, Hal representing halogen,
|| |
O NO
preferably Cl,
– Y represents a hydrogen atom.

12. Nitrosourea derivatives according to claims 1 to 4 and 6 to 8, characterized in that:

– R represents an alkyl group from 1 to 12 carbon atoms, an aralkyl group from 7 to 12 carbon atoms;
– R' or R" represents OH;
– X represents an alkylamino group, in which the alkyl group has from 1 to 6 carbon atoms, or arylamino in which the aryl group has from 4 to 10 carbon atoms, and
– Y represents $NR'_1R'_2$, $R'_1$ representing
$-C-N-CH_2CH_2Hal$, Hal being a halogen, preferably Cl.
|| |
O NO

13. Nitrosourea derivatives according to claims 1 to 4 and 6 to 8, characterized in that:

– R represents an alkyl group from 1 to 12 carbon atoms, an aralkyl group from 7 to 12 carbon atoms;
– R' or R" represent OH;
– X represents a hydroxy group,
– Y represents $NR'_1R'_2$ group, $R'_1$ representing
$-C-N-CH_2CH_2Hal$, Hal being a halogen atom, preferably Cl.
|| |
O NO

14. Nitrosourea derivatives according to claims 2 to 4, characterized in that:

– R represents an alkyl group from 1 to 12 carbon atoms, an aralkyl group from 7 to 12 carbon atoms;
– R' or R" represents an OM group, M being an alkyl group comprising from 1 to 12 carbon atoms, an aryl group comprising from 4 to 10 carbon atoms;
– X represents a $NR_1R_2$ group, $R_1$ representing
$-C-N-CH_2CH_2Hal$, Hal being a halogen, preferably Cl
|| |
O NO
– Y represents a hydrogen atom.

15. Nitrosourea derivatives according to claims 2 to 4, characterized in that:

– R represents an alkyl group from 1 to 12 carbon atoms, a halogenoaralkyl group from 7 to 12 carbon atoms;
– R' or R" represents an OM group, M being an acyl group having from 2 to 8 carbon atoms, an aroyl group having 5 to 12 carbon atoms;
– X represents a $-NR_1R_2$ group, $R_1$ representing

–C–N–CH2CH2Hal, Hal being a halogen,
|| |
O NO
preferably Cl,

- Y represents a hydrogen atom.

16. Nitrosourea derivatives according to claim 5, characterized in that:

- R represents an alkyl group from 1 to 12 carbon atoms, an aralkyl group from 7 to 12 carbon atoms;
- R' or R" represents an OM group, M being an alkyl group comprising from 1 to 12 carbon atoms, an aryl group comprising from 4 to 10 carbon atoms;
- X represents a $NR_1R_2$ group, $R_1$ representing
  –C–N–CH2CH2Hal, Hal being a halogen, preferably Cl
  || |
  O NO and
- Y represents a hydroxy group.

17. Nitrosourea derivatives according to claim 5, characterized in that:

- R represents an alkyl group from 1 to 12 carbon atoms, a halogenoaralkyl group from 7 to 12 carbon atoms;
- R' or R" represents an OM group, M being an acyl group having from 2 to 8 carbon atoms, an aroyl group from 5 to 12 carbon atoms;
- X represents an $-NR_1R_2$ group, $R_1$ representing
  –C–N–CH2CH2Hal, Hal being a halogen,
  || |
  O NO
  preferably Cl,
- Y represents a hydroxy group.

18. Compounds according to claim 1, of the formula:

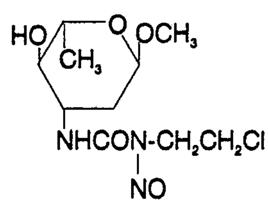

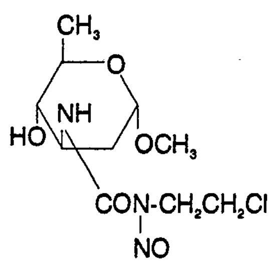

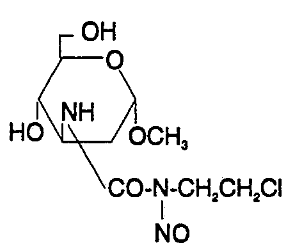

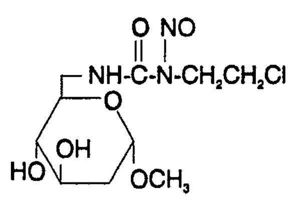

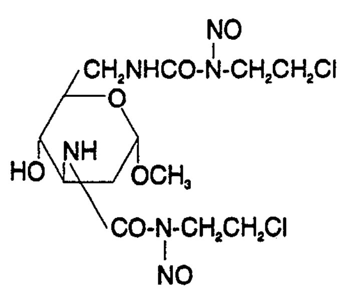

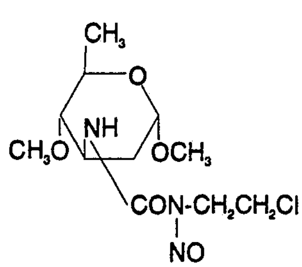

19. Process for the preparation of compounds according to anyone of claims 1 to 18, characterized in that it consists of reacting in a first step an oside group of the general formula (Ibis) below:

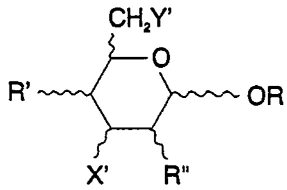

(I bis)

in which:

- R, R' and R" have the meanings indicated in claims 1 to 18;
- X' represents a hydroxy group or $-NHR_2$;
- Y' represents a hydrogen atom, a hydroxy group or $-NHR'_2$;
- $R_2$ and $R'_2$ identical or different, represent an alkyl group from 1 to 12 carbon atoms, an aralkyl group from 7 to 12 carbon atoms, a cycloalkyl group from 3 to 6 carbon atoms, the aryl and aralkyl groups being optionally substituted by one or several, particularly up to 3, halogen atoms, $NO_2$, $NH_2$, $CF_3$ groups or alkoxy groups from 1 to 4 carbon atoms;
  and in which one at least of the X' and Y' groups represents $-NHR_2$ or $-NHR'_2$
  on a 2-halogeno-ethyl isocyanate to convert the $NHR_2$ or $-NHR'_2$ group of the compound of formula (Ibis) respectively into
  $-NR_2CNHCH_2CH_2Hal$ or $-NR'_2CNHCH_2CH_2Hal$,
  || ||
  O O
  Hal being a halogen atom, particularly chlorine, and in one second step, subjecting the compound obtained at the end of the first step to nitrosation, by means of an alkali metal nitrite, preferably sodium nitrite to convert the
  $-NR_2CNHCH_2CH_2Hal$ or $-NR'_2CNHCH_2CH_2Hal$ groups,
  || ||
  O O
  respectively into $-NR_2CNCH_2CH_2Hal$ or $NR'_2CNCH_2CH_2$ Hal.
  || | || |
  O NO O NO

20. Preparation process according to claim 19 to prepare compounds of formula (VI):

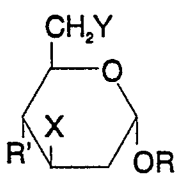

(VI)

characterized in that it consists in reacting a compound of formula (VIbis):

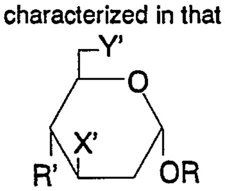

(VI bis)

where:

- R and R' are as defined above;
- X' represents a $-NHR_2$ or a hydroxy group;
- Y' represents a hydrogen atom, a hydroxy group or $-NHR'_2$ group and can represent a halogen atom when X' represents a hydroxy group;
- $R_2$ and $R'_2$ identical or different, represent independently of one another, a hydrogen atom, an alkyl group from 1 to 30 carbon atoms, an aralkyl group from 7 to 12 carbon atoms, an aryl group from 4 to 10 carbon atoms, a cycloalkyl group from 3 to 6 carbon atoms, the aryl and aralkyl groups being optionally substituted by one or several, particularly up to 3, halogen atoms, $NO_2$, $NH_2$, $CF_3$ groups or alkoxy groups from 1 to 4 carbon atoms;
  and in which one at least of the X' and Y' groups represent $-NHR_2$ or $-NHR'_2$
  on a 2-halogeno-ethyl isocyanate to convert the $NHR_2$ or $-NHR'_2$ group of the compound of formula (VIbis) respectively into

$-NR_2CNHCH_2CH_2Hal$ or $-NR'_2CNHCH_2CH_2Hal$
||  ||
O  O

Hal being a halogen atom, particularly chlorine, the compound obtained at the end of the preceding step being then subjected to nitrosation, by means of an alkali metal nitrite, preferably sodium nitrite to convert the
$-NR_2CNHCH_2CH_2Hal$ and $-NR'_2CNHCH_2CH_2Hal$
||  ||
O  O
groups respectively into
$-NR_2CNCH_2CH_2Hal$ or $-NR'_2CNCH_2CH_2Hal$.
|| |  || |
O NO  O NO

21. Preparation process according to claim 19, to prepare compounds of formula (VII):

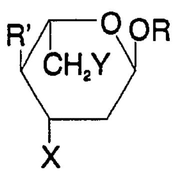

(VII)

characterized in that it consists in reacting a compound of formula (VIIbis):

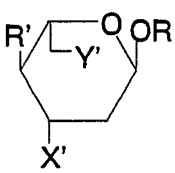

(VII bis)

where:

- R and R' are as defined above;
- X' represents a $-NHR_2$ or a hydroxy group;
- Y' represents a hydrogen atom, a hydroxy group or $-NHR'_2$ group and can represent a halogen atom when X' represents a hydroxy group;
- $R_2$ and $R'_2$ identical or different, represent independently of one another, a hydrogen atom, an alkyl group from 1 to 30 carbon atoms, an aralkyl group from 7 to 12 carbon atoms, an aryl group from 4 to 10 carbon atoms, a cycloalkyl group from 3 to 6 carbon atoms, the aryl and aralkyl groups being optionally substituted by one or several, particularly up to 3, halogen atoms, $NO_2$, $NH_2$, $CF_3$ groups or alkoxy groups from 1 to 4 carbon atoms;
  and in which one at least of the X' and Y' groups represents $-NHR_2$ or $-NHR'_2$
  on a 2-halogeno-ethyl isocyanate to convert the $NHR_2$ or $-NHR'_2$ group of the compound of formula (VIIbis) respectively into
  $-NR_2CNHCH_2CH_2Hal$ or $-NR'_2CNHCH_2CH_2Hal$
  ||  ||
  O  O
  Hal being a halogen atom, particularly chlorine, the compound obtained at the end of the preceding step being then subjected to nitrosation, by means of an alkali metal nitrite, preferably sodium nitrite to convert the
  $-NR_2CNHCH_2CH_2Hal$ and $-NR'_2CNHCH_2CH_2Hal$
  ||  ||
  O  O
  groups respectively into
  $-NR_2CNCH_2CH_2Hal$ or $-NR'_2CNCH_2CH_2Hal$.
  || |  || |
  O NO  O NO

22. Preparation process according to claim 19 for preparing compounds of the following formula:

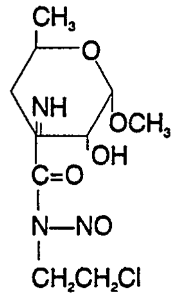

characterized by the fact that a compound of formula:

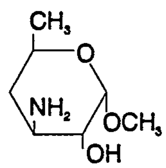

is reacted with 2-chloroethyl-isocyanate to transform the $NH_2$ group into $NHCONHCH_2CH_2Cl$, to give the compound of the following formula:

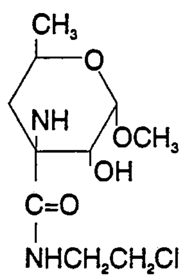

which is treated with sodium nitrite in formic acid to transform the group $NHCONHCH_2CH_2Cl$ into
$NHCONHCH_2CH_2Cl$.
|
NO

23. Pharmaceutical composition, characterized in that it comprises, in association with a pharmaceutical vehicle, a compound according to anyone of claims 1 to 18.

24. Pharmaceutical composition according to claim 23, characterized in that it comprises 100 mg of at least one of the compounds according to claims 1 to 18, presented under the form of sterile freeze-dryed powder associated with an ampoula of a physiologically acceptable solvent, particularly an alcohol, such as ethanol, in an amount of 5 ml per ampoula.

**Claims** for the Contracting State: AT

1. Process for the preparation of nitrosourea derivatives, of the following formula (I):

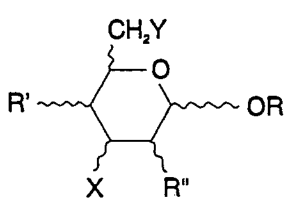

(I)

in which:

- R represents a hydrogen atom, an alkyl group from 1 to 30, preferably from 1 to 12 carbon atoms or an aralkyl group from 7 to 12, preferably from 7 to 9 carbon atoms, optionally substituted by one or several, particularly up to

3, halogen atoms, $NO_2$, $NH_2$, $CF_3$ groups or alkoxy groups from 1 to 4 carbon atoms;

- X represents a hydroxy group or a $-NR_1R_2$ group;
- Y represents:
  - a hydrogen atom;
  - a hydroxy group or a $N—R'_1$ group where $R_1$
    
    |
    
    $R_2$

and/or $R'_1$ each represents a hydrogen atom or a

$-C-N-CH_2-CH_2Hal$ group, Hal being a halogen,

|| |

O NO

preferably Cl

and $R_2$ and/or $R'_2$ each represents a hydrogen atom, an alkyl group comprising from 1 to 6 carbon atoms, an aralkyl group comprising from 7 to 12, preferably from 7 to 9 carbon atoms, a cycloalkyl group comprising from 3 to 6 carbon atoms, an aryl group from 4 to 10 carbon atoms, the aryl and aralkyl groups being optionally substituted by one or several, particularly up to 3 halogen atoms, $NO_2$, $NH_2$, $CF_3$ groups or alkoxy groups from 1 to 4 carbon atoms;

- R' and R" represent hydrogen, OH, OM, M representing an alkyl group comprising from 1 to 30, preferably from 1 to 12 carbon atoms, an aryl group from 4 to 10 carbon atoms, an aralkyl group comprising from 7 to 12, preferably from 7 to 9 carbon atoms, the aryl and aralkyl groups being optionally substituted b one or several, particularly up to 3 halogen atoms, $NO_2$, $NH_2$, $CF_3$ groups, or alkoxy groups from 1 to 4 carbon atoms, or M representing an acyl group from 2 to 8 carbon atoms, preferably 2 or 3, an aroyl group from 5 to 12, preferably from 5 to 9 carbon atoms, unsubstituted or substituted by one or several, particularly up to 3, $NO_2$, $NH_2$, $CF_3$ groups, halogen, alkoxy from 1 to 4 carbon atoms;

provided that at least:

- X represents $-N—R_1$ with $R_1$ representing
  
  |
  
  $R_2$
  
  $-C-N-CH_2CH_2Hal$ or
  
  || |
  
  O NO
- Y represents $-N—R'_1$ with $R'_1$ representing
  
  |
  
  $R'_2$
  
  $-C-N-CH_2CH_2Hal$
  
  || |
  
  O NO

and provided that either R' represents hydrogen or R" represents hydrogen, R' and R" being not simultaneously hydrogen atoms,

characterized in that it consists of reacting in a first step an oside group of the general formula (Ibis) below:

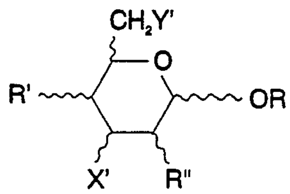

(I bis)

in which:

- R, R' and R" have the above mentioned meanings;
- X' represents a hydroxy group or $-NHR_2$;
- Y' represents a hydrogen atom, a hydroxy group, $-NHR'_2$;
- $R_2$ and $R'_2$ identical or different, represent an alkyl group from 1 to 12 carbon atoms, an aralkyl group from 7 to 12 carbon atoms, a cycloalkyl group from 3 to 6 carbon atoms, the aryl and aralkyl groups being optionally substituted by one or several, particularly up to 3, halogen atoms, $NO_2$, $NH_2$, $CF_3$ groups or alkoxy groups from 1 to 4 carbon atoms;

and in which one at least of the X' and Y' groups represents $-NHR_2$ or $-NHR'_2$

on a 2-halogeno-ethyl isocyanate to convert the $-NHR_2$ or $-NHR'_2$ group of the compound of formula (Ibis) respectively into

$-NR_2CNHCH_2CH_2Hal$ or $-NR'_2CNHCH_2CH_2$ Hal,

|| ||

O O

Hal being a halogen atom, particularly chlorine, and in a second step, subjecting the compound obtained at the end of the preceeding step to nitrosation, by means of an alkali metal nitrite, preferably sodium nitrite to convert the

$-NR_2CNHCH_2CH_2Hal$ or $-NR'_2CNHCH_2CH_2Hal$ groups,
||   ||
O   O
respectively into $-NR_2CNCH_2CH_2Hal$
|| |
O NO
or $-NR'_2CNCH_2CH_2Hal$.
|| |
O NO

2. Process according to claim 1, for preparing nitrosourea derivatives of the following formula (II):

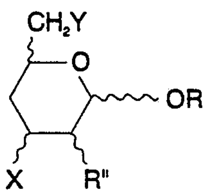

(II)

in which:

- R represents a hydrogen atom, an alkyl group from 1 to 30, preferably from 1 to 12 carbon atoms or an aralkyl group from 7 to 12, preferably from 7 to 9 carbon atoms, optionally substituted by one or several, particularly up to 3, halogen atoms, $NO_2$, $NH_2$, $CF_3$ groups or alkoxy groups from 1 to 4 carbon atoms;
- X represents a hydroxy group or a $-NR_1R_2$ group;
- Y represents:
- a hydrogen atom;
- or a $-N-R'_1$ group where
  |
  $R'_2$

  $R_1$ and/or $R'_1$ each represents a hydrogen atom or a $-C-N-CH_2CH_2Hal$ group, Hal being a halogen,
  || |
  O NO
  preferably Cl,
  and $R_2$ and/or $R'_2$ each represents a hydrogen atom, an alkyl group comprising from 1 to 6 carbon atoms, an aralkyl group comprising from 7 to 12, preferably from 7 to 9 carbon atoms, a cycloalkyl group comprising from 3 to 6 carbon atoms, the aryl and aralkyl groups being optionally substituted by one or several, particularly up to 3, halogen atoms, $NO_2$, $NH_2$, $CF_3$ groups or alkoxy groups from 1 to 4 carbon atoms;
- R" represents preferably OH, but can represent OM, M representing an alkyl group comprising from 1 to 30, preferably from 1 to 12 carbon atoms, an aryl group from 4 to 10 carbon atoms, an aralkyl group comprising from 7 to 12, preferably from 7 to 9 carbon atoms, the aryl and aralkyl groups being optionally substituted by one or several, particularly up to 3 halogen atoms, $NO_2$, $NH_2$, $CF_3$ groups or alkoxy groups from 1 to 4 carbon atoms, or M representing an acyl group from 2 to 8 carbon atoms, preferably 2 or 3, an aroyl group from 5 to 12, preferably from 5 to 9 carbon atoms, unsubstituted or substituted by one or several, particularly up to 3, $NO_2$, $NH_2$, $CF_3$ groups, halogen, alkoxy from 1 to 4 carbon atoms;
  provided that at least X represents $-N-R_1$ with $R_1$
  |
  $R_2$

  representing $-C-N-CH_2CH_2Hal$ or
  || |
  O NO

  Y represents $-N-R'_1$ with $R'_1$
  |
  $R'_2$

  representing $-C-N-CH_2CH_2Hal$
  || |
  O NO

3. Process according to claim 2, for preparing nitrosourea derivatives of the following formula (III):

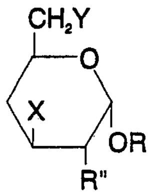

(III)

in which R, R", X and Y have the meanings mentioned in claim 2.

4. Process according to claim 2, for preparing nitrosourea derivatives of the following formula (IV):

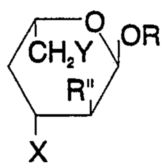

(IV)

in which R, R", X and Y have the meanings mentioned in claim 2.

5. Process according to claim 1, for preparing nitrosourea derivatives of the following formula (IV):

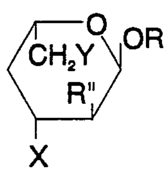

(IV)

in which R, R" and X have the meanings mentioned in claim 1 and Y represents a hydroxy group.

6. Process according to claim 1, for preparing nitrosourea derivatives of the following formula (V):

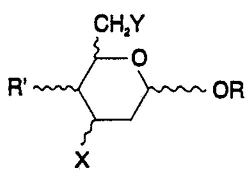

in which:

- R represents a hydrogen atom, an alkyl group from 1 to 30, preferably from 1 to 12 carbon atoms or an aralkyl group from 7 to 12, preferably from 7 to 9 carbon atoms, optionally substituted by one or several, particularly up to 3, halogen atoms, $NO_2$, $NH_2$ or $CF_3$ groups or alkoxy groups having from 1 to 4 carbon atoms,
- X represents a hydroxy group or a $-NR_1R_2$ group
- Y represents a hydrogen atom,
  a hydroxy group or an N—R'₁
  
  |
  
  R'₂
  
  group where $R_1$ and/or $R'_1$ each represents a hydrogen atom or a
  
  $-C-N-CH_2-CH_2Hal$ group, Hal being a halogen,
  
  || |
  
  O NO
  
  preferably Cl,
  
  and $R_2$ and/or $R'_2$ each represents a hydrogen atom, an alkyl group comprising from 1 to 6 carbon atoms, an aralkyl group comprising from 7 to 12, preferably from 7 to 9 carbon atoms, a cycloalkyl group comprising from 3 to 6 carbon atoms, an aryl group from 4 to 10 carbon atoms, the aryl and aralkyl groups being optionally substituted by one or several, particularly up to 3, halogen atoms, $NO_2$, $NH_2$ or $CF_3$ groups or alkoxy groups from 1 to 4 carbon atoms,
- R' represents preferably OH, but can represent OM, M representing an alkyl group comprising from 1 to 30, preferably from 1 to 12 carbon atoms, an aryl group comprising from 4 to 10 carbon atoms, an aralkyl group comprising

from 7 to 12, preferably from 7 to 9 carbon atoms, the aryl and aralkyl groups being optionally substituted by one or several, particularly up to 3, halogen atoms, $NO_2$, $NH_2$ or $CF_3$ groups or alkoxy groups from 1 to 4 carbon atoms, or representing an acyl group from 2 to 8 carbon atoms, preferably 2 or 3, an aroyl group from 5 to 12, preferably from 5 to 9 carbon atoms, unsubstituted or substituted by one or several, particularly up to 3, $NO_2$, $NH_2$ or $CF_3$ groups, halogen atoms or alkoxy groups from 1 to 4 carbon atoms, provided that at least

X represents $-N-R_1$ with $R_1$ representing
|
$R_2$

$-C-N-CH_2-CH_2Hal$
|| |
O NO

or Y represents $-N-R'_1$ with $R'_1$ representing
|
$R'_2$

$-C-N-CH_2CH_2Hal$
|| |
O NO

7. Process according to claim 6, for preparing compounds of the following formula (VI):

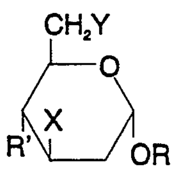

(VI)

in which R, R', X and Y have the meanings indicated in claim 6.

8. Process according to claim 6, for preparing compounds of the following formula (VII):

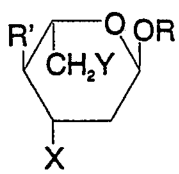

(VII)

in which R, R', X and Y have the meanings indicated in claim 6.

9. Process according to claims 1 and 6 to 8, for preparing compounds of formula (I), (V), (VI) or (VII), in which:

- R represents an alkyl group from 1 to 12 carbon atoms, an aralkyl group from 7 to 12 carbon atoms;
- R' or R" represents an OM group, M being an alkyl group comprising from 1 to 12 carbon atoms, an aryl group comprising from 4 to 10 carbon atoms;
- X represents a $NR_1R_2$ group, $R_2$ representing
  $-C-N-CH_2CH_2Hal$, Hal being a halogen, preferably Cl,
  || |
  O NO
- Y represents a hydrogen atom or a hydroxy group.

10. Process according to claims 1 and 6 to 8, for preparing compounds of formula I, V, VI or VII, in which:

- R represents an alkyl group from 1 to 12 carbon atoms, halogenoaralkyl from 7 to 12 carbon atoms;
- R' or R" represents an OM group, M being an acyl group from 2 to 8 carbon atoms, an aroyl group from 7 to 12 carbon atoms;
- X represents a $-NR_1R_2$ group, $R_1$ representing $-C-N-CH_2CH_2Hal$, Hal being a halogen,
  || |
  O NO
  preferably Cl,
- Y represents a hydrogen atom or a hydroxy group.

11. Process according to claims 1 to 4 and 6 to 8, for preparing compounds of formula I, II, V, VI or VII, in which:

- R represents an alkyl group from 1 to 12 atoms, an aralkyl group from 7 to 12 carbon atoms;
- R' or R" represents OH;
- X represents a $NR_1R_2$ group, $R_1$ representing
  $-\underset{\underset{O}{\|}}{C}-\underset{\underset{NO}{|}}{N}-CH_2CH_2Hal$, Hal representing halogen,
  preferably Cl,
- Y represents a hydrogen atom.

12. Process according to claims 1 to 4 and 6 to 8, for preparing compounds of formula I, II, V, VI or VII, in which:

- R represents an alkyl group from 1 to 12 carbon atoms, an aralkyl group from 7 to 12 carbon atoms;
- R' or R" represents OH;
- X represents an alkylamino group, in which the alkyl group has from 1 to 6 carbon atoms, or arylamino in which the aryl group has from 4 to 10 carbon atoms, and
- Y represents $NR'_1R'_2$, $R'_1$ representing $-\underset{\underset{O}{\|}}{C}-\underset{\underset{NO}{|}}{N}-CH_2CH_2Hal$, Hal being a halogen, preferably Cl.

13. Process according to claims 1 to 4 and 6 to 8, for preparing compounds of formula I, II, V, VI or VII, in which:

- R represents an alkyl group from 1 to 12 carbon atoms, an aralkyl group from 7 to 12 carbon atoms;
- R' or R" represent OH;
- X represents a hydroxy group,and
- Y represents $NR'_1R'_2$ group, $R'_1$ representing
  $-\underset{\underset{O}{\|}}{C}-\underset{\underset{NO}{|}}{N}-CH_2CH_2Hal$, Hal being a halogen atom,
  preferably Cl.

14. Process according to claims 2 to 4, for preparing nitrosourea derivatives, characterized in that:

- R represents an alkyl group from 1 to 12 carbon atoms, an aralkyl group from 7 to 12 carbon atoms;
- R' or R" represents an OM group, M being an alkyl group comprising from 1 to 12 carbon atoms, an aryl group comprising from 4 to 10 carbon atoms;
- X represents a $NR_1R_2$ group, $R_1$ representing $-\underset{\underset{O}{\|}}{C}-\underset{\underset{NO}{|}}{N}-CH_2CH_2Hal$, Hal being a halogen, preferably Cl
- Y represents a hydrogen atom.

15. Process according to claims 2 to 4, for preparing nitrosourea derivatives, characterized in that:

- R represents an alkyl group from 1 to 12 carbon atoms, a halogenoaralkyl group from 7 to 12 carbon atoms;
- R' or R" represents an OM group, M being an acyl group having from 2 to 8 carbon atoms, an aroyl group having 5 to 12 carbon atoms;
- X represents a $-NR_1R_2$ group, $R_1$ representing
  $-\underset{\underset{O}{\|}}{C}-\underset{\underset{NO}{|}}{N}-CH_2CH_2Hal$, Hal being a halogen,
  preferably Cl,
- Y represents a hydrogen atom.

16. Process according to claim 5, for preparing nitrosourea derivatives, characterized in that:

- R represents an alkyl group from 1 to 12 carbon atoms, an aralkyl group from 7 to 12 carbon atoms;
- R' or R" represents an OM group, M being an alkyl group comprising from 1 to 12 carbon atoms, an aryl group comprising from 4 to 10 carbon atoms;
- X represents a $NR_1R_2$ group, $R_1$ representing
  $-\underset{\underset{O}{\|}}{C}-\underset{\underset{NO}{|}}{N}-CH_2CH_2Hal$, Hal being a halogen, preferably Cl;

- Y represents a hydroxy group.

17. Process according to claim 5, for preparing nitrosourea derivatives, characterized in that:

- R represents an alkyl group from 1 to 12 carbon atoms, a halogenoaralkyl group from 7 to 12 carbon atoms;
- R' or R" represents an OM group, M being an acyl group having from 2 to 8 carbon atoms, an aroyl group from 5 to 12 carbon atoms;
- X represents an $-NR_2R_2$ group, $R_1$ representing
  $-C-N-CH_2CH_2Hal$, Hal being a halogen,
  || |
  O NO
  preferably Cl,
- Y represents a hydroxy group.

18. Process according to claim 1, for preparing a nitrosourea derivative of the formula:

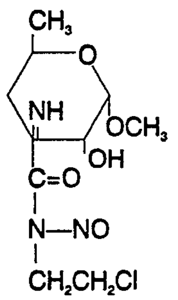

characterized by the fact that a compound of formula:

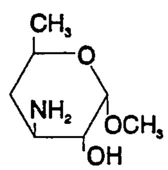

is reacted with 2-chloroethyl-isocyanate to transform the $NH_2$ group into $NHCONHCH_2CH_2Cl$, to give the compound of the following formula:

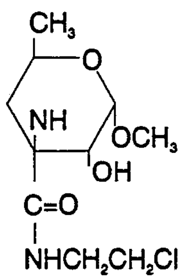

which is treated with sodium nitrite in formic acid to transform the group $NHCONHCH_2CH_2Cl$ into
$NHCONHCH_2CH_2Cl$.
|
NO

19. Process according to claim 1, for the preparation of compounds of formula:

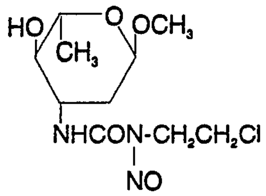

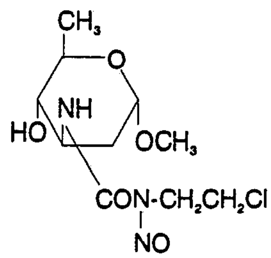

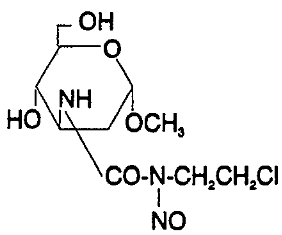

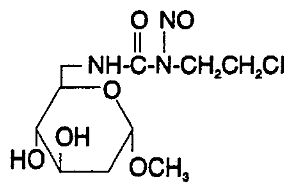

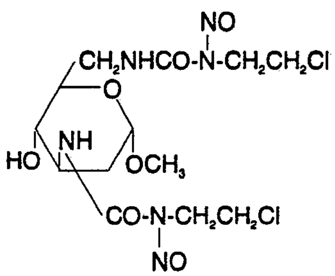

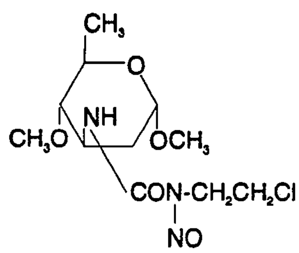

20. Process for the preparation of a pharmaceutical composition, characterized in that anyone of the compounds obtained from the process according to anyone of claims 1 to 19 with a pharmaceutical vehicle acceptable under conditions enabling the production of said pharmaceutical composition.